# EUROPEAN PATENT APPLICATION

(11) **EP 2 677 041 A2**
(43) Date of publication of application: **25.12.2013**
(21) Application number: 13177701.3
(22) Date of filing: 19.02.2009
(51) Int. Cl.: C12Q 1/68

(54) **Detection and prognosis of lung cancer**

(30) Priority: 19.02.2008 US 29693 P
(62) Divisional of application: 09712527.2
(71) Applicant: MDxHealth SA, 4000 Sart-Tilman (Liege) (BE); The Johns Hopkins University, Baltimore MD 21218 (US)
(72) Inventor: Van Criekinge, Wim, B-2550 Waarloos (BE); Straub, Josef, 64342 Seeheim-Jungenheim (DE); Trooskens, Geert, B-9000 Gent (BE); Baylin, Stephen, Baltimore, MD 21230 (US); Herman, James, Baltimore, MD 21093 (US); Schuebel, Kornel, Baltimore, MD 21228 (US); Cope, Leslie, Baltimore, MD 21205 (US); Van Neste, Leander, B-3320 Hoegaarden (BE)
(74) Representative: Tombling, Adrian George

(57) **Abstract**

Methods and tools are provided for detecting and predicting lung cancer. The methods and tools are based on epigenetic modification due to methylation of genes in lung cancer or pre-lung cancer. The tools can be assembled into kits or can be used seperately. Genes found to be epigentically silenced in association with lung cancer include ACSL6, ALS2CL, APC2, ARTS1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655.

## Description

### TECHNICAL FIELD OF THE INVENTION

The present invention relates to the area of cancer diagnostics and therapeutics. In particular, it relates to methods and kits for identifying, diagnosing, prognosing and monitoring lung cancer. These methods include determining the methylation status or the expression levels of particular genes, or a combination thereof. In particular, the lung cancer relates to non-small cell lung cancer.

### BACKGROUND OF THE INVENTION

Lung cancer is the most common cause of cancer-related death and causes over one million deaths worldwide each year (Greenlee et al, 2001). Lung cancer is clinically subdivided into small cell lung cancer (SCLC; comprise about 20% of lung cancers), the most aggressive form of lung cancer, and non-small cell lung cancer (NSCLC, the most common lung cancer accounting for about 80%), consisting of adenocarcinoma, squamous cell carcinoma, large cell carcinoma, and miscellaneous other types such as carcinoids, pleomorphic and mixed carcinomas and a range of neuroendocrine cancers (Travis, 2002).

The first signs of cancer usually come from one or more of the following sources: presentation of symptoms, visual detection or direct palpation, histopathological analysis of a biopsy specimen, remote imaging or the detection of a cancer biomarker in a tissue or bodily fluid specimen. The rather late appearance of symptomatology associated with lung cancer, and the poor accessibility to the lung tissue thwart the timely detection of malignancy, contributing to high mortality rates (Ganti *et al.*, 2006; Greenberg *et al.*, 2007). Therefore, remote imaging and the development of cancer biomarkers offers the best hope for early detection of lung cancer.

Cancer biomarkers have been described in literature. One can distinguish between immunological markers and genetic markers. Genetic markers are based on detection of mutation in distinct genes in particular in tumor suppressor genes. More recently, DNA methylation markers are evaluated as potential genetic markers for detection of cancer because they offer certain advantages when compared to mutation markers. One of the most important features is that they occur at the early stages of cancer development and in many cases are tissue- and tumor-type specific (Esteller et al. 2001). A further advantage, methylation profile is preserved in purified isolated DNA and methylation changes appear to precede apparent malignancy in many cases. In addition, methylation markers may serve for predictive purposes as they often reflect the sensitivity to therapy or duration of patient survival.

DNA methylation is a chemical modification of DNA performed by enzymes called methyltransferases, in which a methyl group (m) is added to certain cytosines (C) of DNA. This non-mutational (epigenetic) process (mC) is a critical factor in gene expression regulation. See, J.G. Herman, Seminars in Cancer Biology, 9: 359-67, 1999. By turning genes off that are not needed, DNA methylation is an essential control mechanism for the normal development and functioning of organisms. Alternatively, abnormal DNA methylation is one of the mechanisms underlying the changes observed with aging and development of many cancers.

Although the phenomenon of gene methylation has attracted the attention of cancer researchers for some time, its true role in the progression of human cancers is just now being recognized. In normal cells, methylation occurs predominantly in regions of DNA that have few CG base repeats, while CpG islands, regions of DNA that have long repeats of CG bases, remain non-methylated. Gene promoter regions that control protein expression are often CpG island-rich. Aberrant methylation of these normally non-methylated CpG islands in the promoter region causes transcriptional inactivation or silencing of certain tumor suppressor expression in human cancers.

Genes that are hypermethylated in tumor cells are strongly specific to the tissue of origin of the tumor. Molecular signatures of cancers of all types can be used to improve cancer detection, the assessment of cancer risk and response to therapy. Promoter hypermethylation events provide some of the most promising markers for such purposes.

An early diagnosis is critical for the successful treatment of many types of cancer, including lung cancer. If the exact methylation profiles of lung tumors are available and drugs targeting the specific genes are obtainable, then the treatment of lung cancer could be more focused and rational. Therefore, the detection and mapping of novel methylation markers is an essential step towards improvement of lung cancer prevention, screening and treatment.

There is a continuing need in the art to identify methylation markers that can be used for improved assessment of lung cancer.

### SUMMARY OF THE INVENTION

According to one embodiment of the invention a method is provided for identifying lung cancer or its precursor, or predisposition to lung cancer. Epigenetic modification of at least one gene selected from the group consisting of ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655 is detected in a test sample containing lung cells or nucleic acids from lung cells. The test sample is identified as containing cells that are neoplastic, precursor to neoplastic, or predisposed to neoplasia, or as containing nucleic acids from cells that are neoplastic, precursor to neoplastic, or predisposed to neoplasia.

According to another embodiment of the invention a kit is provided for assessing lung cancer or its precursor, or predisposition to lung cancer in a test sample containing lung cells or nucleic acids from lung cells. The kit comprises in a package: a reagent that (a) modifies methylated cytosine residues but not non-methylated cytosine residues, or that (b) modifies non-methylated cytosine residues but not methylated cytosine residues; and at least one pair of oligonucleotide primers that specifically hybridizes under amplification conditions to a region of a gene selected from the group consisting of ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655. The region is within about 3 kb of said gene's transcription start site.

Another embodiment of the invention provides a second kit for assessing lung cancer or its precursor, or predisposition to lung cancer in a test sample containing lung cells or nucleic acids from lung cells. The kit comprises in a package: at least two pairs of oligonucleotide primers that specifically hybridize under amplification conditions to a region of a gene selected from the group consisting of ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1,PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655. The region is within about 3 kb of said gene's transcription start site.

An additional aspect of the invention provides an isolated polynucleotide. The polynucleotide comprises a nucleotide sequence selected from the group consisting of SEQ ID NO: 1-449.

These and other embodiments which will be apparent to those of skill in the art upon reading the specification provide the art with reagents and methods for detecting lung cancer, early lung cancer, or predisposition to lung cancer.

### BRIEF DESCRIPTION OF THE DRAWINGS

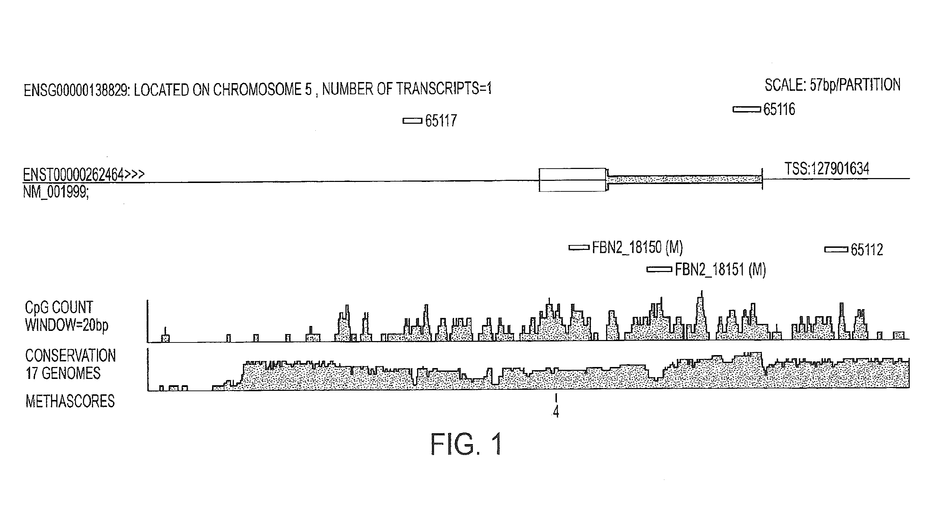

Figure 1: Position of the different primers relative to the TSS (transcription start site). Multiple primer designs are displayed by blue boxes and red boxes (=final primer pairs retained for the assays). The exon of FBN2 is indicated in green. The number of CpG count is spotted in blue over a region of 20 kb.

Figure 2 lists the sequences of the different primer sets and converted and unconverted amplicon sequences used in Figure 1.

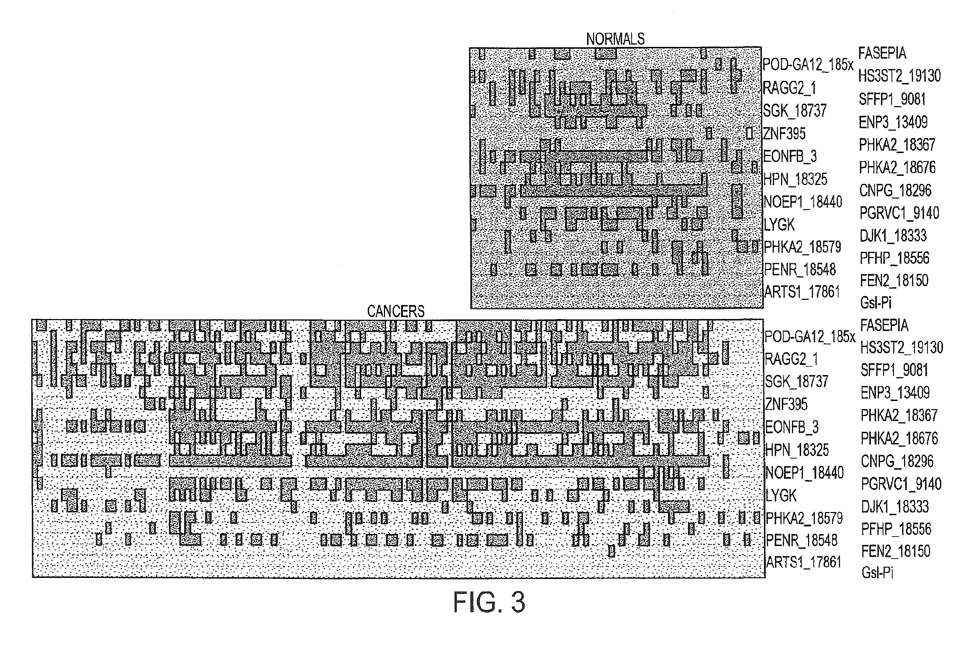

Figure 3: Ranked methylation table obtained with the sample set. 146 methylation profiles from lung cancer samples (right table) are compared against 58 normal tissue samples (left table). Samples are shown along the Y-axis where each horizontal row represents the methylation profile of one individual sample across the 23 different assays (X-axis). Assays demonstrating the best methylation discriminators between the 2 groups are displayed at the left, with discrimination effect decreasing towards the right. The black boxes indicate the methylated results; grey boxes indicate the unmethylated results; white boxes indicate invalid results.

Figure 4: Amplification plot for the standard curve for *JAM3*

Figure 5: Amplification plot for standard curve and samples for *JAM3*

Figure 6: Linear regression of standard curve for *JAM3*

Figure 7: Decision tree for ratio determination

Figure 8: Performance of the individual markers on lung tissue samples using qMSP.

### DETAILED DESCRIPTION OF THE INVENTION

The inventors have found that cytosines within CpG dinucleotides of DNA from particular genes isolated from a test sample are differentially methylated in human lung cancer tissue samples and normal lung tissue control samples. The cancer tissues samples are hypermethylated or hypomethylated with respect to the normal samples (collectively termed epigenetic modification). The differential methylation has been found in genomic DNA of ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655. These genes are all known in the art and fully described by sequence in publicly available databases, *e.g*., Entrez Gene of the National Center for Biotechnology Information. See Gene ID references provided in Table 1 and Table 3, each of which is incorporated by reference herein.

Epigenetic modification of a gene can be determined by any method known in the art. One method is to determine that a gene which is expressed in normal cells or other control cells is less expressed or not expressed in tumor cells. Conversely, a gene can be more highly expressed in tumor cells than in control cells in the case of hypomethylation. This method does not, on its own, however, indicate that the silencing or activation is epigenetic, as the mechanism of the silencing or activation could be genetic, for example, by somatic mutation. One method to determine that silencing is epigenetic is to treat with a reagent, such as DAC (5'-deazacytidine), or with a reagent which changes the histone acetylation status of cellular DNA or any other treatment affecting epigenetic mechanisms present in cells, and observe that the silencing is reversed, *i.e*., that the expression of the gene is reactivated or restored. Another means to determine epigenetic modification is to determine the presence of methylated CpG dinucleotide motifs in the silenced gene or the absence of methylation CpG dinucleotide motifs in the activated gene. Typically these methylated motifs reside near the transcription start site, for example, within about 3 kbp, within about 2.5 kbp, within about 2 kbp, within about 1.5 kbp, within about 1 kbp, within about 750 bp, or within about 500 bp. CpG dinucleotides susceptible to methylation are typically concentrated in the promoter region, intron region or exon region of human genes. Thus, the methylation status of the promoter and/or intron and/or exon region of at least one gene can be assessed. Once a gene has been identified as the target of epigenetic modification in tumor cells, determination of reduced or enhanced expression can be used as an indicator of epigenetic modification.

Expression of a gene can be assessed using any means known in the art. Typically expression is assessed and compared in test samples and control samples which may be normal, non-malignant cells. The test samples may contain cancer cells or pre-cancer cells or nucleic acids from them. For example the sample may contain lung adenoma cells, lung advanced adenoma cells, or lung adenocarcinoma cells. Samples may contain squamous cells, and large cell carcinoma. Samples may contain mixtures of different types and stages of lung cancer cells. Either mRNA (nucleic acids) or protein can be measured to detect epigenetic modification. Methods employing hybridization to nucleic acid probes can be employed for measuring specific mRNAs. Such methods include using nucleic acid probe arrays (microarray technology), in situ hybridization, and using Northern blots. Messenger RNA can also be assessed using amplification techniques, such as RT-PCR. In some embodiments oligonucleotide probes are covalently linked to primers for amplification. Advances in genomic technologies now permit the simultaneous analysis of thousands of genes, although many are based on the same concept of specific probe-target hybridization. Sequencing-based methods are an alternative; these methods started with the use of expressed sequence tags (ESTs), and now include methods based on short tags, such as serial analysis of gene expression (SAGE) and massively parallel signature sequencing (MPSS). Differential display techniques provide yet another means of analyzing gene expression; this family of techniques is based on random amplification of cDNA fragments generated by restriction digestion, and bands that differ between two tissues identify cDNAs of interest. Specific proteins can be assessed using any convenient method including immunoassays and immunocytochemistry but are not limited to that. Most such methods will employ antibodies which are specific for the particular protein or protein fragments. The sequences of the mRNA (cDNA) and proteins of the markers of the present invention are known in the art and publicly available.

Methylation-sensitive restriction endonucleases can be used to detect methylated CpG dinucleotide motifs. Such endonucleases may either preferentially cleave methylated recognition sites relative to non-methylated recognition sites or preferentially cleave non-methylated relative to methylated recognition sites. Examples of the former are Acc III, Ban I, BstN I, Msp I, and Xma 1. Examples of the latter are Acc II, Ava I, BssH II, BstU I, Hpa II, and Not I. Alternatively, chemical reagents can be used which selectively modify either the methylated or non-methylated form of CpG dinucleotide motifs.

Modified products can be detected directly, or after a further reaction which creates products which are easily distinguishable. Means which detect altered size and/or charge can be used to detect modified products, including but not limited to electrophoresis, chromatography, and mass spectrometry. Examples of such chemical reagents for selective modification include hydrazine and bisulfite ions. Hydrazine-modified DNA can be treated with piperidine to cleave it. Bisulfite ion-treated DNA can be treated with alkali. Other means which are reliant on specific sequences can be used, including but not limited to hybridization, amplification, sequencing, and ligase chain reaction, Combinations of such techniques can be uses as is desired.

The principle behind electrophoresis is the separation of nucleic acids via their size and charge. Many assays exist for detecting methylation and most rely on determining the presence or absence of a specific nucleic acid product. Gel electrophoresis is commonly used in a laboratory for this purpose.

One may use MALDI mass spectrometry in combination with a methylation detection assay to observe the size of a nucleic acid product. The principle behind mass spectrometry is the ionizing of nucleic acids and separating them according to their mass to charge ratio. Similar to electrophoresis, one can use mass spectrometry to detect a specific nucleic acid that was created in an experiment to determine methylation. See (Tost, J. *et al.* 2003).

One form of chromatography, high performance liquid chromatography, is used to separate components of a mixture based on a variety of chemical interactions between a substance being analyzed and a chromatography column DNA is first treated with sodium bisulfite, which converts an unmethylated cytosine to uracil, while methylated cytosine residues remain unaffected. One may amplify the region containing potential methylation sites via PCR and separate the products via denaturing high performance liquid chromatography (DHPLC). DHPLC has the resolution capabilities to distinguish between methylated (containing cytosine) and unmethylated (containing uracil) DNA sequences. (Deng, D. *et al.* 2002)

Hybridization is a technique for detecting specific nucleic acid sequences that is based on the annealing of two complementary nucleic acid strands to form a double-stranded molecule. One example of the use of hybridization is a microarray assay to determine the methylation status of DNA. After sodium bisulfite treatment of DNA, which converts an unmethylated cytosine to uracil while methylated cytosine residues remain unaffected, oligonucleotides complementary to potential methylation sites can hybridize to the bisulfite-treated DNA. The oligonucleotides are designed to be complimentary to either sequence containing uracil (thymine) or sequence containing cytosine, representing unmethylated and methylated DNA, respectively. Computer-based microarray technology can determine which oligonucleotides hybridize with the DNA sequence and one can deduce the methylation status of the DNA. Similarly primers can be designed to be complimentary to either sequence containing uracil (thymine) or sequence containing cytosine. Primers and probes that recognize the converted methylated form of DNA are dubbed methylation-specific primers or probes (MSP).

An additional method of determining the results after sodium bisulfite treatment involves sequencing the DNA to directly observe any bisulfite-modifications. Pyrosequencing technology is a method of sequencing-by-synthesis in real time. It is based on an indirect bioluminometric assay of the pyrophosphate (PPi) that is released from each deoxynucleotide (dNTP) upon DNA-chain elongation. This method presents a DNA template-primer complex with a dNTP in the presence of an exonuclease-deficient Klenow DNA polymerase. The four nucleotides are sequentially added to the reaction mix in a predetermined order. If the nucleotide is complementary to the template base and thus incorporated, PPi is released. The PPi and other reagents are used as a substrate in a luciferase reaction producing visible light that is detected by either a luminometer or a charge-coupled device. The light produced is proportional to the number of nucleotides added to the DNA primer and results in a peak indicating the number and type of nucleotide present in the form of a pyrogram. Pyrosequencing can exploit the sequence differences that arise following sodium bisulfite-conversion of DNA.

A variety of amplification techniques may be used in a reaction for creating distinguishable products. Some of these techniques employ PCR. Other suitable amplification methods include the ligase chain reaction (LCR) (Barringer *et al*, 1990), transcription amplification (Kwoh et al. 1989; WO88/10315), selective amplification of target polynucleotide sequences (US Patent No. 6,410,276), consensus sequence primed polymerase chain reaction (US Patent No 4,437,975), arbitrarily primed polymerase chain reaction (WO90/06995), nucleic acid based sequence amplification (NASBA) (US Patent Nos. 5,409,818; 5,554,517; 6,063,603), microsatellite length polymorphism (MLP), and nick displacement amplification (WO2004/067726).

Sequence variation that reflects the methylation status at CpG dinucleotides in the original genomic DNA offers two approaches to PCR primer design. In the first approach, the primers do not themselves "cover" or hybridize to any potential sites of DNA methylation; sequence variation at sites of differential methylation are located between the two primers. Such primers are used in bisulfite genomic sequencing, COBRA, Ms-SNuPE. In the second approach, the primers are designed to anneal specifically with either the methylated or unmethylated version of the converted sequence. If there is a sufficient region of complementarity, *e.g*., 12, 15, 18, or 20 nucleotides, to the target, then the primer may also contain additional nucleotide residues that do not interfere with hybridization but may be useful for other manipulations. Exemplary of such other residues may be sites for restriction endonuclease cleavage, for ligand binding or for factor binding or linkers or repeats. The oligonucleotide primers may or may not be such that they are specific for modified methylated residues

One way to distinguish between modified and unmodified DNA is to hybridize oligonucleotide primers which specifically bind to one form or the other of the DNA. After hybridization, an amplification reaction can be performed and amplification products assayed. The presence of an amplification product indicates that a sample hybridized to the primer. The specificity of the primer indicates whether the DNA had been modified or not, which in turn indicates whether the DNA had been methylated or not. For example, bisulfite ions modify non-methylated cytosine bases, changing them to uracil bases. Uracil bases hybridize to adenine bases under hybridization conditions. Thus an oligonucleotide primer which comprises adenine bases in place of guanine bases would hybridize to the bisulfite-modified DNA, whereas an oligonucleotide primer containing the guanine bases would hybridize to the non-modified (methylated) cytosine residues in the DNA. Amplification using a DNA polymerase and a second primer yield amplification products which can be readily observed. Such a method is termed MSP (Methylation Specific PCR; Patent Nos 5,786,146; 6,017,704; 6,200,756). The amplification products can be optionally hybridized to specific oligonucleotide probes which may also be specific for certain products. Alternatively, oligonucleotide probes can be used which will hybridize to amplification products from both modified and nonmodified DNA.

In one particular embodiment, primers useful in MSP carried out on the gene selected from ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655 are provided. Primers of the invention preferably are designed to amplify the genomic sequences in the regions under investigation. Preferred regions may comprise, consist essentially of or consist of the sequences represented by SEQ ID NO. 129-192 and/or SEQ ID NO. 193-256 and/or SEQ ID NO. 315-329 and/or SEQ ID NO. 330-344 and/or SEQ ID NO. 408-428 and/or SEQ ID NO. 429-449 and/or SEQ ID NO. 271-277 and/or SEQ ID NO. 278-284. Preferred sense primers (5' - 3') may comprise, consist essentially of or consist of the sequences represented by SEQ ID NO. 1-64 and/or SEQ ID NO. 285-299 and/or SEQ ID NO. 345-365 and/or SEQ ID NO. 257-263. Preferred antisense primers (5' - 3') comprise, consist essentially of or consist of the sequences represented by SEQ ID NO. 65-128 and/or SEQ ID NO. 300-314 and/or SEQ ID NO. 366-386 and/or SEQ ID NO. 264-270,

Another way to distinguish between modified and nonmodified DNA is to use oligonucleotide probes which may also be specific for certain products. Such probes can be hybridized directly to modified DNA or to amplification products of modified DNA. Oligonucleotide probes can be labeled using any detection system known in the art. These include but are not limited to fluorescent moieties, radioisotope labeled moieties, bioluminescent moieties, luminescent moieties, chemiluminescent moieties, enzymes, substrates, receptors, or ligands.

In one particular embodiment, probes useful in MSP carried out on the gene selected from ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655 are provided. Probes of the invention preferably are designed to bind to genomic sequences in the regions under investigation. Preferred regions may comprise, consist essentially of or consist of the sequences represented by SEQ ID NO. 129-192 and/or SEQ ID NO. 193-256 and/or SEQ ID NO. 315-329 and/or SEQ ID NO. 330-344 and/or SEQ ID NO. 408-428 and/or SEQ ID NO. 429-449 and/or SEQ ID NO. 271-277 and/or SEQ ID NO. 278-284. Preferred probes (5' - 3') may comprise, consist essentially of or consist of the sequences represented by SEQ ID NO. 387-407.

Still another way for the identification of methylated CpG dinucleotides utilizes the ability of the MBD domain of the McCP2 protein to selectively bind to methylated DNA sequences (Cross et al, 1994; Shiraishi et al, 1999). Restriction enconuclease digested genomic DNA is loaded onto expressed His-tagged methyl-CpG binding domain that is immobilized to a solid matrix and used for preparative column chromatography to isolate highly methylated DNA sequences.

Real time chemistry allows for the detection of PCR amplification during the early phases of the reactions, and makes quantitation of DNA and RNA easier and more precise. A few variations of the real-time PCR are known. They include the TaqMan™ (Roche Molecular Systems) system and Molecular Beacon™ system which have separate probes labeled with a fluorophore and a fuorescence quencher. In the Scorpion™ system the labeled probe in the form of a hairpin structure is linked to the primer. In addition, the Amplifluor™ (Chemicon International) system and the Plexor™ (Promega) system can be used.

DNA methylation analysis has been performed successfully with a number of techniques which include the MALDI-TOFF, MassARRAY, MethyLight, Quantitative analysis of ethylated alleles (QAMA), enzymatic regional methylation assay (ERMA), HeavyMethyl, QBSUPT, MS-SNuPE, MethylQuant, Quantitative PCR sequencing, and Oligonucleotide-based microarray systems.

Subsets of genes for all aspects and embodiments of the invention include ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655. By "gene" is meant any gene which is taken from the family to which the named "gene" belongs and includes according to all aspects of the invention not only the particular sequences found in the publicly available database entries, but also encompasses transcript and nucleotide variants of these sequences, with the proviso that methylation or another epigenetic modification of the gene is linked to lung cancer. The number of genes whose modification is tested and/or detected can vary: one, two, three, four, five, or more genes can be tested and/or detected. In some cases at least two genes are selected. In other embodiments at least three genes are selected.

Testing can be performed diagnostically or in conjunction with a therapeutic regimen. Testing can be used to monitor efficacy of a therapeutic regimen, whether a chemotherapeutic agent or a biological agent, such as a polynucleotide. Testing can also be used to determine what therapeutic or preventive regimen to employ on a patient. Moreover, testing can be used to stratify patients into groups for testing agents and determining their efficacy on various groups of patients. The detection may also link to a cancer stage or grade. The "Stage" refers to how far a cancer has progressed anatomically, while the "grade" refers to cell appearance (differentiation) and DNA make up.

Test samples and normal samples for diagnostic, prognostic, or personalized medicine uses can be obtained from surgical samples, such as biopsies or fine needle aspirates, from paraffin embedded lung, or other organ tissues, from a body fluid such as blood, serum, lymph, saliva, sputum, urine, pleural fluid, bronchoalveolar lavage fluid. Such sources are not meant to be exhaustive, but rather exemplary. A test sample obtainable from such specimens or fluids includes detached tumor cells and/or free nucleic acids that are released from dead or damaged tumor cells. Nucleic acids include RNA, genomic DNA, mitochondrial DNA, single or double stranded, and protein-associated nucleic acids. Any nucleic acid specimen in purified or non-purified form obtained from such specimen cell can be utilized as the starting nucleic acid or acids. The test samples may contain cancer cells or pre-cancer cells or nucleic acids from them. For example the sample may contain lung adenoma cells, lung advanced adenoma cells, or lung adenocarcinoma cells. Samples may contain squamous cells or large cell carcinoma. Samples may contain mixtures of different types and stages of lung cancer cells.

The test sample is generally obtained from a (human) subject suspected of being tumorigenic. Alternatively the test sample is obtained from a subject undergoing routine examination and not necessarily being suspected of having a disease. Thus patients at risk can be identified before the disease has a chance to manifest itself in terms of symptoms identifiable in the patient. Alternatively the sample is obtained from a subject undergoing treatment, or from patients being checked for recurrence of disease.

Demethylating agents can be contacted with cells *in vitro* or *in vivo* for the purpose of restoring normal gene expression to the cell. Suitable demethylating agents include, but are not limited to 5-aza-2'-deoxycytidine, 5-aza-cytidine, Zebularine, procaine, and L-ethionine. This reaction may be used for diagnosis, for determining predisposition, and for determining suitable therapeutic regimes.

Although diagnostic and prognostic accuracy and sensitivity may be achieved by using a combination of markers, such as 5 or 6 markers, or 9 or 10 markers, practical considerations may dictate use of smaller combinations. Any combination of markers for a specific cancer may be used which comprises 2, 3, 4, or 5 markers. Combinations of 2, 3, 4, or 5 markers can be readily envisioned given the specific disclosures of individual markers provided herein. Preferably, the invention involves detecting an epigenetic change in a panel of genes comprising a combination of 2, 3, 4 or 5 markers. Preferably, the panel comprises RASSF1A and/or SOX17 and/or HS3ST2-nor and/or NID2 and/or SFRP 1.

Kits according to the present invention are assemblages of reagents for testing methylation. They are typically in a package which contains all elements, optionally including instructions. The package may be divided so that components are not mixed until desired. Components may be in different physical states. For example, some components may be lyophilized and some in aqueous solution. Some may be frozen. Individual components may be separately packaged within the kit. The kit may contain reagents, as described above for differentially modifying methylated and non-methylated cytosine residues. Desirably the kit will contain oligonucleotide primers which specifically hybridize to regions within 3 kb of the transcription start sites of the genes/markers: ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655. Additional markers may be used. Typically the kit will contain both a forward and a reverse primer for a single gene or marker. If there is a sufficient region of complementarity, *e.g*., 12, 15, 18, or 20 nucleotides, then the primer may also contain additional nucleotide residues that do not interfere with hybridization but may be useful for other manipulations. Exemplary of such other residues may be sites for restriction endonuclease cleavage, for ligand binding or for factor binding or linkers or repeats. The oligonucleotide primers may or may not be such that they are specific for modified methylated residues. The kit may optionally contain oligonucleotide probes. The probes may be specific for sequences containing modified methylated residues or for sequences containing non-methylated residues. The kit may optionally contain reagents for modifying methylated cytosine residues. The kit may also contain components for performing amplification, such as a DNA polymerase (particularly a thermostable DNA polymerase) and deoxyribonucleotides. Means of detection may also be provided in the kit, including detectable labels on primers or probes. Kits may also contain reagents for detecting gene expression for one of the markers of the present invention (Table 1 and Table 3). Such reagents may include probes, primers, or antibodies, for example. In the case of enzymes or ligands, substrates or binding partners may be sued to assess the presence of the marker. Kits may contain 1, 2, 3, 4, or more of the primers or primer pairs of the invention. Kits that contain probes may have them as separate molecules or covalently linked to a primer for amplifying the region to which the probes hybridize. Other useful tools for performing the methods of the invention or associated testing, therapy, or calibration may also be included in the kits, including buffers, enzymes, gels, plates, detectable labels, vessels, etc.

In one aspect of this embodiment, the gene is contacted with hydrazine, which modifies cytosine residues, but not methylated cytosine residues, then the hydrazine treated gene sequence is contacted with a reagent such as piperidine, which cleaves the nucleic acid molecule at hydrazine modified cytosine residues, thereby generating a product comprising fragments. By separating the fragments according to molecular weight, using, for example, an electrophoretic, chromatographic, or mass spectrographic method, and comparing the separation pattern with that of a similarly treated corresponding non-methylated gene sequence, gaps are apparent at positions in the test gene contained methylated cytosine residues. As such, the presence of gaps is indicative of methylation of a cytosine residue in the CpG dinucleotide in the target gene of the test cell.

Bisulfite ions, for example, sodium bisulfite, convert non-methylated cytosine residues to bisulfite modified cytosine residues. The bisulfite ion treated gene sequence can be exposed to alkaline conditions, which convert bisulfite modified cytosine residues to uracil residues. Sodium bisulfite reacts readily with the 5,6-double bond of cytosine (but poorly with methylated cytosine) to form a sulfonated cytosine reaction intermediate that is susceptible to deamination, giving rise to a sulfonated uracil. The sulfonate group can be removed by exposure to alkaline conditions, resulting in the formation of uracil. The DNA can be amplified, for example, by PCR, and sequenced to determine whether CpG sites are methylated in the DNA of the sample. Uracil is recognized as a thymine by Taq polymerase and, upon PCR, the resultant product contains cytosine only at the position where 5-methylcytosine was present in the starting template DNA. One can compare the amount or distribution of uracil residues in the bisulfite ion treated gene sequence of the test cell with a similarly treated corresponding non-methylated gene sequence. A decrease in the amount or distribution of uracil residues in the gene from the test cell indicates methylation of cytosine residues in CpG dinucleotides in the gene of the test cell. The amount or distribution of uracil residues also can be detected by contacting the bisulfite ion treated target gene sequence, following exposure to alkaline conditions, with an oligonucleotide that selectively hybridizes to a nucleotide sequence of the target gene that either contains uracil residues or that lacks uracil residues, but not both, and detecting selective hybridization (or the absence thereof) of the oligonucleotide.

Test compounds can be tested for their potential to treat cancer. Expression of a gene selected from those listed in Table 1 and Table 3 is determined and if it is increased or decreased by the compound in the cell or if methylation of the gene is decreased or increased by the compound in the cell, one can identify it as having potential as a treatment for cancer. The candidate compound will have the effect of reversing the expression/or methylation modification found in the cancer cell.

The above disclosure generally describes the present invention. All references disclosed herein are expressly incorporated by reference. A more complete understanding can be obtained by reference to the following specific examples which are provided herein for purposes of illustration only, and are not intended to limit the scope of the invention.

### EXAMPLES

### EXAMPLE 1: Selection of candidate genes

Using re-expression profiles of lung cancer cell lines, candidate genes were identified and the most promising markers were tested on tissue using the Base5 methylation profiling platform (Straub et al. 2007). Differential methylation of the particular genes was assessed using Base5 methylation profiling platform as follows: DNA was extracted from lung samples, bisulfite converted, and selected regions of the particular genes were amplified using primers whose sequence represented converted or non-converted DNA sequences. Amplification was monitored in real-time set up using cybergreen. Two robust data analyses designed to cope with inherent variance (*i.e.,* noise) in measured Ct and Tm values were applied to withhold 64 different assays for detecting differential methylation of ACSL6, ALS2CL, APC2, BEX1, BMP7, CBR3, CD248, CD44, CHD5, DLK1, DPYSL4, DSC2, EPB41L3, EPHB6, ERBB3, FBLN2, FBN2, FOXL2, GSTP1, HS3ST2, IGFBP7, IRF7, JAM3, LOX, LY6D, LY6K, MACF1, MCAM, NEFH, NID2, PCDHB15, PHACTR3, POMC, PRKCA, PSEN1, RBP1, RRAD, SFRP1, SOD3, SOX17, SULF2, TIMP3, TJP2, TRPV2, UCHL1, WDR69, ZFP42, ZNF442, and ZNF655 in lung cancer tissue samples.

### Materials and methods

### Strategy to identify supplementary gene targets for lung cancer

Promoter sequences were linked with gene expression to identify epigenetically silenced genes. An established pharmacologic unmasking strategy (5-aza-2'-deoxycytidine [DAC] and trichostatin A [TSA]) for re-expression analysis of epigenetically targeted genes was combined with proprietary advanced bioinformatics tools to identify genes prone to promoter methylation. To identify differentially methylated markers associated with non-small cell lung cancer (NSCLC), the information derived from 11 cell lines (ATCC, at domain lgcpromochem-atcc.com/, 2006) was used:
1. NCI-H23: adenocarcinoma, cell line derived from a smoker
2. NCI-H1568: adenocarcinoma, cell line derived from a smoker
3. NCI-H1993: adenocarcinoma, cell line derived from a smoker
4. NCI-H2023: adenocarcinoma, cell line derived from a non-smoker
5. NCI-H2085: adenocarcinoma, cell line derived from a non-smoker
6. NCI-H2228: adenocarcinoma, cell line derived from a non-smoker
7. NCI-H520: squamous cell carcinoma, cell line
8. NCI-H838: adenocarcinoma, cell line derived from a smoker
9. NCI-H2170: squamous cell carcinoma, cell line derived from a non-smoker
10. NCI-H1869: squamous cell carcinoma, cell line derived from a smoker
11. SK-MES-1: squamous cell carcinoma, cell line

Cell culture, microarray and data analysis was done as described in Schuebel *et* al, 2007. In short, the cell lines were cultured with 5-aza-2'-deoxycytidine (AZA) and with trichostatin A (TSA) in parallel. Control cells underwent mock treatment. Total RNA was harvested from AZA-, TSA- and mock-treated cells. Amplification and labeling of the RNA were carried out using the Low RNA Input Linear Amplification kit (Agilent Technologies). The complementary labeled RNA was hybridized and processed according the Agilent microarray protocol. All calculations and normalizations of the expression data were performed using the R statistical computing platform (Ihaka *et al.,* 1996) and packages from Bioconductor bioinformatics software (Gentleman *et al.,* 1996).

A gene was selected as a good candidate if it met the following criteria:
1. Re-expressed under AZA treatment; a gene was termed as a top tier gene if the expression is up regulated by more than two-fold in the AZA treated versus mock sample on the Agilent whole human genome expression microarray platform; if it showed an enrichment between 1.4 and 2 fold it was termed as a next tier gene
2. Silent, *i.e.,* having no basal expression, in the mock cells
3. No response to TSA treatment alone

Following this initial candidate selection, 2 main strategies were taken to further select good gene candidates susceptible to hypomethylation and/or hypermethylation: a computational strategy and a verification strategy based on cell lines and primary tumors.

### Computational Strategy

This strategy was applied on the top and next tier genes of the first 6 cell lines (NCI-H23, NCI-H1568, NCI-H1993, NCI-H2023, NCI-H2085, and NCI-H2228).

Different steps were taken towards identification of good candidate genes susceptible to hypomethylation and/or hypermethylation:

Step 1: The promoters of all the selected and clearly annotated top tier genes were separately mapped on the genome-wide alignment of all promoter associated CpG islands. The genes were selected if they were located less than 9 ancestral nodes from an established list of 56 markers (see BROAD analysis). Using this approach, 100 genes were identified.

BROAD analysis: Genome-wide Promoter Alignment

The "Database of Transcription Start Sites" (DBTSS) (Suzuki *et al.,* 2004) mapped each transcript sequence on the human draft genome sequence to identify its transcriptional start site, providing more detailed information on distribution patterns of transcriptional start sites and adjacent regulatory regions. From ∼14,500 well-characterized human genes present in the Affymetrix GeneChip Human Genome U133A Arrays 8793 sequences were extracted from the DBTSS [5, 6] (DBTSS, version 3.0 based on human assembly build 31). The remaining genes (14,500-8793=5707) on the Affymetrix array contained no reported transcriptional start site (TSS) according to DBTSS. All the promoter sequences were subsequently aligned by clustalw algorithm (Li 2003; Thompson *et al.*, 1994) Treeillustrator (Trooskens *et al.,* 2005) was used to visualize the large guide tree in addition to indicating the location of the known markers. Some regions on the "circle" are denser in known markers than others, indicating that there might be a sequence mechanism located in the small region around the TSS which makes certain genes more methylation-prone.

Step 2: As shown by Schuebel *et al.* and based on the sequencing project from Sjöblom *et al.* (Sjöblom *et al.*, 2006), promoter CpG island methylation and subsequent gene silencing of genes known to be mutated in cancer is more frequent than the mutations themselves. Therefore the genes identified by Sjöblom *et al.* were used to identify possible extra targets from the top or next tiers with a known genetic background in either colon or breast cancer. Taking into account all 6 cell lines, 22 extra genes were found to adhere to this category.

Step 3: A final batch of genes was selected based on their appearance in multiple top tiers of the colorectal cell lines from Schuebel *et al.* and at least one top tier of the lung cancer cell lines. The same approach was used based on multiple breast cancer cell lines, i.e. MDA-MB-231, MDA-MB-468, MCF7 and T-47D. The next tiers of the breast cancer cell lines were also used, since the overlap between multiple top tiers of these breast cancer cell lines and the top tiers of the lung cancer cell lines was minimal compared to the overlap with the colon cancer cell lines. Sixteen genes were selected out of the colon screen and another 17 out of the breast screen.

After removing the duplicates of genes obtained by these different approaches, a list of in total 144 genes was identified by this strategy.

### Verification Strategy

This strategy was applied on a selection of the top and next tier genes of 4 adenocarcinoma cell lines (NCI-H23, NCI-H1568, NCI-H1993, and NCI-H838) and 4 squamous cell carcinoma cell lines (NCI-H520, NCI-H2170, NCI-H1869, and SK-MES-1). These genes were verified in cell lines and/or primary tumors and normal lung samples for expression by reverse transcription-PCR and promoter methylation by MSP. Using this strategy, a list of in total 63 was identified.

Duplicates, imprinted genes and genes for which primer design was not possible were excluded from both lists. This final selection of genes was further analyzed on the Base5 methylation profiling platform (Straub et al. 2007).

### Sample specimen

A total of 132 samples (64 lung cancer samples, the majority derived from lung adenocarcinoma and sqaumous cell carcinomas; and 68 corresponding normal tissues) were used to find markers which distinguish cancer from non-cancer tissue based on methylation status.

### DNA extraction and Bisulfite modification

A high throughput, real-time methylation specific detection platform was applied on two groups of samples totaling 132 genomic DNA samples. The two groups of samples consisted of 64 samples isolated from lung cancer tissue and 68 samples isolated from corresponding normal lung tissue.

From each sample, up to 1 µg of genomic DNA was converted using a bisulfite based protocol (EZ DNA Methylation Kit™, ZYMO Research, Orange, CA.).

### Detection of hypermethylation

After conversion and purification the equivalent of 25-75ng of the starting material was applied per sub-array of an OpenArray™ plate on a real-time qPCR system (BioTrove Inc.) using the DNA double strand-specific dye SYBRgreen for signal detection.

The cycling conditions were: 90°C-10 seconds, (43°C 18 seconds, 49°C 60 seconds, 77°C 22 seconds, 72°C 70seconds, 95°C 28 seconds) for 40 cycles, 70°C for 200 seconds, 45°C for 5 seconds. A melting curve was generated in a temperature range between 45°C and 94°C. Methylation specific PCR (MSP) primers were designed for each of the genes assessed for hypermethylation.

### Analysis of methylation

For each combination of assays and samples two parameters were collected using an algorithm which is part of the standard data analysis package offered by the supplier. The parameters were the Ct value (threshold cycle number) of the assessed amplicon and the melting temperature of the assessed amplicon.

The following data analysis workflow was applied to the results created by the software which came with the system OpenArray™ system. Data was collected for each combination of assays and samples in the two sets of samples used. Results were filtered using the following approach. Read outs from not loaded reaction spaces were removed from analysis. Technical Control assays were removed from the data set. Assays known to not work for other than biological reasons were removed from the analysis. Samples for which Ct calls for the gene beta-Actin were not present were removed from the analysis. Ct values > 0 for each gene were normalized using the Ct values collected for the gene beta-Actin. This resulted in two files containing the results for each set of sample.

Two robust data analyses designed to cope with inherent variance (*i.e*., noise) in measured Ct and Tm values were applied which have common features and data analysis steps. Based on the original data, a p-value was assigned to each marker that corresponds to the probability of obtaining Ct/Tm values at least as favorable assuming these values were the result of chance alone. Next, robustness of the above p-value was computed by introducing increasing levels of noise in the data and recomputing the p-value (pVal) as above. The noise level on the x-axis was plotted against (1- pVal) on the y-axis, and the area under the resulting curve was used as the final score for a particular marker. With robust markers, the initial p-value survives for a while, hence (1-pVal) will stay high for a while, hence the area under the curve (AUC) will tend to be high. With not-so-robust markers an initial (1-pVal) will drop quickly with increasing noise levels on the x-axis, which will result in a lower AUC.

The two analysis methods, called "Ranks" and "Squares," differ only in the way the p-values for each noise level are applied.

### The "Ranks" method

For computing p-values with the Ranks method for a particular marker, four lists of ranks of samples are generated: two based on the Ct values determined for each assay applied to all samples (cancer samples as well as non cancer samples) resulting in one ascending list of ranks and on one descending list of ranks; and two based on the Tm values determined for each assay applied to all samples (cancer samples as well as non-cancer samples) resulting in one ascending list of ranks and on one descending list of ranks.

For each of these four lists of ranks, the sum of the ranks of the cancer samples are calculated. The lowest of these four sums is kept. Depending on this lowest sum, we label the marker as a positive/negative Ct/Tm marker. For instance, if the lowest sum is found with the descending Ct ranking, we label the marker as a negative Ct ranker; alternatively, in case the lowest sum is found with the descending Tm ranking, the marker is labeled as a positive Tm ranker.

Next, the rank sum of the cancers is recorded for 10,000 random rankings. The fraction of cases where this sum is at least as low as the rank sum of the cancers in the original ranking is taken to be the p-value.

In order to asses the correlation between added noise and resulting p-values, random noise is introduced into the list Ct values and Tm values and the ranking procedure is repeated. This process resulted in a series of p-values with increasing levels of noise which was used to determine an AUC score. Assays are ranked based on their AUC from high to low.

### The "Squares" method

Applying this method, a lower and/or upper limit is imposed on the Ct and/or Tm values determined for all samples. Such limits correspond to a "square" imposed on the scatter plot of samples where Ct forms the x-axis and Tm forms the y-axis. When considering all possible squares in this scatter plot, we are in fact exploring all combinations of a lower and/or upper limit in the Ct dimension on the one hand and the Tm dimension on the other hand. The sensitivity and specificity for the detection of cancers is determined for the set of all possible squares as defined above.

Next, for each square, the p-value is computed using the Fisher exact test. The square resulting in the highest sensitivity and specificity for determining methylation in cancer and normal samples can thus be determined for each marker candidate.

To test quality of the best square, an increasing amount of noise is injected as described above, and the p-value is recomputed using the Fisher exact test. When plotting the correlation between injected noise and the resulting p-values, the AUC can be determined. The most optimal square will result in the highest AUC. Assays are ranked based on the maximal AUC achievable.

The results of the applied analysis methods are "zipped" together in the following way. The results of applying the two analysis methods described above to two different sample sets are included into four different lists called "sample_set_1_ranks", "sample_set_2_ranks", "sample_set_1_squares", and "sample_set_2_squares"

A new "zipped" list is created by taking the highest scoring assay from the list "sample_set_1_ranks," followed by a comparison of the highest scoring assay from list "sample_set_2_ranks." If the marker is already present in the zipped list, this finding is noted and the next highly scoring marker of the list "sample_set_2_ranks" is used. This selection procedure is applied comparing the highest scoring assay of lists "sample set_1_squares," noting down if the assay already has scored in the zipped list up to this step. The "sample_set_2_squares" list is used as the source for the next markers in the zipped list. The sequence of lists is maintained until all the assays in all the lists have been assessed.

The cut-offs 0.832, 0.909, 0.687 and 0.743 were applied on the "AUC" determined for each assay and rank in the lists sample_set_1_ranks, sample_set_2_ranks, sample_set_1_squares, and sample_set_2_squares. This resulted in 10 different genes.

### Results

A high throughput, real-time methylation specific detection platform was applied on two groups of samples isolated from lung cancer tissue and from corresponding normal lung tissue. In this study it was shown that a number of genes are differentially methylated in lung cancer, in particular in non-small cell lung cancer, more particularly in lung adenocarcinoma or squamous cell carcinoma. We identified 64 different assays for detecting 49 different genes being differentially methylated in human lung cancer tissue and normal lung tissue control samples. The genes identified are ACSL6, ALS2CL, APC2, BEX1, BMP7, CBR3, CD248, CD44, CHD5, DLK1, DPYSL4, DSC2, EPB41L3, EPHB6, ERBB3, FBLN2, FBN2, FOXL2, GSTP1, HS3ST2, IGFBP7, IRF7, JAM3, LOX, LY6D, LY6K, MACF1, MCAM, NEFH, NID2, PCDHB15, PHACTR3, POMC, PRKCA, PSEN1, RBP1, RRAD, SFRP1, SOD3, SOX17, SULF2, TIMP3, TJP2, TRPV2, UCHL1, WDR69, ZFP42, ZNF442, ZNF655.

The resulting assays have the assay details provided in Table 1.

**Table 1. Methylation Specific PCR (MSP) primers used for the 64 assays:**

| # | **Assay Name** | **Entrez GeneID** | **Official Gene symbol** | **Sense primer sequence (SEQ ID NO:1.64, respectively)** | **Antisense primer sequence (SEQ ID NO:65-128, respectively)** |
|---|---|---|---|---|---|
| 1 | ACSL6_17822 | 23305 | ACSL6 | TTTAATGTTACGTTTTGGCGTT | GAACCAACCCTCTCCGACC |
| 2 | ACSL6_17824 | 23305 | ACSL6 | GCGGTTGTAAGGTTTTTGGTC | ATTTTTCCGCAACCTCTCG |
| 3 | ALS2CL_bay | 259173 | ALS2CL | GGACGGGTGTTTGCGTTTTAC | CGAAACCAAAAAACTAAACGAAAACCG |
| 4 | APC2 | 10297 | APC2 | GTCGTTTGTTTAGGTTCGGATC | GACCCGAAATAACCTCGAAACG |
| 5 | BEX1_12842 | 55859 | BEX1 | TCGGGGTTTTTATTTGGTTC | AATCGTCACTCGTATCTCGCT |
| 6 | BMP7_17905 | 655 | BMP7 | GTACGTGCGTTTATTGCGAG | CGTTATCCAAACTAAAATCGACC |
| 7 | CBR3_17931 | 874 | CBR3 | GGTATCGGTTTGGTTATCGC | CGCCTACAACTACTACACGACC |
| 8 | CBR3_17935 | 874 | CBR3 | GTTTTCGATTGATTTATTAAGGTTC | TCAAAATCCGAACTCTAAACCG |
| 9 | CD248_17939 | 57124 | CD248 | TCGTGGGAAGAGAGCGTAG | TTACTAACCTAAACGACGGCAA |
| 10 | CD24B_17946 | 57124 | CD248 | TTTTGTTAAGAGTTGTCGTTAGTTC | AATATAAACCCTACGACCGCC |
| 11 | CD248_17947 | 57124 | CD248 | GGGGTAGTCGTTAATTGCGT | TCTTCCCCGAAAACCGCTA |
| 12 | CD44_17961 | 960 | CD44 | CGGGAGAAGAAAGT7AGTGCGT | AAATCGAAAAACCTAAAATATCGC |
| 13 | CHD5_bay | 26038 | CHD5 | GAGCGTTCGGGTTTTGC | CGACCTCGACGAAAAAATAACG |
| 14 | CRBP_1 | 5947 | RBP1 | TTGGGAATTTAGTTGTCGTCGTTTC | AAACAACGACTACCGATACTACGCG |
| 15 | DLK1_18031 | 8788 | DLK1 | GAGGTTTGCGGTTTAGGTTC | CTCACACTATACAACACGCGAC |
| 16 | DLK1_18033 | 8788 | DLK1 | GGAGTTGGGGTTTACGAGAC | ATAATAAATTCCCCGACGACC |
| 17 | DPYSL4_18047 | 10570 | DPYSL4 | GGTGTTTTGATAGAAGTCGTTAGTC | AAAACCATTAACGCCCACG |
| 18 | DPYSL4_18050 | 10570 | DPYSL4 | GGGGTTATAGTTTGGCGTTC | GCTCTAAAAACCACACCCGTC |
| 19 | DSC2_18056 | 1824 | DSC2 | GGTTTCGGTTTCGTTTTGTTC | CTCTACGACTCAAACCTCGCT |
| 20 | EPB41L3_19071 | 23136 | EPB41L3 | GGGATAGTGGGGTTGACGC | ATAAAAATCCCGACGAACGA |
| 21 | EPB41L3_19072 | 23136 | EPB41L3 | GCGTGGGTTTTCGTCGTAG | CCCAAAACTACTCGCCGCT |
| 22 | EPHB6_bay | 2051 | EPH86 | GGGTGTTCGATTTAAGTCGAGTTC | CGCGAATCTTAACCGAAAAAATCG |
| 23 | ERBB3_18097 | 2065 | ERBB3 | GTTTAGTTAAGTTCGGTTCGGG | GATTACAATTTACAACCTCCGCT |
| 24 | ERBB3_18099 | 2065 | ERBB3 | AGGGAGTTTAGTTAAGTTCGGTTC | TACAACCTCCGCTACCGTC |
| 25 | FBLN2_13328 | 2199 | FBLN2 | TAGAGCGGAGGAAGTTGCG | CAAATACGAACACAAAAACCGA |
| 26 | FBN2_18150 | 2201 | FBN2 | TCGGAGTTTTATAGGGTAACGAA | CTCTTACTAACCGCACGCC |
| 27 | FBN2_18151 | 2201 | FBN2 | TTGGAGATTTCGATAGAGCGT | AAACTACCGACTACACCTCCG |
| 28 | FOX-L2 | 668 | FOXL2 | GCGATAGGTTTTTAGTAAGTAAGCGC | CTCTCCGCTCCAAACGCTAACGCG |
| 29 | Gst-Pi | 2950 | GSTP1 | TTCGGGGTGTAGCGGTCGTC | GCCCCAATACTAAATCACGACG |
| 30 | HS3ST2_19130 | 9956 | HS3ST2 | ACGTAAGAGTTTGGGAGCGT | GACTCCTCGAAAAACAAACGA |
| 31 | HS3ST2_19131 | 9956 | HS3ST2 | GTTTCGGGGTTCGTTTTTC | CGACTCGCTCTATCTCGCAC |
| 32 | IGFBP7_19196 | 3490 | IGFBP7 | TTTGTCGGCGTCGTTATTTTC | AAACTACCTACTAAACGAAACCCG |
| 33 | IGFBP7_19200 | 3490 | IGFBP7 | CGTTTATGGGTCGGTTACGTC | ATAAAAACACGAAAACCCCGC |
| 34 | IRF7_18346 | 3665 | IRF7 | AGTTGAGAATCGGACGGGG | AACGAATCAAACTCCCGAAA |
| 36 | JAM3 | 83700 | JAM3 | GGGATTATAAGTCGCGTCGC | CGAACGCAAAACCGAAATCG |
| 36 | LOX_18967 | 4015 | LOX | GCGCGTAGAGTTGTAAAGGTTC | ACGTCCTCCTCGAACGAAA |
| 37 | LOX_18977 | 4015 | LOX | GGTAGAGGCGAGGAGTTGTTC | TACACAAACCGTTCTAACCCGA |
| 38 | LY6D_18402 | 8581 | LY6D | GATGTCGTTTGGGAGTAGTGC | ACAAAATACCGCTAACTAACGAA |
| 39 | LY6K | 54742 | LY6K | GCGGGGTTTTTTTTATCGGTTAGATTC | CAACGATACCCAAAAAAAATCAACGCG |
| 40 | MACF1_bay | 23499 | MACF1 | GTTTTCGTTGTCGTTACGGGTTC | GCGCAACGAACAAAACG |
| 41 | MCAM | 4162 | MCAM | AGAATTTAGGTCGGTTTTTATCG | ACGCAAAA TTCTTCTCCCAAAA |
| 42 | NEFH_18452 | 4744 | NEFH | GTCGGATGAAGTATTCGGG | CCCTACAAACGACGACGAAC |
| 43 | NID2_9093 | 22795 | NID2 | TTATTTCGTTTTTAGGGAGTTTTC | CTTACGAACCATTTAATCCCG |
| 44 | NID2_9094 | 22795 | NID2 | TTTCGTGTGGGAAGAGTTCGT | CGAATAACCGAACGACCGATA |
| 45 | PCDHB15_10763 | 56121 | PCDHB15 | TTTTGGTTATTAGGTAGTTCGGTTC | CACTCTTCGTACTATTCCCGCT |
| 46 | PHACTR3_11692 | 116154 | PHACTR3 | TTATTTTGCGAGCGGTTTC | GAATACTCTAATTCCACGCGACT |
| 47 | POMC | 5443 | POMC | GATTTGGGCGTTTTTGGTTTTTCGC | GACTTCTCATACCGCAATCG |
| 48 | PRKCA_18626 | 5578 | PRKCA | GGGCGTTGAGGTAGAAGAAC | CGACACCTACCAAATAAAATCG |
| 49 | PSEN1_16648 | 5663 | PSEN1 | TTAGGTCGGAGGTTTCGTTT | AAACCCTCACCGTTATCGTC |
| 50 | RRAD_18698 | 6236 | RRAD | GATGTTTCGGTCGAGGTTTC | AAACGACTACAAATAAATACGCCA |
| 51 | SFRP1 | 6422 | SFRP1 | TGTAGTTTTCGGAGTTAGTGTCGCGC | CCTACGATCGAAAACGACGCGAACG |
| 52 | SPRP1_9381 | 6422 | SFRP1 | TTTTGTTCGTCGTATTTTCGG | ATAACGACCCTCGACCTACGAT |
| 53 | SOD3_18740 | 6649 | SOD3 | AGTATAGAGTGGGGAGCGTAGC | CTTTCCTACCACCGAAACGA |
| 54 | SOX17 | 64321 | SOX17 | TTGCGTTAGTCGTTTGCGTTC | CAAAAACGAATCCCGTATCCGACG |
| 55 | SULF2_bay | 55959 | SULF2 | GTTAGTCGAGTTCGGAGGTATC | CAACTCCGAACGAAACAATAAACG |
| 56 | TIMP3 | 7078 | TIMP3 | GCGTCGGAGGTTAAGGTTGTT | CTCTCCAAAATTACCGTACGCG |
| 57 | TJP2_18792 | 9414 | TJP2 | CGGGTTAGAGTATTGTTCGGT | GAACACAAATCCCGCGTAA |
| 58 | TJP2_18797 | 9414 | TJP2 | GATTTTATCGGGGAAATATCG | AAACAAATCCCGCTCCGAA |
| 59 | TRPV2_18803 | 51393 | TRPV2 | TTATTTCGTAGGTTGAGGTTAGGGC | TCCTCTACTATCAACGCCGAC |
| 60 | UCHL1 | 7345 | UCHL1 | GTTGTATTTTCGCGGAGCGTTC | CTCACAATACGTCTAACCGACG |
| 61 | WDR69_18844 | 164781 | WDR69 | GTTTAGGTTGTGGTTTAGGTCGTC | ACACCTCGTATCCTCACTAAAAACG |
| 62 | ZFP42_bay | 132625 | ZFP42 | GGGGTTTFTAGGTATTCGGTTCGTAC | AATACGCAATACCCGACGACCG |
| 63 | ZNF442_bay | 79973 | ZNF442 | TCGGTTTTTAGTTTTTTCGGTCGC | CAATTACTACGCAAAAACGAAACAAAACG |
| 64 | ZNF655 | 79027 | ZNF655 | TTATCGAGAAGCGTCGGTTTC | ACCGAAAAAAAAAACGAACCTAACCG |

**Table 2: Amplicon details**

| Amplicon details (converted sequence): | | | | |
|---|---|---|---|---|
| | Assay Name | Entrez GeneID | Official Gene symbol | Amplicon Sequence (converted) (SEQ ID NO:129-192, respectively) |
| 1 | ACSL6_1782 2 | 23305 | ACSL6 | TTTAATGTTACGTTTTGGCGTTCGTCGTTCGTGTTTTTTTTTTTAGTCGGTTTTCGTAGAATGTTAGGTATTG ACGTTGGAGAGCGGGGTCGGAGAGGGTTGGTTC |
| 2 | ACSL6_1782 4 | 23305 | ACSL6 | GCGGTTGTAAGGTTTTTGGTCGGTGAGTGAATTAGTAGGTAAGGATGGTAGTTAGGGTATTTATATTTACG AGGGTGGTGGTCGAGAGGTTGCGGAAAAAT |
| 3 | ALS2CL_bay | 259173 | ALS2CL | GGACGGGTGTTTGCGTTTTACGTTTAGTTCGTTTAGGTGGGGGTTTTCGTTTTTTCGGTTGTTGCGGTTTT CGTTTAGTTTTTTGGTTTCG |
| 4 | APC2 | 10297 | APC2 | GTCGTTTGTTTAGGTTCGGATCGGGTTTTGTTCGTTTCGGAGTTTTTGTTCGCGTCGCGGAGATTTCGGAG TTCGCGCGTTTCGAGGTTATTTCGGGTC |
| 5 | BEX1_12842 | 55859 | BEX1 | TCGGGGTTTTTATTTGGTTCGTTTTTTTTCGGGTCGGATGTTAGTTCGTCGAGCGTAGGGTAGCGGGGAGT TGGTAGCGAGATACGAGTGACGATT |
| 6 | BMP7_1790 5 | 655 | BMP7 | GTACGTGCGTTTATTGCGAGTTGCGGCGTCGTATAGTTTCGTGGCGTTTTGGGTATTTTTGTTTTTGTTGC GTTTCGTTTTGGTCGATTTTAGTTTGGATAACG |
| 7 | CBR3_1793 1 | 874 | CBR3 | GGTATCGGTTTGGTTATCGCGCGCGAATTGTGTCGATAGTTTTTTGGGGATGTGGTGTTTATCGCGCGGG ACGTGGCGCGGGGTTAGGCGGTCGTGTAGTAGTTGTAGGCG |
| 8 | CBR3_17935 | 874 | CBR3 | GTTTTCGATTGATTTATTAAGGTTCGATTTGGTTTCGGATATTTCGTAGATTATTTCGCGGTTTAGAGTTCG GATTTTGA |
| 9 | CD248_1793 9 | 57124 | CD248 | TCGTGGGAAGAGAGCGTAGTAGTTGTTGGGGTCGTAGGCGGTACGGGGTTTAGTAGTTTAGGGGTTTTG GTTTAGTGTGGGTTTTGCGGTCGTTTAGGTTAGTAA |
| 10 | CD248_1794 6 | 57124 | CD248 | TTTTGTTAAGAGTTGTCGTTAGTTCGGGGTCGGATTAGTTCGGGGGTATCGCGATGTTGTTGCGTTTGTTG TTGGTTTGGGCGGTCGTAGGGTTTATATT |
| 11 | CD248_1794 7 | 57124 | CD248 | GGGGTAGTCGTTAATTGCGTTTTTTTTTTTTTTTCGTTTTTAATTTTAGAGTTTTTTATTTTATTGTTTTTTGTT TTAGCGGTTTTCGGGGAAGA |
| 12 | CD44_17961 | 960 | CD44 | CGGGAGAAGAAAGTTAGTGCGTTTTTGGGCGTAGGGGTTAGTGGGGTTCGGAGGTATAGGTATTTCGCG ATATTTTAGGTTTTTCGATTT |
| 13 | CHD5_bay | 26038 | CHD5 | GAGCGTTCGGGTTTTGCGGGGAGTAGGTTAAGGCGGTCGAGAGAAAGGGGGGTCGAGACGGGGGGGTG GAGGTTTGGGGGGGTGGGGGGGTAGGCGGTCGTTATTTTTCGTCGAGGTCG |
| 14 | CRBP_1 | 5947 | RBP1 | TTGGGAATTTAGTTGTCGTCGTTTCGTAGAGTTTTTTGTTTTCGGAGGGCGTTTATTTTCGGGTCGTTTATT ATTCGCGTAGTATCGGTAGTCGTTGTTT |
| 15 | DLK1_18031 | 8788 | DLK1 | GAGGTTTGCGGTTTAGGTTCGATTTTTGCGATTTGTTTTAGGTAGGTTTGTATGTGCGCGGCGGTCGCGTG TTGTATAGTGTGAG |
| 16 | DLK1_18033 | 8788 | DLK1 | GGAGTTGGGGTTTACGAGACGGGGCGTGCGGGGTATCGGGCGGTCGGCGGGGAGTCGTAGGTTTTTTT AGAGGGGGCGCGAGTCGGGTCGTCGGGGAATTTATTAT |
| 17 | DPYSL4_18 047 | 10570 | DPYSL4 | GGTGTTTTGATAGAAGTCGTTAGTCGGTGTTATGTTTAGGATAGGTATTTGTAGTTTTGTGTGGACGTTAAC GTTATTAGGAAGGATTATTAGGTCGTGGGCGTTAATGGTTTT |
| 18 | DPYSL4_18 050 | 10570 | DPYSL4 | GGGGTTATAGTTTGGCGTTCGGATTTTGGTTCGGGTTATTTGCGAAGGAGTCGGTTTTGGTTAAGGTGTTT TTTTGGACGGGTGTGGTTTTTAGAGC |
| 19 | DSC2_1805 6 | 1824 | DSC2 | GGTTTCGGTTTCGTTTTGTTCGTTGTTTTCGGCGACGGTCGTGGTTTTTTGTTTTGGGGTTAATTATAGAGC GAGGTTTGAGTCGTAGAG |
| 20 | EPB41L3_19 071 | 23136 | EPB41L3 | GGGATAGTGGGGTTGACGCGTGGTTTCGGCGTCGCGCGGTTTTTCGAATTTCGAGTTTCGCGTTCGGCG CGGTCGGGGTTTTTAATCGTTTTTTCGTTCGTCGGGATTTTTAT |
| 21 | EPB41L3_19 072 | 23136 | EPB41L3 | GCGTGGGTTTTCGTCGTAGTTTCGCGGAGTTTCGGTGTTTTTTGTAATAGGGGGCGGGGGGAATAGCGG CGAGTAGTTTTGGG |
| 22 | EPHB6_bay | 2051 | EPHB6 | GGGTGTTCGATTTAAGTCGAGTTCGAGTTCGAGTTTAGGTAGGAGTTTTATAGATAGTTTTTTTTTTTTTTAT TTTTTGTAGGCGTTTTACGCGTGCGATTTTTTCGGTTAAGATTCGCG |
| 23 | ERBB3_180 97 | 2065 | ERBB3 | GTTTAGTTAAGTTCGGTTCGGGGGTTTTTAGGTTAGGATATCGAGGTAAGAGTTATTTGAATCGTTGGCGA ATTGGTGGTTGTTGCGGCGACGGTAGCGGAGGTTGTAAATTGTAATC |
| 24 | ERBB3_180 99 | 2065 | ERBB3 | AGGGAGTTTAGTTAAGTTCGGTTCGGGGGTTTTTAGGTTAGGATATCGAGGTAAGAGTTATTTGAATCGTT GGCGAATTGGTGGTTGTTGCGGCGACGGTAGCGGAGGTTGTA |
| 25 | FBLN2_1332 8 | 2199 | FBLN2 | TAGAGCGGAGGAAGTTGCGGATTTGGGGTGGGGGAATTCGTTCGCGGATTTTTGGTTTTTATTTCGCGTC GGTTTTTGTGTTCGTATTTG |
| 26 | FBN2_18150 | 2201 | FBN2 | TCGGAGTTTTATAGGGTAACGAAGCGCGGGTAGCGGTTGCGGAGTCGGGCGGAGGTGCGCGGGGTCGG GGCGTGCGGTTAGTAAGAG |
| 27 | FBN2_16151 | 2201 | FBN2 | TTGGAGATTTCGATAGAGCGTCGGTTTTTTGATTGTTCGCGAAGCGAGACGCGGGGCGTCGGGTTTAGCG TAGTGAGCGGCGAGGCGCGGCGGAGGTGTAGTCGGTAGTTT |
| 28 | FOX-L2 | 668 | FOXL2 | GCGATAGGTTTTTAGTAAGTAAGCGCGGGCGGTATTCGTAGTTTTTAGAAGTTTGAGATTTGGTCGTAAGC GGATTCGTGCGTTTTAATTTTTTGTCGCGTTAGCGTTTGGAGCGGAGAG |
| 29 | Gst-Pi | 2950 | GSTP1 | TTCGGGGTGTAGCGGTCGTCGGGGTTGGGGTCGGCGGGAGTTCGCGGGATTTTTTAGAAGAGCGGTCG GCGTCGTGATTTAGTATTGGGGC |
| 30 | HS3ST2_19 130 | 9956 | HS3ST2 | ACGTAAGAGTTTGGGAGCGTTCGAGTCGTTCGGTTGTTCGGAGTTTTATCGTTTAGGATCGGGAGATGTT GGAAATGTAATCGTTTGTTTTTCGAGGAGTC |
| 31 | HS3ST2_19 131 | 9956 | HS3ST2 | GTTTCGGGGTTCGTTTTTCGGTAGGTTCGGGGAGAGGTGGGGTGATAATGGGTTGGGGTGCGCGCGTGT TTTATAGGTGCGAGATAGAGCGAGTCG |
| 32 | IGFBP7_191 96 | 3490 | IGFBP7 | TTTGTCGGCGTCGTTATTTTCGTACGGTTCGTTTTCGTCGCGGGCGTATATAGGGTAGTAGTCGTACGCGT CGCGGGTTTCGTTTAGTAGGTAGTTT |
| 33 | IGFBP7_192 00 | 3490 | IGFBP7 | CGTTTATGGGTCGGTTACGTCGGGTGTTCGTTTATTTTTCGACGTTAGTAGGAGCGCGCGCGTAGGTTTC GCGGGGTCGGGAGGGCGGTACGGGCGGGGTTTTCGTGTTTTTAT |
| 34 | IRF7_18346 | 3665 | IRF7 | AGTTGAGAATCGGACGGGGTGGGATCGAGGAGGGTGCGAAGCGTTATTGTTTAGGTTTCGTTTTTTCGGG AGTTTGATTCGTT |
| 35 | JAM3 | 83700 | JAM3 | GGGATTATAAGTCGCGTCGCGTTGTCGTTGGTTTTTTAGTAATTTTCGATATGGCGTTGAGGCGGTTATCG CGATTTCGGTTTTGCGTTCG |
| 36 | LOX_18967 | 4015 | LOX | GCGCGTAGAGTTGTAAAGGTTCGAGTAGGAGTACGGTTTAGGCGAAGCGTATTATTTTTTTTGTTAGATTG ATTTCGTTCGAGGAGGACGT |
| 37 | LOX_18977 | 4015 | LOX | GGTAGAGGCGAGGAGTTGTTCGTTTTGTACGTTTTTAATCGTATTACGTGAATAAATAGTTGAGGGGCGGT CGGGTTAGAACGGTTTGTGTA |
| 38 | LY6D_18402 | 8581 | LY6D | GATGTCGTTTGGGAGTAGTGCGGGTTTTTGTATTGTTAAGGTTTTATAGGTACGGGTTGGGCGGGGGTGG GTAGTTCGTTAGTTAGCGGTATTTTGT |
| 39 | LY6K | 54742 | LY6K | GCGGGGTTTTTTTATCGGTTAGATTCGGGGAGAGGCGCGCGGAGGTTGCGAAGGTTTTAGAAGGCGG GGAGGGGGCGTCGCGCGTTGATTTTTTTTGGGTATCGTTG |
| 40 | MACF1_bay | 23499 | MACF1 | GTTTTCGTTGTCGTTACGGGTTCGTTTTTTTTTTTTTTCGGTTTTTAGGGTAAGGCGCGGGGCGCGGGGTT GGATGTAGGCGTTTTGTTCGTTGCGC |
| 41 | MCAM | 4162 | MCAM | AGAATTTAGGTCGGTTTTTATCGTTTTTTAGAACGATTGTATTATTGTCGTTGTCGTCGGTTTGATATTGTTT TAGTTTTAGTGTTGGTAGTTTTGGGAGAAGAATTTTGCGT |
| 42 | NEFH_1845 2 | 4744 | NEFH | GTCGGATGAAGTATTCGGGCGTTTTTATTGCGGAAGGGCGGGGATGGTTGTGACGTAGGCGTGTTCGTC GTCGTTTGTAGGG |
| 43 | NID2_9093 | 22795 | NID2 | TTATTTCGTTTTTAGGGAGTTTTCGGGTTATTTTTTTATTCGGGTTGTTTCGCGGTTTTTAAGGAGTTTTATT TTCGGGATTAAATGGTTCGTAAG |
| 44 | NID2_9094 | 22795 | NID2 | TTTCGTGTGGGAAGAGTTCGTTTGGGTGTAGCGTCGCGGTTCGTAATATTAGTAACGGTAGTAGTAGTAGT ATTGGTAACGACGATAGTATCGGTCGTTCGGTTATTCG |
| 45 | PCDHB15_1 0763 | 56121 | PCDHB15 | TTTTGGTTATTAGGTAGTTCGGTTCGGCGGTTCGTTCGGGGTATTAGTTCGGTGTAGGGCGCGGAGTCGT TTTGTAGCGGGAATAGTACGAAGAGTG |
| 46 | PHACTR3_1 1692 | 116154 | PHACTR3 | TTATTTTGCGAGCGGTTTCGCGATACGAGGTAGTCGTTTTCGTTTTTCGACGCGGTTATGGGTTCGGTCGG CGCGGGGGTAAGTTAGAGCGAGTCGCGTGGAATTAGAGTATTC |
| 47 | POMC | 5443 | POMC | GATTTGGGCGTTTTTGGTTTTTCGCGGTTTCGAGTTTTCGATAAATTTTTTGCGTCGATTGCGGTATGAGAA GTC |
| 48 | PRKCA_186 26 | 5578 | PRKCA | GGGCGTTGAGGTAGAAGAACGTGTACGAGGTGAAGGATTATAAATTTATCGCGCGTTTTTTTAAGTAGTTT ATTTTTTGTAGTTATTGTATCGATTTTATTTGGTAGGTGTCG |
| 49 | PSEN1_186 48 | 5663 | PSEN1 | TTAGGTCGGAGGTTTCGTTTTTTTTTTTTTGGTTTTTTTTTTTTTTCGTGGGTCGGTCGTTAACGACGTTAGA GTCGGAAATGACGATAACGGTGAGGGTTT |
| 50 | RRAD_1869 8 | 6236 | RRAD | GATGTTTCGGTCGAGGTTTCGTCGTAGTTTTTTTTTAGTTTTTAGGTCGCGGCGTTTTTATTCGGGATTTTT TCGGATTTGGCGTATTTATTTGTAGTCGTTT |
| 51 | SFRP1 | 6422 | SFRP1 | TGTAGTTTTCGGAGTTAGTgtcgcgcgttcgtcgtttcgcgttTTTTTGTTCGTCGTATTTTCGGGAGTCGGGGCGTATT TAGTTCGTAGCGTCGTTTTTTCGTTCGCGTCGTTTTCGATCGTAGG |
| 52 | SFRP1_938 1 | 6422 | SFRP1 | TTTTGTTCGTCGTATTTTCGGGAGTCGGGGCGTATTTAGTTCGTAGCGTCGTTTTTTCGTTCGCGTCGTTTT CGATCGTAGGTCGAGGGTCGTTAT |
| 53 | SOD3_1874 0 | 6649 | SOD3 | AGTATAGAGTGGGGAGCGTAGCGACGAAGAATGAATAGGGTTTCGTGAGGTTTTAAATATTCGTTTCGGT GGTAGGAAAG |
| 64 | SOX17 | 64321 | SOX17 | TTGCGTTAGTCGTTTGCGTTCGTTTTTAGTTTATATTATGAAAGCGTTTATCGGTCGTCGGATACGGGATTC GTTTTTG |
| 55 | SULF2_bay | 55959 | SULF2 | GTTAGTCGAGTTCGGAGGTATCGGGAGGTCGAGAGTCGTCGGGATTTTAGTTTTGCGTTTATTGTTTCGTT CGGAGTTG |
| 56 | TIMP3 | 7078 | TIMP3 | GCGTCGGAGGTTAAGGTTGTTTCGTACGGTTCGGCGGGCGAGCGAGTTCGGGTTGTAGTAGTTTCGTCG GCGGCGCGTACGGTAATTTTGGAGAG |
| 57 | TJP2_18792 | 9414 | TJP2 | CGGGTTAGAGTATTGTTCGGTGGTGTTTAGGAGGAGTAGGAGTAGGAGTAGAAGTAGAAGCGGGGTTCG GAGTTGCGCGTTTACGCGGGATTTGTGTTC |
| 58 | TJP2_18797 | 9414 | TJP2 | GATTTTATCGGGGAAATATCGCGGATAGTCGGGTTAGTAGCGTTCGGAGTTTATTTTAGGTTTTTAAATTTG TAGTATTTTTTAGAGCGCGCGCGTTCGGAGCGGGATTTGTTT |
| 59 | TRPV2_188 03 | 51393 | TRPV2 | TTATTTCGTAGGTTGAGGTTAGGGCGTGGCGGTTGTTGGGATTTCGGAGTTTTTTAGTAGTAGGGGTTGC GGGAGGAAGTGAAGTCGGGAGGGGTTGTCGGCGTTGATAGTAGAGGA |
| 60 | UCHL1 | 7345 | UCHL1 | GTTGTATTTTCGCGGAGCGTTCGGTAGAAATAGTTTAGGGAAGACGAAAAATAGTTAGCGGAGTCGTTTAG GTTGTAGTTATAAAGCGTCGGTTAGACGTATTGTGAG |
| 61 | WDR69_188 44 | 164781 | WDR69 | GTTTAGGTTGTGGTTTAGGTCGTCGGTTTTCGGTTATGTTTAGTTTTTTTGAGGTCGTTTTTAGTGAGGATA CGAGGTGT |
| 62 | ZFP42_bay | 132625 | ZFP42 | GGGGTTTTTAGGTATTCGGTTCGTACGTAAATTTTTAGTTCGGGGTTTTTTGATTTTCGCGTTTATTTTTTAG TTCGGTCGTCGGGTATTGCGTATT |
| 63 | ZNF442_bay | 79973 | ZNF442 | TCGGTTTTTAGTTTTTTCGGTCGCGGGGTGGGAGTTGGGGGTTGGGTCGGTAGTCGGGATTTCGGGCGT TTTGTTTCGTTTTTGCGTAGTAATTG |
| 64 | ZNF655 | 79027 | ZNF655 | TTATCGAGAAGCGTCGGTTTCGGGGTTGTTTATAGCGGTTCGGGAGAGGTTGTGGTGGTTTCGAGCGCGA GTGTGTAGGTGATAGGATAGCGGTTAGGTTCGTTTTTTTTTTCGGT |

| Amplicon details (non-converted sequence): | | | | |
|---|---|---|---|---|
| | Assay Name | Entrez GeneID | Official Gene symbol | amplicon sequence (not converted) (SEQ ID NO:193-256, respectively) |
| 1 | ACSL6_17 822 | 23305 | ACSL6 | CTCAATGTCACGCTCTGGCGCTCGTCGCCCGTGCTCCCCCTTCCAGCCGGTTTCCGCAGAATGCCAGGT ACTGACGTTGGAGAGCGGGGCCGGAGAGGGCTGGTTC |
| 2 | ACSL6_17 824 | 23305 | ACSL6 | GCGGCTGCAAGGCCTTTGGCCGGTGAGTGAACCAGTAGGCAAGGATGGCAGCCAGGGCACCCATACTCA CGAGGGTGGTGGCCGAGAGGCTGCGGAAAAAC |
| 3 | ALS2CL_b ay | 259173 | ALS2CL | GGACGGGTGTCTGCGCTCCACGCTTAGCTCGTCCAGGTGGGGGCTCCCGCCTCCTCGGCTGCTGCGGT CCCCGCCCAGCTCCTTGGTCCCG |
| 4 | APC2 | 10297 | APC2 | GCCGCCTGCCCAGGCCCGGACCGGGCTTTGTCCGCCCCGGAGCCCCTGCCCGCGCCGCGGAGACCCC GGAGCCCGCGCGCTCCGAGGCCACCCCGGGCC |
| 5 | BEX1_128 42 | 55859 | BEX1 | CCGGGGCCCTTACCTGGTCCGCTTTCCCCCGGGCCGGATGCCAGCCCGCCGAGCGCAGGGCAGCGGG GAGCTGGTAGCGAGACACGAGTGACGACT |
| 6 | BMP7_179 05 | 655 | BMP7 | GCACGTGCGCTCACTGCGAGCTGCGGCGCCGCACAGCTTCGTGGCGCTCTGGGCACCCCTGTTCCTGCT GCGCTCCGCCCTGGCCGACTTCAGCCTGGACAACG |
| 7 | CBR3_179 31 | 874 | CBR3 | GGCATCGGCTTGGCCATCGCGCGCGAACTGTGCCGACAGTTCTCTGGGGATGTGGTGCTCACCGCGCG GGACGTGGCGCGGGGCCAGGCGGCCGTGCAGCAGCTGCAGGCG |
| 8 | CBR3_179 35 | 874 | CBR3 | GCCCCCGACTGACCCATCAAGGTCCGATTTGGCTTCGGACACCTCGCAGATCACCCCGCGGCTCAGAGC CCGGATCCTGA |
| 9 | CD248_17 939 | 57124 | CD248 | CCGTGGGAAGAGAGCGTAGCAGCTGCTGGGGCCGCAGGCGGCACGGGGCTCAGCAGCCCAGGGGTCC TGGCCCAGTGTGGGCCCTGCGGCCGCCCAGGCCAGCAA |
| 10 | CD248_17 946 | 57124 | CD248 | CCCTGTCAAGAGCTGCCGCCAGCCCGGGGCCGGACCAGTCCGGGGGCATCGCGATGCTGCTGCGCCTG TTGCTGGCCTGGGCGGCCGCAGGGCCCACACT |
| 11 | CD248_17 947 | 57124 | CD248 | GGGGCAGCCGTCAACTGCGCCTTCTCCCCTCCTCCGCCCCCAACCTTAGAGCCCCCCACCCCACTGCTT CCTGCTCTAGCGGCCCCCGGGGAAGA |
| 12 | CD44_179 61 | 960 | CD44 | CGGGAGAAGAAAGCCAGTGCGTCTCTGGGCGCAGGGGCCAGTGGGGCTCGGAGGCACAGGCACCCCG CGACACTCCAGGTTCCCCGACCC |
| 13 | CHD5_bay | 26038 | CHD5 | GAGCGCCCGGGCTTTGCGGGGAGCAGGCTAAGGCGGCCGAGAGAAAGGGGGGTCGAGACGGGGGGGT GGAGGTTTGGGGGGGTGGGGGGGCAGGCGGCCGCCATCTTCTCGCCGAGGCCG |
| 14 | CRBP_1 | 5947 | RBP1 | CTGGGAATCCAGCTGTCGCCGCCCCGCAGAGCCCCCTGTCCCCGGAGGGCGCTCATTTCCGGGCCGCC CACCACCCGCGTAGCACCGGCAGCCGCTGTCC |
| 15 | DLK1_180 31 | 8788 | DLK1 | GAGGTCTGCGGCCCAGGTTCGATTCCTGCGACTTGTCCTAGGCAGGCCTGTATGTGCGCGGCGGCCGC GTGCTGTACAGTGTGAG |
| 16 | DLK1_180 33 | 8788 | DLK1 | GGAGTTGGGGCTCACGAGACGGGGCGTGCGGGGCACCGGGCGGCCGGCGGGGAGTCGCAGGCTTCCC CAGAGGGGGCGCGAGCCGGGCCGCCGGGGAACTCACCAT |
| 17 | DPYSL4_1 8047 | 10570 | DPYSL4 | GGTGCCCTGACAGAAGTCGTCAGCCGGTGTCATGCCCAGGACAGGCATCTGCAGCCTTGTGTGGACGTC AACGCCACCAGGAAGGACCATCAGGCCGTGGGCGTCAATGGTCTT |
| 18 | DPYSL4_1 8050 | 10570 | DPYSL4 | GGGGTCACAGCCTGGCGCTCGGACCCTGGCCCGGGTCATCTGCGAAGGAGCCGGCTTTGGCCAAGGTG CCTTCCTGGACGGGTGTGGTTCCCAGAGC |
| 19 | DSC2_180 56 | 1824 | DSC2 | GGCCCCGGCTCCGCCCTGCCCGCTGCCCTCGGCGACGGCCGTGGTCCCTGCCCTGGGGTCAATTACAG AGCGAGGTCTGAGCCGCAGAG |
| 20 | EPB41L3_ 19071 | 23136 | EPB41L3 | GGGACAGTGGGGCTGACGCGTGGCTTCGGCGCCGCGCGGTCTCCCGAATCCCGAGCCCCGCGCCCGG CGCGGCCGGGGTCCCCAACCGCCCTCCCGCTCGCCGGGACCCCCAC |
| 21 | EPB41L3_ 19072 | 23136 | EPB41L3 | GCGTGGGCCCCCGCCGCAGCTCCGCGGAGCCTCGGTGTCTCCTGCAACAGGGGGCGGGGGGAACAGC GGCGAGCAGCCCTGGG |
| 22 | EPHB6_ba y | 2051 | EPHB6 | GGGTGTCCGACCCAAGCCGAGCCCGAGCCCGAGCCCAGGCAGGAGCTTTACAGACAGCCTCTTCCCTTC CCACTTCCTGCAGGCGCCCCACGCGTGCGATCCTCCCGGCCAAGACCCGCG |
| 23 | ERBB3_18 097 | 2065 | ERBB3 | GCCCAGCCAAGTCCGGCCCGGGGGCCCCTAGGCTAGGACATCGAGGCAAGAGCCACCTGAACCGCTGG CGAATTGGTGGCTGCTGCGGCGACGGCAGCGGAGGTTGCAAATTGCAATC |
| 24 | ERBB3_18 099 | 2065 | ERBB3 | AGGGAGCCCAGCCAAGTCCGGCCCGGGGGCCCCTAGGCTAGGACATCGAGGCAAGAGCCACCTGAACC GCTGGCGAATTGGTGGCTGCTGCGGCGACGGCAGCGGAGGTTGCA |
| 26 | FBLN2_13 328 | 2199 | FBLN2 | CAGAGCGGAGGAAGCTGCGGACCTGGGGTGGGGGAACCCGCCCGCGGACCCCTGGCCCCCACCCCGC GCCGGCCTCTGTGCCCGCATCTG |
| 26 | FBN2_181 50 | 2201 | FBN2 | TCGGAGTCCCACAGGGCAACGAAGCGCGGGTAGCGGCTGCGGAGCCGGGCGGAGGTGCGCGGGGCCG GGGCGTGCGGCCAGCAAGAG |
| 27 | FBN2_181 51 | 2201 | FBN2 | CTGGAGACCTCGACAGAGCGCCGGCCCCCTGACTGCCCGCGAAGCGAGACGCGGGCGCCGGGTCTA GCGCAGTGAGCGGCGAGGCGCGGCGGAGGTGCAGCCGGCAGCCC |
| 28 | FOX-L2 | 668 | FOXL2 | GCGACAGGCCTCCAGCAAGCAAGCGCGGGCGGCATCCGCAGTCTCCAGAAGTTTGAGACTTGGCCGTAA GCGGACTCGTGCGCCCCAACTCTTTGCCGCGCCAGCGCCTGGAGCGGAGAG |
| 29 | Gst-Pi | 2950 | GSTP1 | CCCGGGGTGCAGCGGCCGCCGGGGCTGGGGCCGGCGGAGTCCGCGGACCCTCCAGAAGAGCGGC CGGCGCCGTGACTCAGCACTGGGGC |
| 30 | HS3ST2_1 9130 | 9956 | HS3ST2 | ACGTAAGAGCCTGGGAGCGCCCGAGCCGCCCGGCTGCCCGGAGCCCCATCGCCTAGGACCGGGAGATG CTGGAAATGCAACCGCCTGTTCCCCGAGGAGCC |
| 31 | HS3ST2_1 9131 | 9956 | HS3ST2 | GCTCCGGGGCTCGCTCTCCGGCAGGCCCGGGGAGAGGTGGGGTGACAATGGGTTGGGGTGCGCGCGT GCCTCATAGGTGCGAGACAGAGCGAGCCG |
| 32 | IGFBP7_1 9196 | 3490 | IGFBP7 | CCTGCCGGCGCCGCCACCCCCGCACGGCTCGCCCTCGCCGCGGGCGCACATAGGGCAGCAGCCGCAC GCGTCGCGGGTCTCGCCCAGCAGGCAGCCC |
| 33 | IGFBP7_1 9200 | 3490 | IGFBP7 | CGCCCATGGCCGGTCACGCCGGGTGCCCGCTCACCCCCCGACGCCAGCAGGAGCGCGCGCGCAGGC CCCGCGGGGCCGGGAGGGCGGCACGGGCGGGGCCCCCGTGCTCTCAC |
| 34 | IRF7_1834 6 | 3665 | IRF7 | AGCTGAGAACCGGACGGGGTGGGATCGAGGAGGGTGCGAAGCGCCACTGTTTAGGTTTCGCTTTCCCGG GAGCCTGACCCGCC |
| 35 | JAM3 | 83700 | JAM3 | GGGACTACAAGCCGCGCCGCGCTGCCGCTGGCCCCTCAGCAACCCTCGACATGGCGCTGAGGCGGCCA CCGCGACTCCGGCTCTGCGCTCG |
| 36 | LOX_1896 7 | 4015 | LOX | GCGCGCAGAGCTGCAAAGGCCCGAGCAGGAGCACGGTCCAGGCGAAGCGCATCACTCCTTTTGCCAGAT TGACCCCGCTCGAGGAGGACGT |
| 37 | LOX_1897 7 | 4015 | LOX | GGCAGAGGCGAGGAGCTGTCCGCCTTGCACGTTTCCAATCGCATTACGTGAACAAATAGCTGAGGGGCG GCCGGGCCAGAACGGCTTGTGTA |
| 38 | LY6D_184 02 | 8581 | LY6D | GATGTCGTCTGGGAGCAGTGCGGCCCCTGCATTGCCAAGGCCTTATAGGCACGGGCTGGGCGGGGT GGGCAGTCCGCCAGCCAGCGGCATTCTGC |
| 39 | LY6K | 54742 | LY6K | GCGGGGCTCCCCTACCGGCCAGACCCG GGGAGAGGCGCGCGGAGGCTGCGAAGGTTCCAGAAGGGC GGGGAGGGGGCGCCGCGCGCTGACCCTCCCTGGGCACCGCTG |
| 40 | MACF1_b ay | 23499 | MACF1 | GCCTTCGCTGCCGCCACGGGCCCGTCTTCTTCCTCCTTCGGCTCCCAGGGTAAGGCGCGGGGCGCGGG GTTGGATGCAGGCGCCCTGCCCGCTGCGC |
| 41 | MCAM | 4162 | MCAM | AGAATTCAGGCCGGCCTCTATCGCTTCCCAGAACGATTGCACCACTGCCGCTGCCGCCGGCCTGACACT GCCTCAGCCTCAGTGCTGGCAGCTTTGGGAGAAGAACCCTGCGC |
| 42 | NEFH_184 52 | 4744 | NEFH | GCCGGATGAAGCATTCGGGCGTTCCCACTGCGGAAGGGCGGGGATGGCTGTGACGCAGGCGTGCCCGC CGTCGCCTGCAGGG |
| 43 | NID2_909 3 | 22795 | NID2 | CCACTCCGCCCCCAGGGAGCTCCCGGGTCATCCTCTCATCCGGGCTGCCCCGCGGCCCCCAAGGAGCC CCACCCCCGGGACCAAATGGCCCGCAAG |
| 44 | NID2_909 4 | 22795 | NID2 | CCCCGTGTGGGAAGAGCTCGTCTGGGTGCAGCGCCGCGGCCCGCAACATTAGCAACGGCAGCAGCAGT AGCACTGGTAACGACGACAGCACCGGCCGCCCGGCCACCCG |
| 45 | PCDHB15 _10763 | 56121 | PCDHB15 | CCTTGGTCACCAGGTAGCCCGGCTCGGCGGCCCGCCCGGGGCATCAGCTCGGTGCAGGGCGCGGAGC CGTTCTGCAGCGGGAACAGCACGAAGAGTG |
| 46 | PHACTR3 _11692 | 116154 | PHACTR3 | TCACTCTGCGAGCGGCCCCGCGACACGAGGCAGCCGCTCCCGTCCTCCGACGCGGCCATGGGCCCGGC CGGCGCGGGGGCAAGTTAGAGCGAGCCGCGTGGAATCAGAGCATCC |
| 47 | POMC | 5443 | POMC | GACCTGGGCGCCTCTGGCTCTCCGCGGTCCCGAGTTCTCGACAAACTTTCTGCGCCGACTGCGGCATGA GAAGCC |
| 48 | PRKCA_1 8626 | 5578 | PRKCA | GGGCGCTGAGGCAGAAGAACGTGCACGAGGTGAAGGACCACAAATTCATCGCGCGCTTCTTCAAGCAGC CCACCTTCTGCAGCCACTGCACCGACTTCATCTGGTAGGTGCCG |
| 49 | PSEN1_18 648 | 5663 | PSEN1 | CCAGGCCGGAGGCCCCGCCCCCTTCCTCCTGGCTCCTCCCCTCCTCCGTGGGCCGGCCGCCAACGACG CCAGAGCCGGAAATGACGACAACGGTGAGGGTTC |
| 50 | RRAD_18 698 | 6236 | RRAD | GATGCTCCGGCCGAGGTCCCGCCGCAGCCCTCCCCCAGCCCCCAGGTCGCGGCGCCCTCACCCGGGAC CCCTCCGGACCTGGCGCATCCATCTGCAGCCGCCC |
| 51 | SFRP1 | 6422 | SFRP1 | TGCAGCCTCCGGAGTCAGTgccgcgcgcccgccgcccccgcgccTTCCTGCTCGCCGCACCTCCGGGAGCCGGGGC GCACCCAGCCCGCAGCGCCGCCTCCCCGCCCGCGCCGCCTCCGACCGCAGG |
| 52 | SFRP1_93 81 | 6422 | SFRP1 | TCCTGCTCGCCGCACCTCCGGGAGCCGGGGCGCACCCAGCCCGCAGCGCCGCCTCCCCGCCCGCGCC GCCTCCGACCGCAGGCCGAGGGCCGCCAC |
| 53 | SOD3_187 40 | 6649 | SOD3 | AGTACAGAGTGGGGAGCGCAGCGACGAAGAATGAACAGGGCCTCGTGAGGTCCCAAACACCCGTTTCGG TGGCAGGAAAG |
| 54 | SOX17 | 64321 | SOX17 | CTGCGCCAGCCGCTTGCGCTCGTCCTTAGCCCACACCATGAAAGCGTTCATCGGCCGCCGGATACGGGA CTCGCCCTTG |
| 55 | SULF2_ba y | 55959 | SULF2 | GCCAGCCGAGTCCGGAGGCATCGGGAGGTCGAGAGCCGCCGGGACCCCAGCTCTGCGTTCACTGCCCC GTCCGGAGCTG |
| 56 | TIMP3 | 7078 | TIMP3 | GCGCCGGAGGCCAAGGTTGCCCCGCACGGCCCGGCGGGCGAGCGAGCTCGGGCTGCAGCAGCCCCGC CGGCGGCGCGCACGGCAACTTTGGAGAG |
| 57 | TJP2_187 92 | 9414 | TJP2 | CGGGTCAGAGCACTGTCCGGTGGTGCCCAGGAGGAGTAGGAGCAGGAGCAGAAGCAGAAGCGGGGTCC GGAGCTGCGCGCCTACGCGGGACCTGTGTCC |
| 58 | TJP2_187 97 | 9414 | TJP2 | GACCTCACCGGGGAAACACCGCGGACAGTCGGGCCAGCAGCGCCCGGAGCTCACTCCAGGTCTCCAAA CTTGCAGCACTTCCCAGAGCGCGCGCGCTCGGAGCGGGACCTGCTT |
| 59 | TRPV2_18 803 | 51393 | TRPV2 | TTACCCCGCAGGCTGAGGCCAGGGCGTGGCGGCTGCTGGGATCCCGGAGCTTCTCAGTAGCAGGGGCT GCGGGAGGAAGTGAAGCCGGGAGGGGCTGCCGGCGCTGACAGCAGAGGA |
| 60 | UCHL1 | 7345 | UCHL1 | GCTGCATCTTCGCGGAGCGCCCGGCAGAAATAGCCTAGGGAAGACGAAAAACAGCTAGCGGAGCCGCC CAGGCTGCAGCTATAAAGCGCCGGCCAGACGCACTGTGAG |
| 61 | WDR69_1 8844 | 164781 | WDR69 | GCCCAGGCTGTGGCCTAGGCCGTCGGTTCCCGGCCATGCCTAGCTCCTCTGAGGTCGCCCTTAGTGAGG ACACGAGGTGC |
| 62 | ZFP42_ba y | 132625 | ZFP42 | GGGGCCCCCAGGCACCCGGCCCGCACGCAAACCCTCAGCCCGGGGCCCCCTGACCCCCGCGTTCACCC CTCAGCCCGGCCGCCGGGCACTGCGCATC |
| 63 | ZNF442_b ay | 79973 | ZNF442 | CCGGCCTTCAGTCCCCTCGGCCGCGGGGTGGAGCTGGGGGCTGGGCCGGCAGCCGGGACCCCGGGC GTCCTGTCCCGTTTCTGCGCAGCAACTG |
| 64 | ZNF655 | 79027 | ZNF655 | CCACCGAGAAGCGCCGGCCTCGGGGCTGTCTACAGCGGCCCGGGAGAGGCTGTGGTGGCCCCGAGCG CGAGTGTGTAGGTGACAGGACAGCGGCCAGGCCCGCCCCTCCCCTCGGT |

### EXAMPLE 2: Final selection of assays for Base 5

Finally a total number of 80 different assays (62 different genes), comprising:
- 64 assays designed for detecting the methylation status of 49 cancer markers identified by the aforementioned strategy,
- assays for known published markers, and
- good performing assays for cancer markers from other in-house cancer projects, were retained for analysis.

Differential methylation was assessed using the Base 5 platform; genes were ranked based on the best selectivity (sensitivity and specificity) between human lung cancer tissue and normal lung tissue samples. The investigated genes were ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, ZNF655.

Primer and amplicon sequences for the 49 genes are summarized in Table 1 and 2. Primer and amplicon sequences for the remainder 13 genes are listed in Table 3 and Table 4.

**Table 3: MSP Primer sequences**

| **Gene ID** | **Symbol** | **Assay** | **Sense primer sequence (5' - 3') (SEQ ID NO: 285-299, respectively)** | **Antisense primer sequence (5' - 3') (SEQ ID NO: 300-314, respectively)** |
|---|---|---|---|---|
| 51752 | ARTS-1 | ARTS-1_17861 | GTAGTGGCGAGATGACGGA | AACCGAAACCAAACAAACG |
| 664 | BNIP3 | BNIP3_13409 | AGTGTTTAGAGAGTTCGTCGGTT | CGTAACGAATAAACTACGCGAT |
| 1910 | EDNRB | EDNRB_3 | GTCGGGTGTTATATGGTGCGT | AAAAACAATCCTCGTCCGAAA |
| 2778 | GNAS | GNAS_18295 | TTTTGAGAGGTCGTTATCGTGT | TTACTCGAACTATTCCCCGATT |
| 3249 | HPN | HPN_18326 | CGTTAGGTAGGGAGGAGGC | AACGATAAAATAAAAACAACGAC C |
| 4686 | NCBP1 | NCBP1_18440 | ATTTGGGTAGAAAAGTTCGTTC | CTCAATAATTTTCCCGACGAC |
| 26025 | PCDHGA12 | PCDHGA12_18516 | AACGATTTGGGGTTAGAGTTTC | TAACCAAACTACCGCTTTACGA |
| 5214 | PFKP | PFKP_18555 | TTTTCGTTATGGACGCGGA | ATAACCTTACCGACCCCGAA |
| 10857 | PGRMC1 | PGRMC1_9140 | CGTTCGTATAGAGTTCGGTAATGT C | CCTATAACTAAACGCGACGCAC |
| 5256 | PHKA2 | PHKA2_18567 | CGTTTTTGGTTTTGTTTTCGT | AACCTAATTCCCGCCCGTT |
| 5256 | PHKA2 | PHKA2_18576 | TTTAGTAGGTTTGGTCGAGGC | ACGCTAACCCCAAAATCCG |
| 5256 | PHKA2 | PHKA2_18579 | TATAGGTAAGGGGGCGGTTTC | GCGACTCTAAAAATTCCGCT |
| 11186 | RASSF1A | RASSF1A | GCGTTGAAGTCGGGGTTC | CCCGTACTTCGCTAACTTTAAACG |
| 9770 | RASSF2 | RASSF2_1 | TTAGAGGGGCGTAGGGTGC | GCCAAACTAAAATCCCAACGA |
| 6446 | SGK | SGK_18737 | CGTTGTAGGATTTTGGGGGTC | ACCCTTCTCCCGCTCGATA |

**Table 4: MSP amplicon sequences**

| **Assay** | **Amplicon Sequence (converted) (5' - 3') (SEQ ID NO: 315-329 respectively)** | **Amplicon Sequence (not converted) (5' - 3') (SEQ ID NO: 330-344, respectively)** |
|---|---|---|
| ARTS-1_17861 | GTAGTGGCGAGATGACGGATATTTAGCGAGTTTA ATGGGCGTCGAACGCGTTTAGGTTTGGTGGATTT GTTAGCGTTTGTTTGGTTTCGGTT | GCAGTGGCGAGATGACGGACACCCAGCGAGTCCA ATGGGCGTCGAACGCGTCTAGGCTTGGTGGACTTG TCAGCGCCTGCCTGGCTTCGGTC |
| BNIP3_13409 | AGTGTTTAGAGAGTTCGTCGGTTTTATCGTTTTTT TAAAGGAGAATTCGGTTTATCGTTCGTCGCGGCG GCGATCGCGTAGTTTATTCGTTACG | AGTGCCCAGAGAGTCCGCCGGTCCCACCGCCCCTT CAAAGGAGAACCCGGCCCACCGCCCGCCGCGGCG GCGACCGCGCAGCCCACTCGTCACG |
| EDNRB_3 | GTCGGGTGTTATATGGTGCGTGATAATTTGTTTTT GATTTGGGTTTATTTGAAGAGCGTAGAATTTTTAA TAAATAAATAGTTTTTGGGATTTGTTTTCGGACG AGGATTGTTTTT | GCCGGGTGTCACATGGTGCGTGATAACTTGCCCTT GATTTGGGTTCATTTGAAGAGCGTAGAACTCTAAC AAATAAACAGCCTTTTGGGACCTGTCCCCGGACGA GGACTGCCCCC |
| GNAS_18295 | TTTTGAGAGGTCGTTATCGTGTTATGGGCGTGCG TAATTGTTTTTACGGTAATAATATGTTAGGATAA CGCGATATTTTTTTGAAATCGGGGAATAGTTCG AGTAA | TTTTGAGAGGCCGCCACCGTGTTATGGGCGTGCGC AACTGCCTCTACGGCAATAATATGTCAGGACAACG CGATATCCCCCCTGAAATCGGGGAACAGCCCGAGC AA |
| HPN_18326 | CGTTAGGTAGGGAGGAGGCGGGGAGGGGTTGGT TTTAGAAGTGCGTGTTTGAAGCGGTTAATGTGTG TAAATTAGTAAGGAGGAGGGGTGCGGGGTCGTT GTTTTTATTTTATCGTT | CGCCAGGCAGGGAGGAGGCGGGGAGGGGCTGGCC CCAGAAGTGCGTGTCTGAAGCGGCCAATGTGTGCA AATCAGCAAGGAGGAGGGGTGCGGGGCCGCTGCC CCCACCTCACCGCC |
| NCBP1_18440 | ATTTGGGTAGAAAAGTTCGTTCGTGACGTTATTA AGTTTCGGAAGTTTTTTGGCGTCGGCGTAAGGGT CGTCGGGAAAATTATTGAG | ATTTGGGTAGAAAAGCTCGCTCGTGACGTCACCAA GCTCCGGAAGTCTCCTGGCGTCGGCGCAAGGGCCG CCGGGAAAACCATTGAG |
| PCDHGA12_18 516 | AACGATTTGGGGTTAGAGTTTCGGGAGTTGGCGG AGCGCGGAGTTCGTATCGTTTTTAGAGGTAGGAC GTAGTTTTTTTTTTTGAATTCGTAAAGCGGTAGTT TGGTTA | AACGACCTGGGGCTAGAGCCCCGGGAGCTGGCGG AGCGCGGAGTCCGCATCGTCTCCAGAGGTAGGAC GCAGCTTTTCTCTCTGAATCCGCAAAGCGGCAGCT TGGTCA |
| PFKP_18555 | TTTTCGTTATGGACGCGGACGATTTTCGGGTTTTT AAGGGTTTTTTGCGGAAGTTTTTGGAGTATTTTTT CGGGGTCGGTAAGGTTAT | TCCTCGCCATGGACGCGGACGACTCCCGGGCCCCC AAGGGCTCCTTGCGGAAGTTCCTGGAGCACCTCTC CGGGGCCGGCAAGGCCAT |
| PGRMC1_9140 | CGTTCGTATAGAGTTCGGTAATGTCGAGGTTTTTT TAACGGGTCGGTTTGCGAGGAGTAAAAAAGGGG TTTAGAGGAGGGTAGCGCGTGCGTCGCGTTTAGT TATAGG | CGCTCGCACAGAGCCCGGCAATGCCGAGGCCCTCC CAACGGGTCGGTCTGCGAGGAGCAAAAAAGGGGT TCAGAGGAGGGCAGCGCGTGCGTCGCGCTCAGCT ATAGG |
| PHKA2_18567 | CGTTTTGGTTTTGTTTTCGTCGCGGAGCGGAATT TTTTAAGTCGCGGTTTGAGGAGGAAGGAAAAGG GGGCGGTTCGGGAGAGTCGTTGCGAAATTAGTA ACGGGCGGGAATTAGGTT | CCCAGCAGGCCTGGCCGAGGCGGACCTTCGTCGC TCCAGCCCCCGTCCCCGCCCCCGCGCCTCCCCGCC GCGCGGAGCTCTGGTTGGCTTGCTTTCCAACCGGA CTTTGGGGCTAGCGT |
| PHKA2_18576 | TTTAGTAGGTTTGGTCGAGGCGGATTTCGTCG TTTTAGTTTTCGTTTTCGTTTTCGCGTTTTTTCGTC GCGCGGAGTTTTGGTTGGTTTGTTTTTTAATCGGA TTTTGGGGTTAGCGT | CCCAGCAGGCCTGGCCGAGGCGGACCTTCGTCGC TCCAGCCCCCGTCCCCGCCCCCGCGCCTCCCCGCC GCGCGGAGCTCTGGTTGGCTTGCTTTCCAACCGGA CTTTGGGGCTAGCGT |
| PHKA2_18579 | TATAGGTAAGGGGGCGGTTTCGTTTCGCGTTTTG GAACGATTTTACGGTTTCGTTTATATTTTCGTTTT TGGTTTTATTTTCGTCGTAGAGCGGAATTTTTAGA GTCGC | CACAGGTAAGGGGGCGGCCCCGCCCCGCGCCCTG GAACGACCTCACGGCCCCGCCCACATCCCCGCCCC TGGCCCCACCTCCGCCGCAGAGCGGAACCCTCAGA GTCGC |
| RASSF1A | GCGTTGAAGTCGGGGTTCGTTTTGTGGTTTCGTTC GGTTCGCGTTTGTTAGCGTTTAAAGTTAGCGAAG TACGGG | GCGCTGAAGTCGGGGCCCGCCCTGTGGCCCCGCCC GGCCCGCGCTTGCTAGCGCCCAAAGCCAGCGAAG CACGGG |
| RASSF2_1 | TTAGAGGGGCGTAGGGTGCGCGGGGGTCGTTGG TTTTTCGGGTATTTTTTTTTGCGGTTTTTTCGTTT TTTTTCGGAGTTGGTGTTTGAGGTCGTTGGGATTT TAGTTTGGC | TCAGAGGGGCGCAGGGTGCGCGGGGGCCGTTGGC CCTCCGGGCACTTCCCCTTTGCGGTCTCCCCGCCCT CCTTCGGAGCTGGTGCCTGAGGTCGCTGGGACCTC AGCCTGGC |
| SGK_18737 | CGTTGTAGGATTTTGGGGGTCGGACGGTGGGATA CGGTTAATTTTCGGGGAGATGTTGTGGTTTTTATC GAGCGGGAGAAGGGT | CGCTGCAGGACCCTGGGGCCGGACGGTGGGATA CGGCCAATCTCCGGGGAGATGCTGTGGCTCTTACC GAGCGGGAGAAGGGT |

### EXAMPLE 3: Lightcycler

Twenty three assays issuing from the Base 5 analysis were selected and transferred to the Lightcycler platform in order to confirm the Base 5 results using 3 independent sample sets (JHU, Baltimore, USA; UMCG, Groningen, The Netherlands and Ulg, Liège, Belgium) and to define the best lung cancer methylation markers (Table 5). A beta-actin (ACTB) assay was included as an internal control. The assays were applied on a 384 well plate. The samples were randomized per plate. On this platform Ct values (cycle number at which the amplification curves cross the threshold value, set automatically by the software) and melting curves (Tm) were generated on the Roche LightCycler 480 using SYBR green as detector and for verification of the melting temperature. The size of the amplicon and intensity of the signal detected were analyzed using the Caliper LabChip electrophoretic separation system. Well-defined cut offs were set up on Ct, Tm, amplicon size and signal to get similar methylation calls when using the final Molecular Beacon (MB) detection system for further verification of the markers. DNA methylation calls were compared between 146 lung cancer and 58 normal tissue samples. DNA was isolated using proteinase K digestion and phenol/chloroform extraction method. DNA concentration was measured using NanoDrop Spectrophotometer. From each sample, up to 3 µg of genomic DNA was converted using a bisulphite based protocol (EZ DNA Methylation Kit™, ZYMO Research). After conversion and purification the equivalent of 20 ng of gDNA was used per reaction. An assay ranking was generated and the results are summarized in a methylation table (Figure 3).

A sample was considered methylated if Ct is under 40 and if Tm and amplicon size are within the boundaries of Tm +/- 2 degrees and amplicon size +/- 10 bp. The intensity of the band detected by capillary electrophoresis had to be higher than 20. Those cut offs were set up to get similar methylation calls after Lightcycler analysis and real time PCR with Beacon detection system.

DNA methylation calls were compared between lung cancer tissue and normal lung tissue. An assay ranking with the set of samples was generated and the results are summarized in a methylation table (Figure 3). A one-tailed Fisher's exact test was used as a scoring function to rank the candidate markers. The calculation of Fisher's exact test was based on a formula as described by Haseeb Ahmad Khan in "A visual basic software for computing Fisher's exact probability" (Journal of Statistical Software, vol. 08, issue i21, 2003).

A general overview of the ranking is given in Table 6.

**Table 5: The 23 selected assays which were applied on the Lightcycler platform**

| **N°** | **Assays** | **N°** | **Assays** |
|---|---|---|---|
| **1** | ARTS-1_17861 | **13** | PFKP_18555 |
| **2** | BNIP3_13409 | **14** | PGRMC1_9140 |
| **3** | DLK1_18033 | **15** | PHKA2_18567 |
| **4** | EDNRB_3 | **16** | PHKA2_18576 |
| **5** | FBN2_18150 | **17** | PHKA2_18579 |
| **6** | GNAS_18295 | **18** | PSEN1_18648 |
| **7** | GSTP1 | **19** | RASSF1A |
| **8** | HPN_18326 | **20** | RASSF2_1 |
| **9** | HS3ST2_19130 | **21** | SFRP1_9381 |
| **10** | LY6K | **22** | SGK_18737 |
| **11** | NCBP1_18440 | **23** | ZNF655 |
| **12** | PCDHGA12_18516 | | |

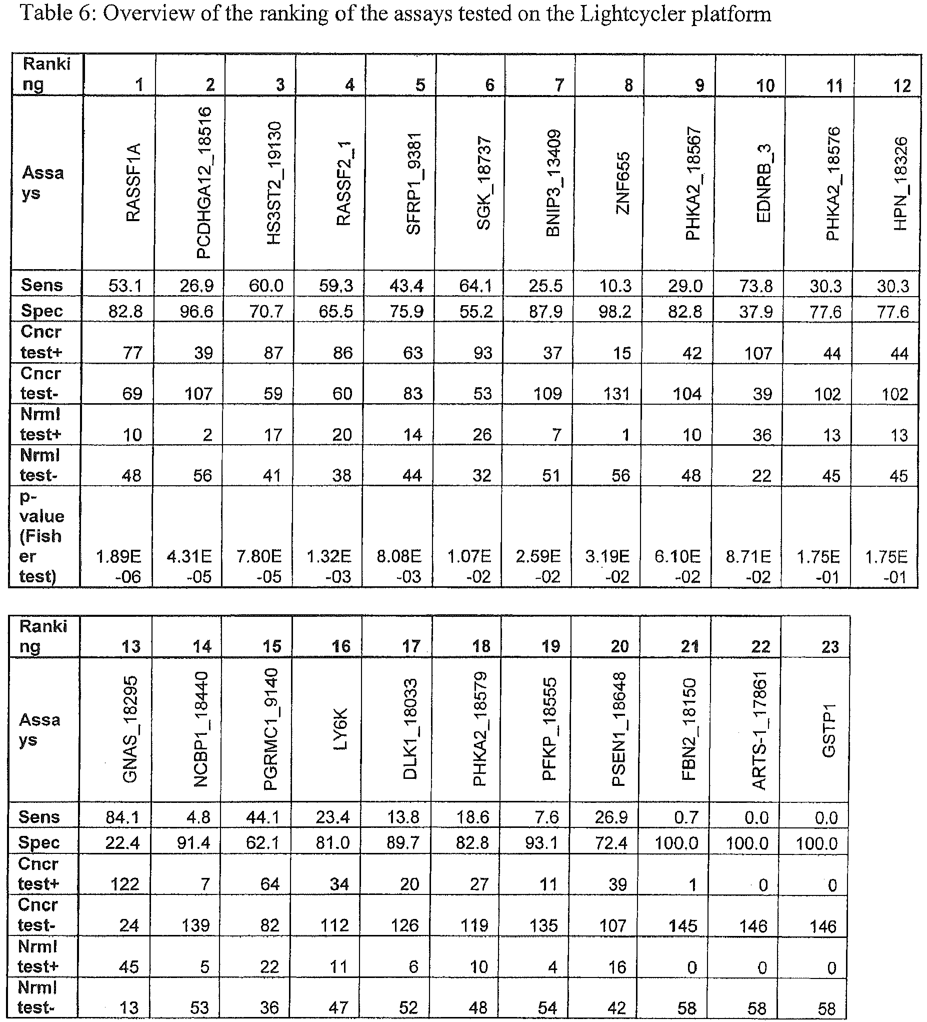

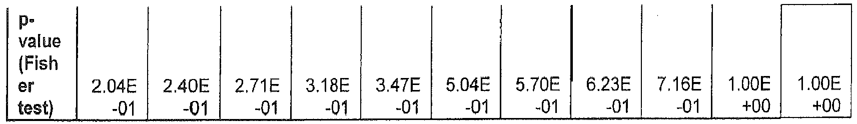

A comparison between the results coming from the Base 5 and the Lightcycler platforms has been performed.

Most of the interesting assays discovered on the Base 5 platform were confirmed on the Lightcycler platform.

### EXAMPLE 4: QMSP

Nineteen genes (APC2, BMP7, BNIP3, DLK1, DPYSL4, GSTP1, HS3ST2, JAM3, LOX, LY6K, NID2, PCDHGA12, PGRMC1, PHKA2, RASSF1A, RASSF2, SFRP1, SOX17, SULF2), were further selected based on the ranking on the Base 5 and/or Lightcycler platforms (marker discovery). For these assays, qMSPs using molecular beacons as detection system were designed (3 designs are evaluated per assay) and tested on control samples (cell lines). Several parameters (background, dynamic of the curve, highest range in fluorescence between beginning of the amplification and plateau phase, etc) were checked. In this phase of assay development, PCR material was used for generating the standard curves (instead of plasmids).

These assays were further verified on lung tissue samples collected by Ulg (Liège, Belgium), VUmc (Amsterdam, The Netherlands), UMCG (Groningen, The Netherlands) and Durham VA Medical Center (Durham, NC, USA) (normal PE tissue samples #60, cancer PE tissue samples #86 (adenocarcinoma #30, squamous cell carcinoma #15, large cell carcinoma #6, carcinoid #1, neuroendocrine #1, NSCLC #33)). DNA was isolated from the lung tissue samples using a phenol-chloroform procedure, quantified using the picogreen method and 1 µg of DNA was bisulphite treated using the ZYMO kit.

The primers and molecular beacons used for the different qMSPs are summarized in Table 7. The amplicons are summarized in Table 8. qMSPs were carried out in a total volume of 12 µl in 384 well plates in an ABI PRISM 7900HT instrument (Applied Biosystems). The final reaction mixture consisted of in-house qMSP buffer (including 80.4 nmol of MgCl2), 60 nmol of each dNTPs, 0.5 U of Jump Start Taq polymerase (SIGMA), 72ng of forward primer, 216 ng of reverse primer, 1.92 pmol of molecular beacon, 6.0 pmol of ROX (passive reference dye) and 50 ng of bisulphite converted genomic DNA. Thermal cycling was initiated with an incubation step of 5 minutes at 95°C, followed by 45 cycles (95°C for 30 seconds, 57°C for 30 seconds, 72°C for 30 seconds). The last step was performed at 72°C for 5 minutes. These conditions were similar for all the test genes as well as for *ACTB.*

Ct values were determined using the SDS software (version 2.2.2) supplied by Applied Biosystems with automatic baseline settings and threshold. The slopes and R² values for the different standard curves were determined after exporting data into excel.

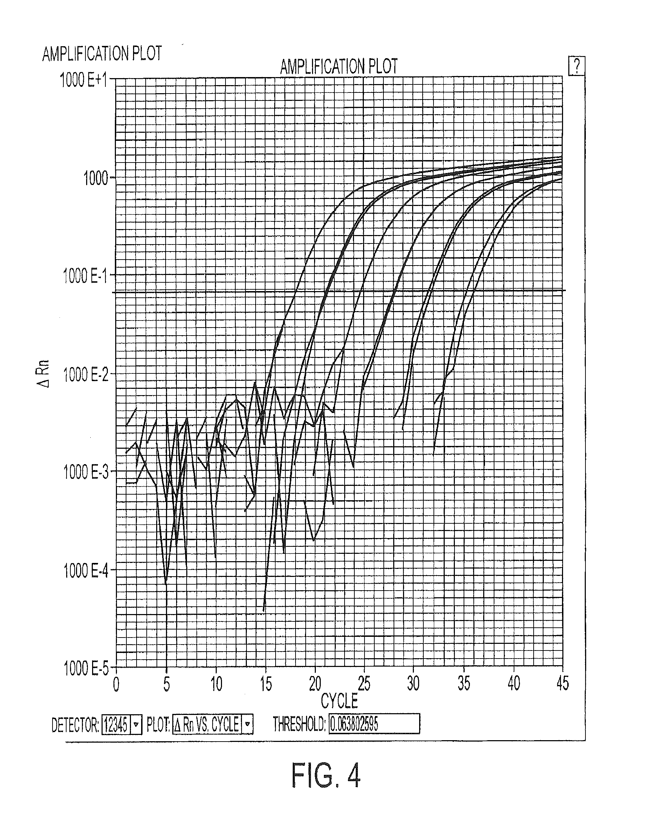

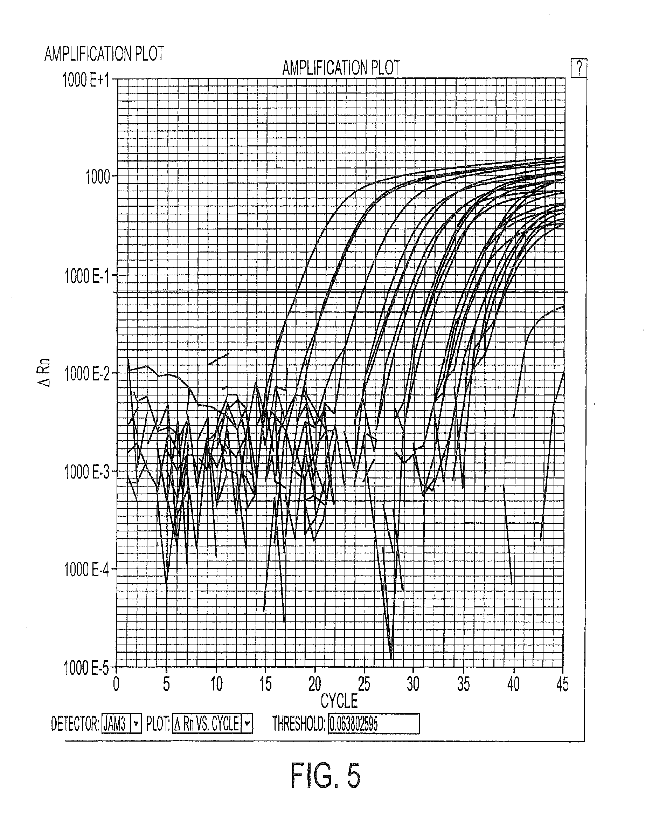

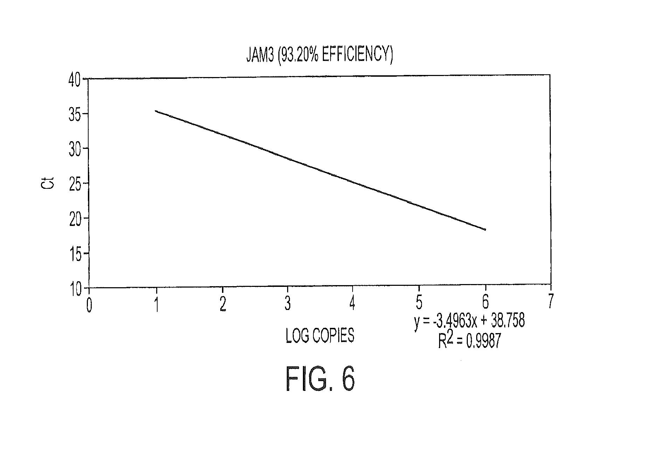

As an example, Figure 4 shows the amplification plot for *JAM3* obtained for the standard curve (960000 copies to 9.6 copies of the gene) and Figure 5 shows the amplification plot for *JAM3* obtained for the standard curve and for some samples. The Ct values plotted against the Log Copies of *JAM3* (Figure 6) give a R² of 0.9987 and the efficiency of the reaction is 93.20%.

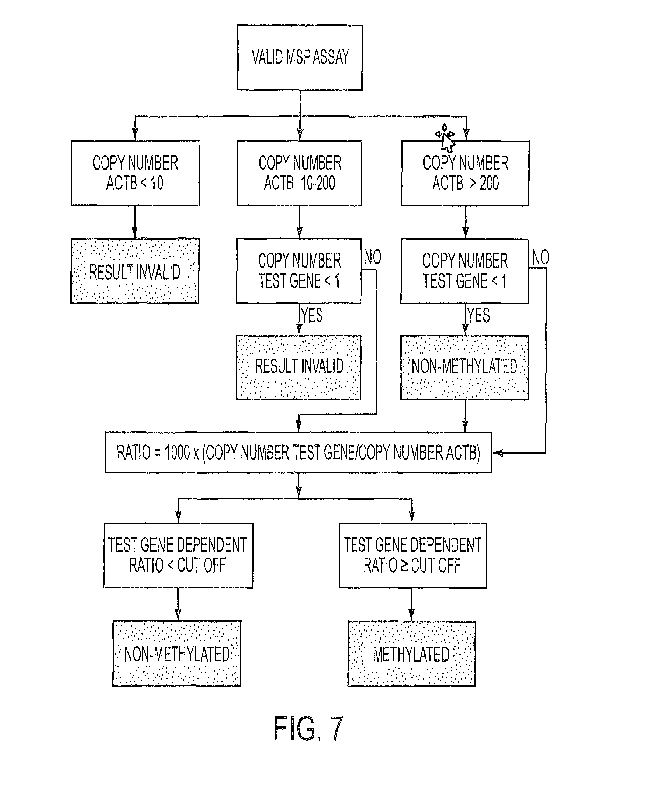

In addition to the test genes, the independent reference gene ACTB was also measured. The ratios between the test genes and ACTB were calculated to generate the test result. The samples were classified as methylated, unmethylated, or invalid based on the decision tree shown in Figure 7.

**Table 7: qMSP primers and molecular beacons sequences**

| **Gene ID** | **Symbol** | **Assay** | **Sense primer sequence (5' - 3') (SEQ ID NO:345-365, respectively)** | **Antisense primer sequence (5' - 3') (SEQ ID NO: 366-386, respectively)** | **Molecular Beacon (5' - 3') (modification beacons : 5' FAM, 3' DABCYL) (SEQ ID NO: 387-407, respectively)** |
|---|---|---|---|---|---|
| 10297 | *APC2* | APC2 | TTATATGTCGGTTAC GTGCGTTTATAT | GAACCAAAACGCTC CCCAT | CGTCTGCCCCGTCGAAAACCCG CCGATTAACGCAGACG |
| 655 | *BMP7* | BMP7_17911 | AGCGTAGAGATAGG TTGGTAACG | AAAACGATAACCCT TAAACCGA | CGACATGCGCGGAGGGGTTAG CGTGGTTGCATGTCG |
| 664 | *BNIP3* | BNIP3 | TACGCGTAGGTTTTA AGTCGC | TCCCGAACTAAACG AAACCCCG | CGACATGCCTACGACCGCGTC GCCCATTAGCATGTCG |
| 8788 | *DLK1* | DLK1_68536 | AAAGTTAGTAGGAG TAAGAGGACGC | AATACGACGCCAAA AACCG | CGACATGCGGGCGGTCGGGGT CGCGCATGTCG |
| 10570 | *DPYSL4* | DPYSL4_18050 | GGGGTTATAGTTTGG CGTTC | GCTCTAAAAACCAC ACCCGTC | CGACATGCGGTTCGGGTTATTT GCGAAGGAGTCGGCATGTCG |
| 2950 | *GSTP1* | GSTPi current | TTCGGGGTGTAGCG GTCGTC | GCCCCAATACTAAA TCACGACG | CGTCTGCTTGGGGTCGGCGGG AGTTCGCGGGATTGCAGACG |
| 9956 | *HS3ST2* | HS3ST2_2 | GTTTCGGGGTTCGTT TTTC | CGACTCGCTCTATCT CGCAC | CGACATGCACGCGCGCACCCC AACCCAGCATGTCG |
| 9956 | *HS3ST2* | HS3ST2_8 | AGTTTTCGGAGAAG ACGGC | ACGACTAAACTACT ATAACCCTACGA | CGACATGCACCACGACCACGC GAATCGAACGCATGTCG |
| 9956 | *HS3ST2* | HS3ST2_nor | CGTTAGGTTATTTTT TAAATAGAGTCGGT AGC | CGACTATACGAACT AACGAATAAACCG | CGACATGCACGCCGACCGCGA TCTAACTCGCATGTCG |
| 83700 | *JAM3* | JAM3 | GGGATTATAAGTCG CGTCGC | CGAACGCAAAACCG AAATCG | CGACACGATATGGCGTTGAGG CGGTTATCGTGTCG |
| 4015 | *LOX* | LOX_25068 | CGTGAATAAATAGT TGAGGGGC | GACAATCCCGAAAA ACGAAC | CGTCTGCCACAAACCGTTCTAA CCCGACCGCGCAGACG |
| 54742 | *LY6K* | LY6K | GCGGGGTTTTTTTA TCGGTTAGATTC | CAACGATACCCAAA AAAAATCAACGCG | CGACATGCCGACGCCCCCTCCC CGGCATGTCG |
| 22795 | *NID2* | NID2_9091 | GCGGTTTTTAAGGA GTTTTATTTTC | CTACGAAATTCCCTT TACGCT | CGACATGGGTTCGTAAGGTTTG GGGTAGCGGCCATGTCG |
| 26025 | *PCDHGA12* | PCDHGA12_1851 6 | AACGATTTGGGGTT AGAGTTTC | TAACCAAACTACCG CTTTACGA | CGACATGCGCGCTCCGCCAACT CCGCATGTCG |
| 10857 | *PGRMC1* | PGRMC1_9140 | CGTTCGTATAGAGTT CGGTAATGTC | CCTATAACTAAACG CGACGCAC | CGACATGCGGGGTTTAGAGGA GGGTAGCGCGCATGTCG |
| 5256 | *PHKA2* | PHKA2_70210 | TCGTCGTTTTAGTTT TCGTTTTC | ACGCTAACCCCAAA ATCCG | ACTCCGCGTTTTTTCGTCGCGC GGAGT |
| 11186 | *RASSF1A* | RASSF1A | GCGTTGAAGTCGGG GTTC | CCCGTACTTCGCTAA CTTTAAACG | CGTCTGCGTGGTTTCGTTCGGT TCGCGTTTGTTAGGCAGACG |
| 9770 | *RASSF2* | RASSF2_2b | AGGTAGGTTTTAGTT TTCGGC | GACCTCAAACACCA ACTCCG | CGACATGCGGGTGCGCGGGGG TCGTTGGGCATGTCG |
| 6422 | *SFRP1* | SFRP1 | TGTAGTTTTCGGAGT TAGTGTCGCGC | CCTACGATCGAAAA CGACGCGAACG | CGACATGCTCGGGAGTCGGGG CGTATTTAGTTCGTAGCGGCAT GTCG |
| 64321 | *SOX17* | SOX17_66072 | GAGATGTTTCGAGG GTTGC | CCGCAATATCACTA AACCGA | CGACATGCGTTCGTGTTTTGGT TTGTCGCGGTTTGGCATGTCG |
| 55959 | *SULF2* | SULF2_Bay | GTTAGTCGAGTTCGG AGGTATC | CAACTCCGAACGAA ACAATAAACG | CGACATGCCCGACGACTCTCG ACCTCCCGCATGTCG |

**Table 8: qMSP amplicon sequences**

| **Assay** | **Amplicon Sequence (converted) (5' - 3') (SEQ ID NO: 408-428, respectively)** | **Amplicon Sequence (non converted) (5' - 3') (SEQ ID NO: 429-449, respectively)** |
|---|---|---|
| APC2 | TTATATGTCGGTTACGTGCGTTTATATTTAGTTAAT CGGCGGGTTTTCGACGGGAATGGGGAGCGTTTTG GTTC | CCACATGTCGGTCACGTGCGCCCACACCCAGCCAA TCGGCGGGCTCCCGACGGGAATGGGGAGCGCCCT GGTCC |
| BMP7_17911 | AGCGTAGAGATAGGTTGGTAACGGTTTTAGGGAG GCGCGGAGGGGTTAGCGTGGTTGGTTTAAAAGGA TATAGGGATTGAGGGGTAAGATCGGTTTAAGGGT TATCGTTTT | AGCGCAGAGACAGGCTGGCAACGGCTTCAGGGAG GCGCGGAGGGGTCAGCGTGGCTGGCTTAAAAGGA TACAGGGACTGAGGGGCAAGACCGGCTCAAGGGT CACCGCTTC |
| BNIP3 | TACGCGTAGGTTTTAAGTCGCGGTTAATGGGCGAC GCGGTCGTAGATTCGTTCGGTTTCGTTTTGTTTTGT GAGTTTTTTCGGTCGGGTTGCGGGGTTTCGTTTAG TTCGGGA | CACGCGCAGGCCCCAAGTCGCGGCCAATGGGCGA CGCGGCCGCAGATCCGCCCGGCCCCGCCCTGCCCT GTGAGTTCCTCCGGCCGGGCTGCGGGGCTCCGCTC AGTCCGGGA |
| DLK1_68536 | AAAGTTAGTAGGAGTAAGAGGACGCGTAGGAGG TTTCGGTCGCGGTTATTTTTGGGCGGTCGGGGTCG CGGTTTCGGGAGCGGTGCGGGCGCGGGTTCGGTTT TTGGCGTCGTATT | AAAGCCAGCAGGAGCAAGAGGACGCGCAGGAGG GCTTCGGTCGCGGTCATCTCTGGGCGGCCGGGGTC GCGGTCCCGGGAGCGGTGCGGGCGCGGGTCCGGC TCCTGGCGCCGCACT |
| DPYSL4_18050 | GGGGTTATAGTTTGGCGTTCGGATTTTGGTTCGGG TTATTTGCGAAGGAGTCGGTTTTGGTTAAGGTGTT TTTTTGGACGGGTGTGGTTTTTAGAGC | GGGGTCACAGCCTGGCGCTCGGACCCTGGCCCGG GTCATCTGCGAAGGAGCCGGCTTTGGCCAAGGTG CCTTCCTGGACGGGTGTGGTTCCCAGAGC |
| GSTPi current | TTCGGGGTGTAGCGGTCGTCGGGGTTGGGGTCGGC GGGAGTTCGCGGGATTTTTTAGAAGAGCGGTCGG CGTCGTGATTTAGTATTGGGGC | CCCGGGGTGCAGCGGCCGCCGGGGCTGGGGCCGG CGGGAGTCCGCGGGACCCTCCAGAAGAGCGGCCG GCGCCGTGACTCAGCACTGGGGC |
| HS3ST2_2 | GTTTCGGGGTTCGTTTTCGGTAGGTTCGGGGAGA GGTGGGGTGATAATGGGTTGGGGTGCGCGCGTGT TTTATAGGTGCGAGATAGAGCGAGTCG | GCTCCGGGGCTCGCTCTCCGGCAGGCCCGGGGAG AGGTGGGGTGACAATGGGTTGGGGTGCGCGCGTG CCTCATAGGTGCGAGACAGAGCGAGCCG |
| HS3ST2_8 | AGTTTTCGGAGAAGACGGCGTTTTTAACGTTCGAT TCGCGTGGTCGTGGTAGCGTTACGCGAGTTTTTTA GGCGATCGTAGGGTTATAGTAGTTTAGTCGT | AGCCCCCGGAGAAGACGGCGCCCCCAACGCCCGA CCCGCGTGGCCGTGGCAGCGCCACGCGAGCCCTCT AGGCGACCGCAGGGCCACAGCAGCTCAGCCGC |
| HS3ST2_nor | CGTTAGGTTATTTTTAAATAGAGTCGGTAGCGCG TTTCGTTCGGTATTTTTCGAAGAGTTAGATCGCGG TCGGCGTTAGCGTTATCGTTCGGTTTATTCGTTAGT TCGTATAGTCG | CGTCAGGCCACTCCTTAAATAGAGCCGGCAGCGC GCTCCGCTCGGCATTTCCCGAAGAGCCAGATCGCG GCCGGCGCCAGCGCCACCGTCCGGTCCACCCGCC AGCCCGCACAGCCG |
| JAM3 | GGGATTATAAGTCGCGTCGCGTTGTCGTTGGTTTT TTAGTAATTTTCGATATGGCGTTGAGGCGGTTATC GCGATTTCGGTTTTGCGTTCG | GGGACTACAAGCCGCGCCGCGCTGCCGCTGGCCC CTCAGCAACCCTCGACATGGCGCTGAGGCGGCCA CCGCGACTCCGGCTCTGCGCTCG |
| LOX_25068 | CGTGAATAAATAGTTGAGGGGCGGTCGGGTTAGA ACGGTTTGTGTAATTTTGTAAACGTGTTAGAAAGT TTAAAATTTTTTTTTTTTTTTTATTTTAGATATTGT TCGTTTTTCGGGATTGTC | CGTGAACAAATAGCTGAGGGGCGGCCGGGCCAGA ACGGCTTGTGTAACTTTGCAAACGTGCCAGAAAGT TTAAAATCTCTCCTCCTTCCTTCACTCCAGACACTG CCCGCTCTCCGGGACTGCC |
| LY6K | GCGGGGTTTTTTTATCGGTTAGATTCGGGGAGAG GCGCGCGGAGGTTGCGAAGGTTTTAGAAGGGCGG GGAGGGGGCGTCGCGCGTTGATTTTTTTGGGTAT CGTTG | GCGGGGCTCCCCCTACCGGCCAGACCCGGGGAGA GGCGCGCGGAGGCTGCGAAGGTTCCAGAAGGGCG GGGAGGGGGCGCCGCGCGCTGACCCTCCCTGGGC ACCGCTG |
| NID2_9091 | GCGGTTTTTAAGGAGTTTTATTTTCGGGATTAAAT GGTTCGTAAGGTTTGGGGTAGCGGCGTTGTAGGA GATGAGTTTAGCGTAAAGGGAATTTCGTAG | GCGGCCCCCAAGGAGCCCACCCCCGGGACCAAA TGGCCCGCAAGGTTTGGGGCAGCGGCGTTGCAGG AGATGAGCTCAGCGCAAAGGGAACCCCGCAG |
| PCDHGA12_185 16 | AACGATTTGGGGTTAGAGTTTCGGGAGTTGGCGG AGCGCGGAGTTCGTATCGTTTTTAGAGGTAGGACG TAGTTTTTTTTTTGAATTCGTAAAGCGGTAGTTTG GTTA | AACGACCTGGGGCTAGAGCCCCGGGAGCTGGCGG AGCGCGGAGTCCGCATCGTCTCCAGAGGTAGGAC GCAGCTTTTCTCTCTGAATCCGCAAAGCGGCAGCT TGGTCA |
| PGRMC1_9140 | CGTTCGTATAGAGTTCGGTAATGTCGAGGTTTTT TAACGGGTCGGTTTGCGAGGAGTAAAAAGGGGT TTAGAGGAGGGTAGCGCGTGCGTCGCGTTTAGTTA TAGG | CGCTCGCACAGAGCCCGGCAATGCCGAGGCCCTC CCAACGGGTCGGTCTGCGAGGAGCAAAAAAGGGG TTCAGAGGAGGGCAGCGCGTGCGTCGCGCTCAGC TATAGG |
| PHKA2_70210 | TCGTCGTTTTAGTTTTCGTTTTCGTTTTCGCGTTTTT TCGTCGCGCGGAGTTTTGGTTGGTTTGTTTTTTAAT CGGATTTTGGGGTTAGCGT | TCGTCGCTCCAGCCCCCGTCCCCGCCCCCGCGCCT CCCCGCCGCGCGGAGCTCTGGTTGGCTTGCTTTCC AACCGGACTTTGGGGCTAGCGT |
| RASSF1A | GCGTTGAAGTCGGGGTTCGTTTTGTGGTTTCGTTC GGTTCGCGTTTGTTAGCGTTTAAAGTTAGCGAAGT ACGGG | GCGCTGAAGTCGGGGCCCGCCCTGTGGCCCCGCCC GGCCCGCGCTTGCTAGCGCCCAAAGCCAGCGAAG CACGGG |
| RASSF2_2b | AGGTAGGTTTTAGTTTTCGGCGCGGGGAGGCGGC GCGTTTTAGAGGGGCGTAGGTGCGCGGGGGTCG TTGGTTTTTCGGGTATTTTTTTTTGCGGTTTTTCG TTTTTTTTCGGAGTTGGTGTTTGAGGTC | AGGCAGGTCCCAGTCCCCGGCGCGGGAGGCGGC GCGCTTCAGAGGGGCGCAGGGTGCGCGGGGGCCG TTGGCCCTCCGGGCACTTCCCCTTTGCGGTCTCCCC GCCCTCCTTCGGAGC |
| SFRP1 | TGTAGTTTTCGGAGTTAGTGTCGCGCGTTCGTCGT TTCGCGTTTTTTTGTTCGTCGTATTTTCGGGAGTCG GGCGTATTTAGTTCGTAGCGTCGTTTTTCGTTCG CGTCGTTTTCGATCGTAGG | TGCAGCCTCCGGAGTCAGTGCCGCGCGCCCGCCGC CCCGCGCCTTCCTGCTCGCCGCACCTCCGGGAGCC GGGGCGCACCCAGCCCGCAGCGCCGCCTCCCCGC CCGCGCCGCCTCCGACCGCAGG |
| SOX17_66072 | GAGATGTTTCGAGGGTTGCGCGGGTTTTTCGGTTC GAAGTCGTCGTTCGTGTTTTGGTTTGTCGCGGTTTG GTTTATAGCGTATTTAGGGTTTTTAGTCGGTTTAGT GATATTGCGG | GAGATGCCCCGAGGGCTGCGCGGGTCTCCCGGCC CGAAGCCGCCGCCCGTGTTCTGGCCTGTCGCGGTC TGGTCTACAGCGTACCCAGGGCCCCCAGCCGGCCT AGTGACACTGCGG |
| SULF2_Bay | GTTAGTCGAGTTCGGAGGTATCGGGAGGTCGAGA GTCGTCGGGATTTTAGTTTTGCGTTTATTGTTTCGT TCGGAGTTG | GCCAGCCGAGTCCGGAGGCATCGGGAGGTCGAGA GCCGCCGGGACCCCAGCTCTGCGTTCACTGCCCCG TCCGGAGCTG |

The highest methylation value of the normal tissue specimens was taken as a directive to define a cut off above which the cases were considered to be methylated. The analytical cut off was finally set to give the highest possible specificity and/or above 3 times STDEV (Normal) (excluding outliers).

The one-tailed Fisher's exact test as described above was used as a scoring function to rank the candidate markers (Journal of Statistical Software, vol. 08, issue i21, 2003).

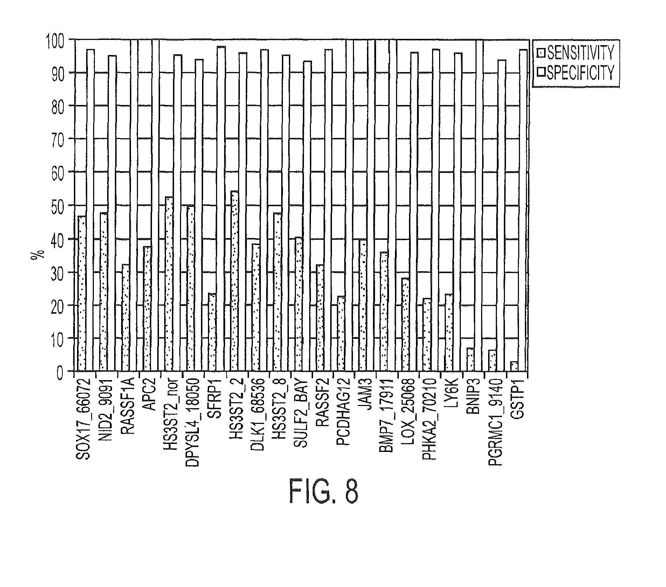

Table 9 summarizes the results obtained for *JAM3.* Table 10 summarizes the results obtained for all the tested markers on tissue samples. The individual performances of the assays are shown in Figure 8 and the assays are ranked according their p-value (Fisher's exact test). The best performing markers were further tested on clinical samples (sputum samples).

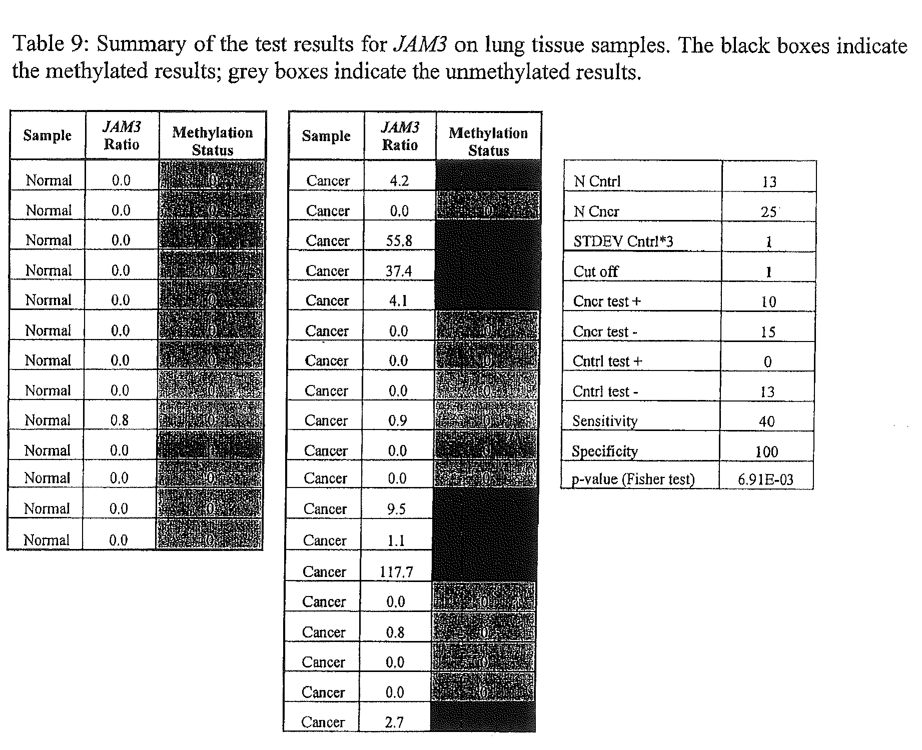

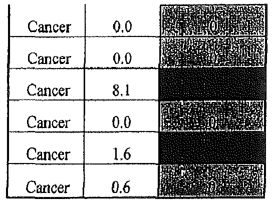

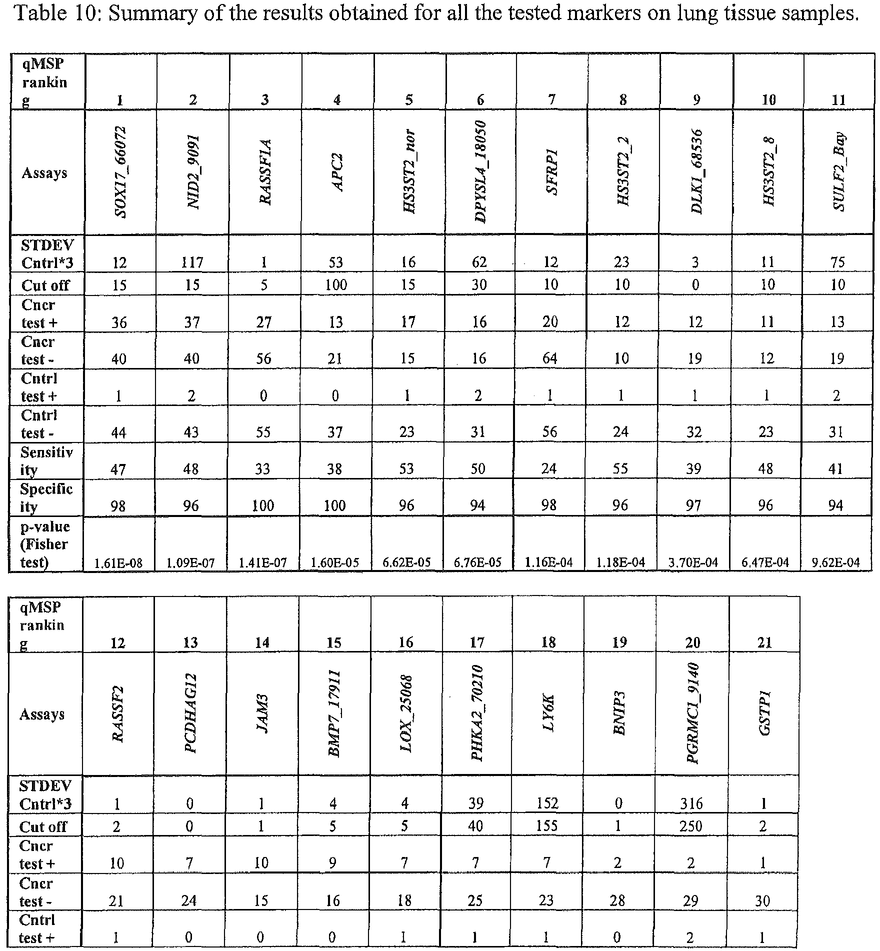

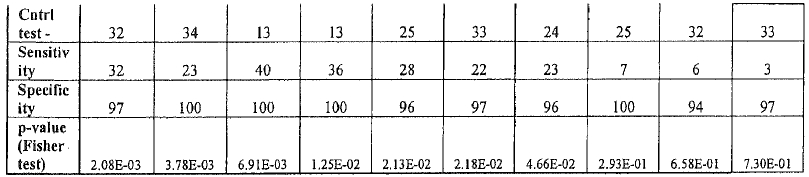

### EXAMPLE 5: Best performing markers tested on sputum samples

The control sputum samples were collected from the Lung Cancer Clinical Collaborative Research Agreement study of ONCO with the UMCG hospital (Groningen, The Netherlands). These samples were taken from participants to the NELSON screening program (a randomized controlled screening trial for lung cancer using multi-slice low-dose CT in high risk subjects - current smokers (55%) and former smokers (45%) who (had) smoked at least 16 cigarettes a day for at least 26 years or at least 11 cigarettes a day for at least 31 years).

The cancer sputum samples (stage IA #2, stage IIIA #3, stage IIIB #1, stage IV #1, stage unknown #1) were collected from the Lung Cancer Clinical Collaborative Research Agreement study of ONCO with Durham VA Medical Center (Durham, NC, USA). Patients with histologically proven NSCLC or patients suspected of having NSCLC planning to undergo resection and who have a predicted probability of 75% or more of having NSCLC (*e.g*., using nomograms such as at the worldwide web domain chestx-ray.com, at the page SPN/SPNProb.html) were included in the study.

Subjects were provided with a sterile cup containing Saccomanno's fixative and instructed to take a deep breath, cough deeply, and expectorate into the cup for 3 consecutive days. The samples were centrifuged at 1500xg for 15min to sediment all cellular material, the supernatants were removed and the cell pellet was washed with PBS. DNA was extracted from the sputum cells using standard salt-chloroform extraction and ethanol precipitation for high molecular DNA and dissolved in 250 µL TE buffer (10 mM Tris; 1 mM EDTA (pH 8.0)). DNA was quantified using the picogreen method and 20µg (or maximum amount if less than 20µg recovered from DNA extraction) of DNA was bisulphite treated using the EpiTect bisulfite kit (QIAGEN).

QMSP was performed after bisulphite treatment on denatured genomic DNA. The assays were carried out as described above, except that 960 ng of bisulphite converted genomic DNA was added in the reaction mixture. The samples were classified as methylated, unmethylated, or invalid as described above. The results based on **ratio** (copy number gene tested / copy number ACTB) and based on **copy number** obtained for all the tested markers on sputum samples from lung cancer patients and from control patients were ranked according their p-value (Fisher's exact test) (Table 11 - ratio, Table 12 - copy number).

Several combinations of markers were investigated to maximize sensitivity of detection, without significantly compromising specificity. The samples were classified as methylated if at least one of the tested markers scored positive based on ratio or based on copy number. Examples of the performance of combination of markers are summarized in Table 13 (ratio) and in Table 14 (copy number). Specificity above 90% is obtained for some combinations of markers (based on ratio and copy number). Sensitivity of 100% is obtained for some combinations of markers (based copy number).

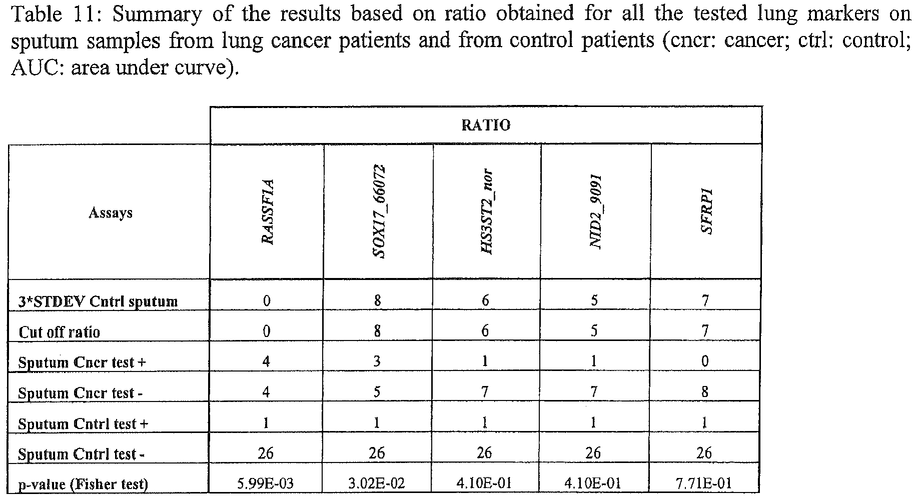

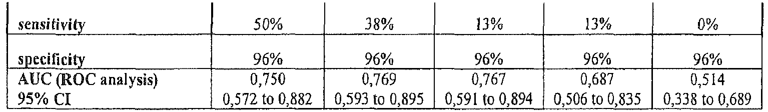

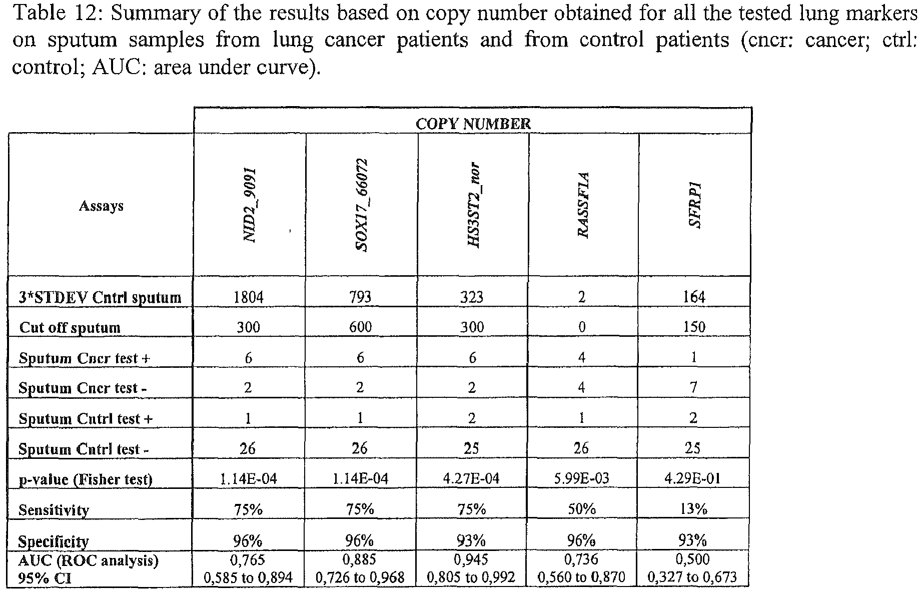

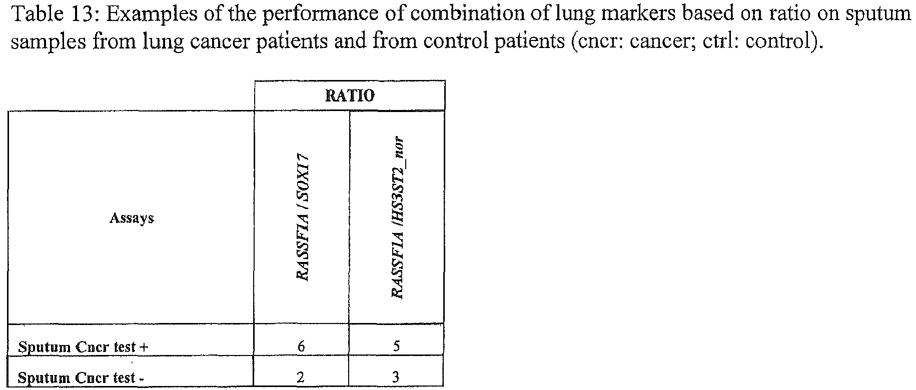

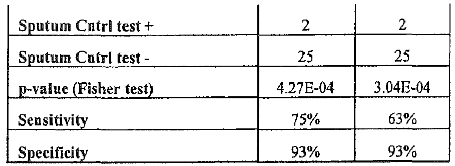

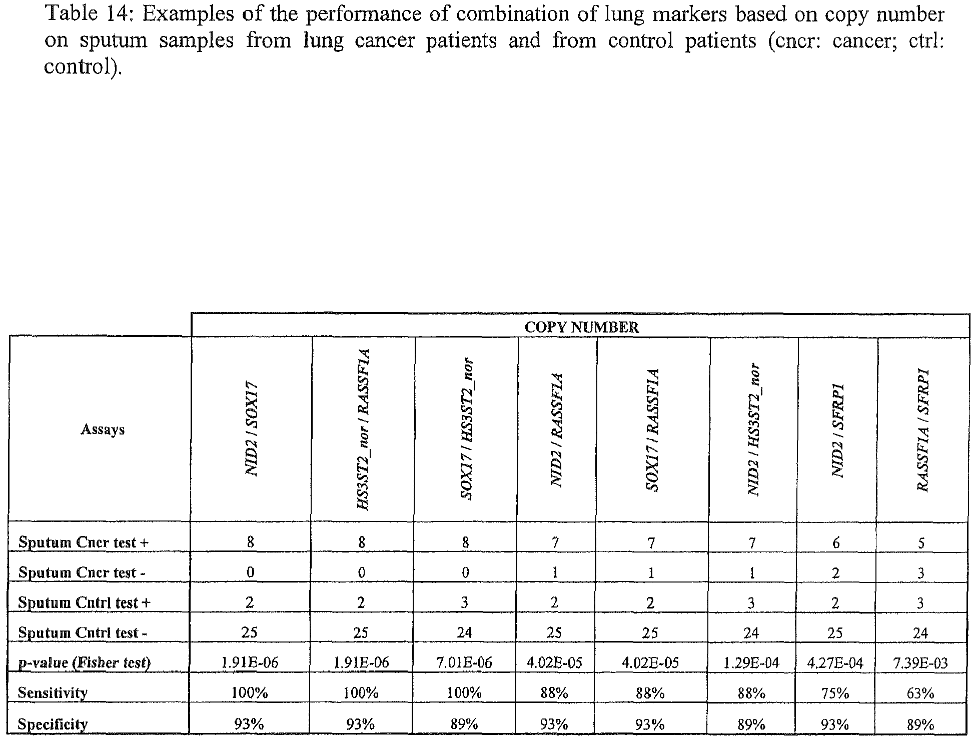

### References

The disclosure of each reference cited in this disclosure is expressly incorporated herein.
Barringer KJ, Orgel L, Wahl G, Gingeras TR.Gene. 1990 Apr 30;89(1):117-22
Esteller M, Corn PG, Baylin SB, Herman JG. A gene hypermethylation profile of human cancer. Cancer Res. 2001 Apr 15;61(8):3225-9.
Cross, SH et al. Nature Genetics 1994, 6, 236-244
Deng, D. et al. Simultaneous detection of CpG methylation and single nucleotide polymorphism by denaturing high performance liquid chromatography. 2002 Nuc. Acid Res, 30, 3.
Ganti, and Mulshine. Lung cancer screening. The Oncologist 2006, Vol. 11, No. 5, 481-487
Gentleman RC, Carey VJ, Bates DM, Bolstad B, Dettling M, et al. (2004) Bioconductor: open software development for computational biology and bioinformatics. Genome Biol 5: R80.
Greenberg and Lee. Biomarkers for Lung Cancer: Clinical Uses Curr Opin Pulm Med.2007;13(4):249-255.
Greenlee, Hill-Harmon, Murray and Thun, Cancer statistics 2001, CA Cancer J. Clin. 2001; 51: 15-36.
Ihaka R, Gentleman RC (1996) A language for data analysis and graphics. Journal of Computational and Graphical Statistics 5: 299-314.
Kwoh DY, Davis GR, Whitfield KM, Chappelle HL, DiMichele LJ, Gingeras TR. Transcription-based amplification system and detection of amplified human immunodeficiency virus type 1 with a bead-based sandwich hybridization format. Proc Natl Acad Sci U S A. 1989 Feb;86(4):1173-7.
Li KB. ClustalW-MPI: ClustalW analysis using distributed and parallel computing. Bioinformatics 2003;19(12):1585-6.
Schuebel KE, Chen W, Cope L, Glöckner SC, Suzuki H, Yi JM, Chan TA, Van Neste L, Van Criekinge W, van den Bosch S, van Engeland M, Ting AH, Jair K, Yu W, Toyota M, Imai K, Ahuja N, Herman JG & Baylin SB (2007). Comparing the DNA hypermethylome with Gene Mutations in Human Colorectal Cancer. PLoS Genetics, 3 (8), Early Online Release*.*
Shiraisi, M et al. Biol Chem. 1999, 380(9):1127-1131
Sjöblom T, Jones S, Wood LD, Parsons DW, Lin J, Barber TD, Mandelker D, Leary RJ, Ptak J, Silliman N, Szabo S, Buckhaults P, Farrell C, Meeh P, Markowitz SD, Willis J, Dawson D, Willson JK, Gazdar AF, Hartigan J, Wu L, Liu C, Parmigiani G, Park BH, Bachman KE, Papadopoulos N, Vogelstein B, Kinzler KW & Velculescu VE (2006). The Consensus Coding Sequences of Human Breast and Colorectal Cancers. Science, 314 (5797), 268-274.
Straub, J. et al., A64-AACRMD (2007): Base5, a versatile, highly integrated high-throughput methylation profiling platform for Methylation-Specific PCR based marker identification applied to CRC
Suzuki Y, Yamashita R, Sugano S, Nakai K. DBTSS, DataBase of Transcriptional Start Sites: progress report 2004. Nucleic Acids Res 2004;32(Database issue):D78-81.
Suzuki Y, Yamashita R, Nakai K, Sugano S. DBTSS: DataBase of human Transcriptional Start Sites and full-length cDNAs. Nucleic Acids Res 2002;30(1):328-31.
Thompson JD, Higgins DG, Gibson TJ. CLUSTAL W: improving the sensitivity of progressive multiple sequence alignment through sequence weighting, position-specific gap penalties and weight matrix choice. Nucleic Acids Res 1994; 22(22):4673-80.
Tost, J. et al. Analysis and accurate quantification of CpG methylation by MALDI mass spectrometry. Nuc. Acid Res, 2003, 31(9): e50
Travis W.D., Pathology of lung cancer, Clin. Chest Med. 23 (2002), 65-81.
Trooskens G, De Beule D, Decouttere F, Van Criekinge W. Phylogenetic trees: visualizing, customizing and detecting incongruence. Bioinformatics 2005; 21(19):3801-2.

### CLAUSES

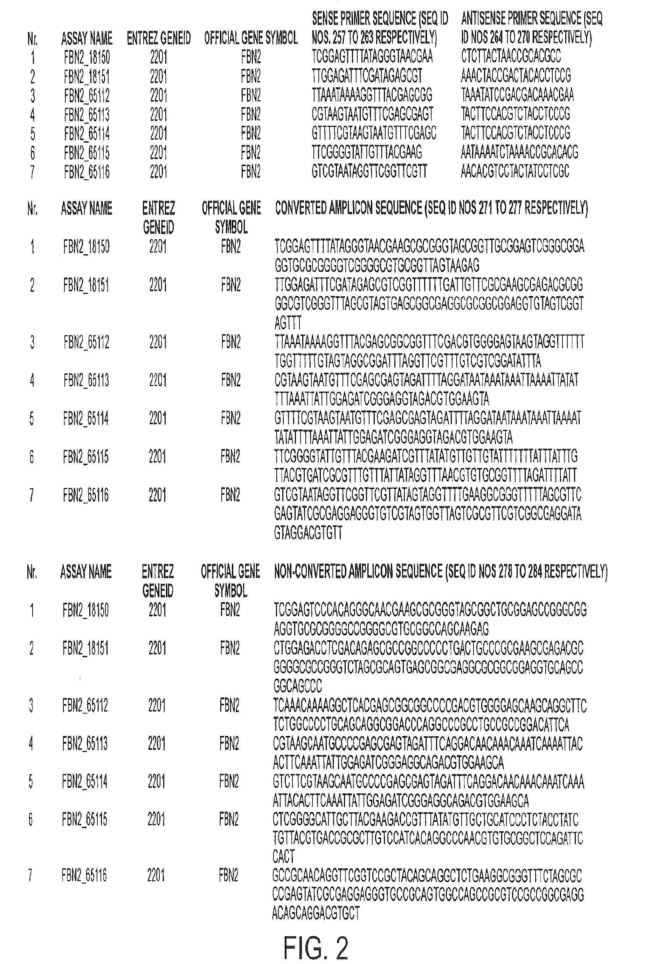

1. A method for identifying lung cancer or its precursor, or predisposition to lung cancer, comprising:
   detecting in a test sample containing lung cells or nucleic acids from lung cells, epigenetic modification of at least one gene selected from the group consisting of DPYSL4, SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; and
   identifying the test sample as containing cells that are neoplastic, precursor to neoplastic, or predisposed to neoplasia, or as containing nucleic acids from cells that are neoplastic, precursor to neoplastic, or predisposed to neoplasia.
2. The method of clause 1 wherein the test sample contains squamous cells or nucleic acids from squamous cells.
3. The method of clause 1 wherein the test sample contains adenocarcinoma cells or nucleic acids from adenocarcinoma cells.
4. The method of clause 1 wherein the test sample contains large cell carcinoma cells or nucleic acids from large cell carcinoma cells.
5. The method of clause 1 wherein the test sample contains a mixture of squamous cells, adenocarcinoma cells, and large cell carcinoma cells.
6. The method of clause 1 wherein the test sample is from a specimen selected from the group consisting of a tissue specimen, a biopsy specimen, a surgical specimen, a cytological specimen, sputum specimen, pleural fluid and a bronchoalveolar lavage.
7. The method of clause 6 wherein the test sample is from a biopsy specimen and surgical removal of neoplastic tissue is recommended to the patient
8. The method of clause 6 wherein the specimen is a surgical specimen and adjuvant chemotherapy or adjuvant radiation therapy is recommended to the patient.
9. The method of clause 1 wherein an epigenetic modification in a panel of genes comprising two, three, four or five genes is detected, wherein detection of an epigenetic change in at least one of the genes in the panel is indicative of a predisposition to, or the incidence of lung cancer.
10. The method of clause 9 wherein epigenetic modification of RASSF1A and/or SOX17 and/or HS3ST2-nor and/or NID2 and/or SFRP1 is detected
11. The method of clause 1 wherein epigenetic modification is detected by detecting methylation of a CpG dinucleotide motif in the gene.
12. The method of clause 1 wherein epigenetic modification is detected by detecting methylation of a CpG dinucleotide motif in a promoter, intron or exon of the gene.
13. The method of clause 1 wherein epigenetic modification is detected by detecting diminished expression of mRNA of the gene.
14. The method of clause 11 wherein methylation is detected by contacting at least a portion of the gene with a methylation-sensitive restriction endonuclease, said endonuclease preferentially cleaving methylated recognition sites relative to non-methylated recognition sites, whereby cleavage of the portion of the gene indicates methylation of the portion of the gene.
15. The method of clause 11 wherein methylation is detected by contacting at least a portion of the gene with a methylation-sensitive restriction endonuclease, said endonuclease preferentially cleaving non-methylated recognition sites relative to methylated recognition sites, whereby cleavage of the portion of the gene indicates non-methylation of the portion of the gene provided that the gene comprises a recognition site for the methylation-sensitive restriction endonuclease.
16. The method of clause 11 wherein methylation is detected by:
   contacting at least a portion of the gene of the test sample with a chemical reagent that selectively modifies a non-methylated cytosine residue relative to a methylated cytosine residue, or selectively modifies a methylated cytosine residue relative to a non-methylated cytosine residue; and detecting a product generated due to said contacting.
17. The method of clause 16 wherein the step of detecting a product employs amplification with at least one primer that hybridizes to a sequence comprising a modified non-methylated CpG dinucleotide motif but not to a sequence comprising an unmodified methylated CpG dinucleotide motif thereby forming amplification products.
18. The method of clause 16 wherein the step of detecting a product comprises amplification with at least one primer that hybridizes to a sequence comprising an unmodified methylated CpG dinucleotide motif but not to a sequence comprising a modified non-methylated CpG dinucleotide motif thereby forming amplification products.
19. The method of clause 16 wherein the product is detected by a method selected from the group consisting of electrophoresis, hybridization, amplification, sequencing, ligase chain reaction, chromatography, mass spectrometry, and combinations thereof.
20. The method of clause 16 wherein the chemical reagent is hydrazine.
21. The method of clause 20 further comprising cleavage of the hydrazine-contacted at least a portion of the gene with piperidine.
22. The method of clause 16 wherein the chemical reagent comprises bisulfite ions.
23. The method of clause 22 further comprising treating the bisulfite ion-contacted, at least a portion of the gene with alkali.
24. The method of clause 1 wherein the step of detecting employs amplification of at least a portion of the at least one gene using an oligonucleotide primer that specifically hybridizes under amplification conditions to a region of a gene selected from the group consisting of DPYSL4, SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site.
25. The method of clause 1 wherein the step of detecting employs amplification of at least a portion of the at least one gene using at least one pair of oligonucleotide primers that specifically hybridizes under amplification conditions to a region of a gene selected from the group consisting of DPYSL4, SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site.
26. The method of clause 25 wherein the region comprise, consist essentially of or consist of the sequences represented by SEQ ID NO. 129-192 and/or SEQ ID NO. 193-256 and/or SEQ ID NO. 315-329 and/or SEQ ID NO. 330-344 and/or SEQ ID NO. 408-428 and/or SEQ ID NO. 429-449 and/or SEQ ID NO. 271-277 and/or SEQ ID NO. 278-284.
27. The method of clause 1 wherein the step of detecting a product comprises amplification with at least one sense primer comprising, consisting essentially of or consisting of SEQ ID NO. 1-64 and/or SEQ ID NO. 285-299 and/or SEQ ID NO. 345-365 and/or SEQ ID NO. 257-263.
28. The method of clause 1 wherein the step of detecting a product comprises amplification with at least one antisense primer comprising, consisting essentially of or consisting of SEQ ID NO. 65-128 and/or SEQ ID NO. 300-314 and/or SEQ ID NO. 366-386 and/or SEQ ID NO. 264-270.
29. The method of clause 1 wherein the step of detecting employs amplification of at least a portion of the at least one gene, and further employs at least one oligonucleotide probe which hybridizes to an amplicon selected from the group consisting of SEQ ID NO: 129-292 and/or SEQ ID NO. 193-256 and/or SEQ ID NO. 315-329 and/or SEQ ID NO. 330-344 and/or SEQ ID NO. 408-428 and/or SEQ ID NO. 429-449 and/or SEQ ID NO. 271-277 and/or SEQ ID NO. 278-284.under amplification conditions.
30. The method of clause 29 wherein the probe comprises, consists essentially of or consists of sequences represented by SEQ ID NO. 387-407.
31. The method of clause 1 wherein the step of detecting employs amplification of at least a portion of the at least one gene and a detectable reagent which preferentially binds to double stranded DNA relative to single stranded DNA.
32. The method of clause 25 wherein an oligonucleotide probe is covalently linked to the oligonucleotide primer.
33. A kit for assessing lung cancer or its precursor, or predisposition to lung cancer in a test sample containing lung cells or nucleic acids from lung cells, said kit comprising in a package:
   a reagent that (a) modifies methylated cytosine residues but not non-methylated cytosine residues, or that (b) modifies non-methylated cytosine residues but not methylated cytosine residues; and
   at least one pair of oligonucleotide primers that specifically hybridizes under amplification conditions to a region of a gene selected from the group consisting of DPYSL4, SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site.
34. The kit of clause 33 wherein the at least one pair of primers is selected from Table 1 (SEQ ID NO: 1-128), Figure 2 (SEQ ID NO: 257-270), Table 3 (SEQ ID NO: 285-314) and Table 7 (SEQ ID NO: 345-386).
35. The kit of clause 33 wherein the at least one pair of oligonucleotide primers amplifies an amplicon selected from Table 2 (SEQ ID NO: 129-256), Figure 2 (SEQ ID NO: 271-284),Table 4 (SEQ ID NO: 315-344) and Table 8 (SEQ ID NO:408-449).
36. A kit for assessing lung cancer or its precursor, or predisposition to lung cancer in a test sample containing lung cells or nucleic acids from lung cells, said kit comprising in a package:
   at least two pairs of oligonucleotide primers that specifically hybridize under amplification conditions to a region of a gene selected from the group consisting of DPYSL4, SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site.
37. The kit of clause 36 wherein the at least two pairs of primers are selected from SEQ ID NO: 1-128 (Table 1), SEQ ID NO: 257-270 (Figure 2), SEQ ID NO:285-314 (Table 3), SEQ ID NO: 345-386 (Table 7).
38. The kit of clause 36 wherein the at least two pairs of oligonucleotide primers amplify amplicons selected from Table 2 (SEQ ID NO: 129-256), Figure 2 (SEQ ID NO: 271-284), Table 4 (SEQ ID NO: 315-344) and Table 8 (SEQ ID NO: 408-449).
39. The kit of clause 33 or 36 further comprising at least one oligonucleotide probe which hybridizes to an amplicon selected from the group consisting of Table 2 (SEQ ID NO: 129-256), Figure 2 (SEQ ID NO: 271-284), Table 4 (SEQ ID NO: 315-344), Table 8 (SEQ ID NO: 408-449) under amplification conditions.
40. The kit of clause 39 wherein the oligonucleotide probe is selected from the group consisting of SEQ ID NO: 387-407.
41. The kit of clause 40 wherein the oligonucleotide probe comprises a fluorescent label.
42. The kit of clause 40 wherein the oligonucleotide probe comprises a fluorescence quenching agent.
43. The kit of clause 40 wherein the oligonucleotide probe comprises a fluorescent label and fluorescence quenching agent.
44. The kit of clause 33 or 36 which comprises a detectable reagent which preferentially binds to double stranded DNA relative to single stranded DNA.
45. The kit of clause 33 or 36 further comprising a DNA polymerase for amplifying DNA.
46. The kit of clause 33 or 36 further comprising at least one oligonucleotide probe which is covalently linked to at least one of said oligonucleotide primers.
47. An isolated polynucleotide comprising a nucleotide sequence selected from the group consisting of SEQ ID NO: 1-449.
48. The polynucleotide of clause 41 which is detectably labeled.
49. The polynucleotide of clause 41 which is detectably labeled with a fluorescent label.
50. The isolated polynucleotide of clause 41 which consists of the selected nucleotide sequence.
51. The method of clause 1 wherein epigenetic modification is detected by detecting hypomethylation of a CpG dinucleotide motif in the gene.
52. The method of clause 1 wherein epigenetic modification is detected by detecting hypomethylation of a CpG dinucleotide motif in a promoter of the gene.
53. The method of clause 1 wherein epigenetic modification is detected by detecting increased expression of mRNA of the gene.

## Claims

1. A method for identifying lung cancer or its precursor, or predisposition to lung cancer, comprising:
detecting in a test sample containing lung cells or nucleic acids from lung cells, epigenetic modification of at least one gene selected from the group consisting of JAM3, SULF2, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; and
identifying the test sample as containing cells that are neoplastic, precursor to neoplastic, or predisposed to neoplasia, or as containing nucleic acids from cells that are neoplastic, precursor to neoplastic, or predisposed to neoplasia.

2. The method of claim 1 wherein:
(1) the test sample contains squamous cells or nucleic acids from squamous cells;
(2) the test sample contains adenocarcinoma cells or nucleic acids from adenocarcinoma cells;
(3) the test sample contains large cell carcinoma cells or nucleic acids from large cell carcinoma cells;
(4) the test sample contains a mixture of squamous cells, adenocarcinoma cells, and large cell carcinoma cells; or
(5) the test sample is from a specimen selected from the group consisting of a tissue specimen, a biopsy specimen, a surgical specimen, a cytological specimen, sputum specimen, pleural fluid and a bronchoalveolar lavage, wherein preferably the test sample is from a biopsy specimen and surgical removal of neoplastic tissue is recommended to the patient or the specimen is a surgical specimen and adjuvant chemotherapy or adjuvant radiation therapy is recommended to the patient.

3. The method of claim 1 wherein an epigenetic modification in a panel of genes comprising two, three, four or five genes is detected, wherein detection of an epigenetic change in at least one of the genes in the panel is indicative of a predisposition to, or the incidence of lung cancer, wherein preferably epigenetic modification of RASSF1A and/or SOX17 and/or HS3ST2-nor and/or NID2 and/or SFRP1 is detected.

4. The method of claim 1 wherein epigenetic modification is detected by detecting methylation of a CpG dinucleotide motif in the gene.

5. The method of claim 1 wherein epigenetic modification is detected by
(1) detecting methylation of a CpG dinucleotide motif in a promoter, intron or exon of the gene;
(2) detecting diminished expression of mRNA of the gene;
(3) detecting hypomethylation of a CpG dinucleotide motif in the gene;
(4) detecting hypomethylation of a CpG dinucleotide motif in a promoter of the gene; or
(5) detecting increased expression of mRNA of the gene.

6. The method of claim 4 wherein:
(1) methylation is detected by contacting at least a portion of the gene with a methylation-sensitive restriction endonuclease, said endonuclease preferentially cleaving methylated recognition sites relative to non-methylated recognition sites, whereby cleavage of the portion of the gene indicates methylation of the portion of the gene; or
(2) methylation is detected by contacting at least a portion of the gene with a methylation-sensitive restriction endonuclease, said endonuclease preferentially cleaving non-methylated recognition sites relative to methylated recognition sites, whereby cleavage of the portion of the gene indicates non-methylation of the portion of the gene provided that the gene comprises a recognition site for the methylation-sensitive restriction endonuclease.

7. The method of claim 4 wherein methylation is detected by:
contacting at least a portion of the gene of the test sample with a chemical reagent that selectively modifies a non-methylated cytosine residue relative to a methylated cytosine residue, or selectively modifies a methylated cytosine residue relative to a non-methylated cytosine residue; and
detecting a product generated due to said contacting.

8. The method of claim 7 wherein:
(1) the step of detecting a product employs amplification with at least one primer that hybridizes to a sequence comprising a modified non-methylated CpG dinucleotide motif but not to a sequence comprising an unmodified methylated CpG dinucleotide motif thereby forming amplification products;
(2) the step of detecting a product comprises amplification with at least one primer that hybridizes to a sequence comprising an unmodified methylated CpG dinucleotide motif but not to a sequence comprising a modified non-methylated CpG dinucleotide motif thereby forming amplification products;
(3) the product is detected by a method selected from the group consisting of electrophoresis, hybridization, amplification, sequencing, ligase chain reaction, chromatography, mass spectrometry, and combinations thereof;
(4) the chemical reagent is hydrazine, wherein preferably the method further comprises cleavage of the hydrazine contacted portion of the gene with piperidine; or
(5) the chemical reagent comprises bisulfite ions, wherein preferably the method further comprises treating the bisulfite ion-contacted portion of the gene with alkali.

9. The method of claim 1 wherein
(1) the step of detecting employs amplification of at least a portion of the at least one gene using an oligonucleotide primer that specifically hybridizes under amplification conditions to a region of a gene selected from the group consisting of JAM3, SULF2, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site;
(2) the step of detecting employs amplification of at least a portion of the at least one gene using at least one pair of oligonucleotide primers that specifically hybridizes under amplification conditions to a region of a gene selected from the group consisting of JAM3, SULF2, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site, wherein optionally an oligonucleotide probe is covalently linked to the oligonucleotide primer, and wherein it is further preferred that the region comprises, consists essentially of or consists of the sequences represented by SEQ ID NO. 129-192 and/or SEQ ID NO. 193-256 and/or SEQ ID NO. 315-329 and/or SEQ ID NO. 330-344 and/or SEQ ID NO. 408-428 and/or SEQ ID NO. 429-449 and/or SEQ ID NO. 271-277 and/or SEQ ID NO. 278-284;
(3) the step of detecting a product comprises amplification with at least one sense primer comprising, consisting essentially of or consisting of SEQ ID NO. 1-64 and/or SEQ ID NO. 285-299 and/or SEQ ID NO. 345-365 and/or SEQ ID NO. 257-263;
(4) the step of detecting a product comprises amplification with at least one antisense primer comprising, consisting essentially of or consisting of SEQ ID NO. 65-128 and/or SEQ ID NO. 300-314 and/or SEQ ID NO. 366-386 and/or SEQ ID NO. 264-270;
(5) the step of detecting employs amplification of at least a portion of the at least one gene, and further employs at least one oligonucleotide probe which hybridizes to an amplicon selected from the group consisting of SEQ ID NO: 129-292 and/or SEQ ID NO. 193-256 and/or SEQ ID NO. 315-329 and/or SEQ ID NO. 330-344 and/or SEQ ID NO. 408-428 and/or SEQ ID NO. 429-449 and/or SEQ ID NO. 271-277 and/or SEQ ID NO. 278-284.under amplification conditions, wherein preferably the probe comprises, consists essentially of or consists of sequences represented by SEQ ID NO. 387-407; or
(6) the step of detecting employs amplification of at least a portion of the at least one gene and a detectable reagent which preferentially binds to double stranded DNA relative to single stranded DNA.

10. A kit for assessing lung cancer or its precursor, or predisposition to lung cancer in a test sample containing lung cells or nucleic acids from lung cells, said kit comprising in a package:
a reagent that (a) modifies methylated cytosine residues but not non-methylated cytosine residues, or that (b) modifies non-methylated cytosine residues but not methylated cytosine residues; and
at least one pair of oligonucleotide primers that specifically hybridizes under amplification conditions to a region of a gene selected from the group consisting of JAM3, SULF2, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site.

11. The kit of claim 10 wherein:
(1) the at least one pair ofprimers is selected from Table 1 (SEQ ID NO: 1-128), Figure 2 (SEQ ID NO: 257-270), Table 3 (SEQ ID NO: 285-314) and Table 7 (SEQ ID NO: 345-386); or
(2) the at least one pair of oligonucleotide primers amplifies an amplicon selected from Table 2 (SEQ ID NO: 129-256), Figure 2 (SEQ ID NO: 271-284),Table 4 (SEQ ID NO: 315-344) and Table 8 (SEQ ID NO:408-449).

12. A kit for assessing lung cancer or its precursor, or predisposition to lung cancer in a test sample containing lung cells or nucleic acids from lung cells, said kit comprising in a package:
at least two pairs of oligonucleotide primers that specifically hybridize under amplification conditions to a region of a gene selected from the group consisting of JAM3, SULF2, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site.

13. The kit of claim 12 wherein:
(1) the at least two pairs of primers are selected from SEQ ID NO: 1-128 (Table 1), SEQ ID NO: 257-270 (Figure 2), SEQ ID NO:285-314 (Table 3), SEQ ID NO: 345-386 (Table 7); or
(2) the at least two pairs of oligonucleotide primers amplify amplicons selected from Table 2 (SEQ ID NO: 129-256), Figure 2 (SEQ ID NO: 271-284), Table 4 (SEQ ID NO: 315-344) and Table 8 (SEQ ID NO: 408-449).

14. The kit of claim 10 or 12 further comprising at least one oligonucleotide probe which hybridizes to an amplicon selected from the group consisting of Table 2 (SEQ ID NO: 129-256), Figure 2 (SEQ ID NO: 271-284), Table 4 (SEQ ID NO: 315-344) and Table 8 (SEQ ID NO: 408-449) under amplification conditions.

15. The kit of claim 14 wherein the oligonucleotide probe is selected from the group consisting of SEQ ID NO: 387-407, wherein preferably the oligonucleotide probe comprises a fluorescent label, a fluorescence quenching agent, or a fluorescent label and fluorescence quenching agent.

16. The kit of claim 10 or 12 which comprises:
(1) a detectable reagent which preferentially binds to double stranded DNA relative to single stranded DNA;
(2) a DNA polymerase for amplifying DNA; and/or
(3) at least one oligonucleotide probe which is covalently linked to at least one of said oligonucleotide primers.

17. An isolated polynucleotide comprising a nucleotide sequence selected from the group consisting of SEQ ID NO: 1-16, 19-80, 83-144, 147-208, 211-348, 350-411, 413-432, 434-449, wherein optionally the polynucleotide is detectably labeled, preferably with a fluorescent label.

18. The isolated polynucleotide of claim 17 which consists of the selected nucleotide sequence.


SEQUENCE LISTING

<110> BAYLIN, Stephen
      HERMAN, James
      SCHUEBEL, Kornel
      COPE, Leslie
      VAN CRIEKINGE, Wim
      STRAUB, Josef
      TROOSKENS, Geert
      VAN NESTE, Leander

<120> DETECTION AND PROGNOSIS OF LUNG CANCER


<130> 006791.00038

<150> 61/029693
<151> 2008-02-19

<160> 449

<170> FastSEQ for Windows Version 4.0

<210> 1
<211> 22
<212> DNA
<213> Homo sapiens

<400> 1
tttaatgtta cgttttggcg tt                                                22

<210> 2
<211> 21
<212> DNA
<213> Homo sapiens

<400> 2
gcggttgtaa ggtttttggt c                                                 21

<210> 3
<211> 21
<212> DNA
<213> Homo sapiens

<400> 3
ggacgggtgt ttgcgtttta c                                                 21

<210> 4
<211> 22
<212> DNA
<213> Homo sapiens

<400> 4
gtcgtttgtt taggttcgga tc                                                22

<210> 5
<211> 20
<212> DNA
<213> Homo sapiens

<400> 5
tcggggtttt tatttggttc                                                   20

<210> 6
<211> 20
<212> DNA
<213> Homo sapiens

<400> 6
gtacgtgcgt ttattgcgag                                                    20

<210> 7
<211> 20
<212> DNA
<213> Homo sapiens

<400> 7
ggtatcggtt tggttatcgc                                                    20

<210> 8
<211> 25
<212> DNA
<213> Homo sapiens

<400> 8
gttttcgatt gatttattaa ggttc                                              25

<210> 9
<211> 19
<212> DNA
<213> Homo sapiens

<400> 9
tcgtgggaag agagcgtag                                                     19

<210> 10
<211> 25
<212> DNA
<213> Homo sapiens

<400> 10
ttttgttaag agttgtcgtt agttc                                              25

<210> 11
<211> 20
<212> DNA
<213> Homo sapiens

<400> 11
ggggtagtcg ttaattgcgt                                                    20

<210> 12
<211> 22
<212> DNA
<213> Homo sapiens

<400> 12
cgggagaaga aagttagtgc gt                                                 22

<210> 13
<211> 17
<212> DNA
<213> Homo sapiens

<400> 13

gagcgttcgg gttttgc                                                    17

<210> 14
<211> 25
<212> DNA
<213> Homo sapiens

<400> 14
ttgggaattt agttgtcgtc gtttc                                           25

<210> 15
<211> 20
<212> DNA
<213> Homo sapiens

<400> 15
gaggtttgcg gtttaggttc                                                 20

<210> 16
<211> 20
<212> DNA
<213> Homo sapiens

<400> 16
ggagttgggg tttacgagac                                                 20

<210> 17
<211> 25
<212> DNA
<213> Homo sapiens

<400> 17
ggtgttttga tagaagtcgt tagtc                                           25

<210> 18
<211> 20
<212> DNA
<213> Homo sapiens

<400> 18
ggggttatag tttggcgttc                                                 20

<210> 19
<211> 21
<212> DNA
<213> Homo sapiens

<400> 19
ggtttcggtt tcgttttgtt c                                               21

<210> 20
<211> 19
<212> DNA
<213> Homo sapiens

<400> 20
gggatagtgg ggttgacgc                                                  19

<210> 21
<211> 19
<212> DNA
<213> Homo sapiens

<400> 21
gcgtgggttt tcgtcgtag                                                    19

<210> 22
<211> 24
<212> DNA
<213> Homo sapiens

<400> 22
gggtgttcga tttaagtcga gttc                                              24

<210> 23
<211> 22
<212> DNA
<213> Homo sapiens

<400> 23
gtttagttaa gttcggttcg gg                                                22

<210> 24
<211> 24
<212> DNA
<213> Homo sapiens

<400> 24
agggagttta gttaagttcg gttc                                              24

<210> 25
<211> 19
<212> DNA
<213> Homo sapiens

<400> 25
tagagcggag gaagttgcg                                                    19

<210> 26
<211> 23
<212> DNA
<213> Homo sapiens

<400> 26
tcggagtttt atagggtaac gaa                                               23

<210> 27
<211> 21
<212> DNA
<213> Homo sapiens

<400> 27
ttggagattt cgatagagcg t                                                 21

<210> 28
<211> 26
<212> DNA
<213> Homo sapiens

<400> 28
gcgataggtt tttagtaagt aagcgc                                            26

<210> 29
<211> 20
<212> DNA
<213> Homo sapiens

<400> 29
ttcggggtgt agcggtcgtc                                                    20

<210> 30
<211> 20
<212> DNA
<213> Homo sapiens

<400> 30
acgtaagagt ttgggagcgt                                                    20

<210> 31
<211> 19
<212> DNA
<213> Homo sapiens

<400> 31
gtttcggggt tcgtttttc                                                     19

<210> 32
<211> 21
<212> DNA
<213> Homo sapiens

<400> 32
tttgtcggcg tcgttatttt c                                                  21

<210> 33
<211> 21
<212> DNA
<213> Homo sapiens

<400> 33
cgtttatggg tcggttacgt c                                                  21

<210> 34
<211> 19
<212> DNA
<213> Homo sapiens

<400> 34
agttgagaat cggacgggg                                                     19

<210> 35
<211> 20
<212> DNA
<213> Homo sapiens

<400> 35
gggattataa gtcgcgtcgc                                                    20

<210> 36
<211> 22
<212> DNA
<213> Homo sapiens

<400> 36
gcgcgtagag ttgtaaaggt tc                                                 22

<210> 37
<211> 21
<212> DNA

<213> Homo sapiens

<400> 37
ggtagaggcg aggagttgtt c                                                21

<210> 38
<211> 21
<212> DNA
<213> Homo sapiens

<400> 38
gatgtcgttt gggagtagtg c                                                21

<210> 39
<211> 27
<212> DNA
<213> Homo sapiens

<400> 39
gcggggtttt ttttatcggt tagattc                                          27

<210> 40
<211> 23
<212> DNA
<213> Homo sapiens

<400> 40
gttttcgttg tcgttacggg ttc                                              23

<210> 41
<211> 23
<212> DNA
<213> Homo sapiens

<400> 41
agaatttagg tcggttttta tcg                                              23

<210> 42
<211> 19
<212> DNA
<213> Homo sapiens

<400> 42
gtcggatgaa gtattcggg                                                   19

<210> 43
<211> 24
<212> DNA
<213> Homo sapiens

<400> 43
ttatttcgtt tttagggagt tttc                                             24

<210> 44
<211> 21
<212> DNA
<213> Homo sapiens

<400> 44
tttcgtgtgg gaagagttcg t                                                21

<210> 45
<211> 25

<212> DNA
<213> Homo sapiens

<400> 45
ttttggttat taggtagttc ggttc                                          25

<210> 46
<211> 19
<212> DNA
<213> Homo sapiens

<400> 46
ttattttgcg agcggtttc                                                 19

<210> 47
<211> 25
<212> DNA
<213> Homo sapiens

<400> 47
gatttgggcg tttttggttt ttcgc                                          25

<210> 48
<211> 20
<212> DNA
<213> Homo sapiens

<400> 48
gggcgttgag gtagaagaac                                                20

<210> 49
<211> 20
<212> DNA
<213> Homo sapiens

<400> 49
ttaggtcgga ggtttcgttt                                                20

<210> 50
<211> 20
<212> DNA
<213> Homo sapiens

<400> 50
gatgtttcgg tcgaggtttc                                                20

<210> 51
<211> 26
<212> DNA
<213> Homo sapiens

<400> 51
tgtagttttc ggagttagtg tcgcgc                                         26

<210> 52
<211> 21
<212> DNA
<213> Homo sapiens

<400> 52
ttttgttcgt cgtattttcg g                                              21

<210> 53

<211> 22
<212> DNA
<213> Homo sapiens

<400> 53
agtatagagt ggggagcgta gc                                                22

<210> 54
<211> 21
<212> DNA
<213> Homo sapiens

<400> 54
ttgcgttagt cgtttgcgtt c                                                 21

<210> 55
<211> 22
<212> DNA
<213> Homo sapiens

<400> 55
gttagtcgag ttcggaggta tc                                                22

<210> 56
<211> 21
<212> DNA
<213> Homo sapiens

<400> 56
gcgtcggagg ttaaggttgt t                                                 21

<210> 57
<211> 21
<212> DNA
<213> Homo sapiens

<400> 57
cgggttagag tattgttcgg t                                                 21

<210> 58
<211> 21
<212> DNA
<213> Homo sapiens

<400> 58
gattttatcg gggaaatatc g                                                 21

<210> 59
<211> 25
<212> DNA
<213> Homo sapiens

<400> 59
ttatttcgta ggttgaggtt agggc                                             25

<210> 60
<211> 22
<212> DNA
<213> Homo sapiens

<400> 60
gttgtatttt cgcggagcgt tc                                                22

<210> 61
<211> 24
<212> DNA
<213> Homo sapiens

<400> 61
gtttaggttg tggtttaggt cgtc                                              24

<210> 62
<211> 26
<212> DNA
<213> Homo sapiens

<400> 62
ggggttttta ggtattcggt tcgtac                                            26

<210> 63
<211> 24
<212> DNA
<213> Homo sapiens

<400> 63
tcggttttta gtttttcgg tcgc                                               24

<210> 64
<211> 21
<212> DNA
<213> Homo sapiens

<400> 64
ttatcgagaa gcgtcggttt c                                                 21

<210> 65
<211> 19
<212> DNA
<213> Homo sapiens

<400> 65
gaaccaaccc tctccgacc                                                    19

<210> 66
<211> 19
<212> DNA
<213> Homo sapiens

<400> 66
atttttccgc aacctctcg                                                    19

<210> 67
<211> 27
<212> DNA
<213> Homo sapiens

<400> 67
cgaaaccaaa aaactaaacg aaaaccg                                           27

<210> 68
<211> 22
<212> DNA
<213> Homo sapiens

<400> 68
gacccgaaat aacctcgaaa cg                                                22

<210> 69
<211> 21
<212> DNA
<213> Homo sapiens

<400> 69
aatcgtcact cgtatctcgc t                                                21

<210> 70
<211> 23
<212> DNA
<213> Homo sapiens

<400> 70
cgttatccaa actaaaatcg acc                                              23

<210> 71
<211> 22
<212> DNA
<213> Homo sapiens

<400> 71
cgcctacaac tactacacga cc                                               22

<210> 72
<211> 22
<212> DNA
<213> Homo sapiens

<400> 72
tcaaaatccg aactctaaac cg                                               22

<210> 73
<211> 22
<212> DNA
<213> Homo sapiens

<400> 73
ttactaacct aaacgaccgc aa                                               22

<210> 74
<211> 21
<212> DNA
<213> Homo sapiens

<400> 74
aatataaacc ctacgaccgc c                                                21

<210> 75
<211> 19
<212> DNA
<213> Homo sapiens

<400> 75
tcttccccga aaaccgcta                                                   19

<210> 76
<211> 24
<212> DNA
<213> Homo sapiens

<400> 76

aaatcgaaaa acctaaaata tcgc 24

<210> 77
<211> 22
<212> DNA
<213> Homo sapiens

<400> 77
cgacctcgac gaaaaaataa cg 22

<210> 78
<211> 25
<212> DNA
<213> Homo sapiens

<400> 78
aaacaacgac taccgatact acgcg 25

<210> 79
<211> 22
<212> DNA
<213> Homo sapiens

<400> 79
ctcacactat acaacacgcg ac 22

<210> 80
<211> 21
<212> DNA
<213> Homo sapiens

<400> 80
ataataaatt ccccgacgac c 21

<210> 81
<211> 19
<212> DNA
<213> Homo sapiens

<400> 81
aaaaccatta acgcccacg 19

<210> 82
<211> 21
<212> DNA
<213> Homo sapiens

<400> 82
gctctaaaaa ccacacccgt c 21

<210> 83
<211> 21
<212> DNA
<213> Homo sapiens

<400> 83
ctctacgact caaacctcgc t 21

<210> 84
<211> 20
<212> DNA
<213> Homo sapiens

<400> 84
ataaaaatcc cgacgaacga                                                    20

<210> 85
<211> 19
<212> DNA
<213> Homo sapiens

<400> 85
cccaaaacta ctcgccgct                                                     19

<210> 86
<211> 24
<212> DNA
<213> Homo sapiens

<400> 86
cgcgaatctt aaccgaaaaa atcg                                               24

<210> 87
<211> 23
<212> DNA
<213> Homo sapiens

<400> 87
gattacaatt tacaacctcc gct                                                23

<210> 88
<211> 19
<212> DNA
<213> Homo sapiens

<400> 88
tacaacctcc gctaccgtc                                                     19

<210> 89
<211> 22
<212> DNA
<213> Homo sapiens

<400> 89
caaatacgaa cacaaaaacc ga                                                 22

<210> 90
<211> 19
<212> DNA
<213> Homo sapiens

<400> 90
ctcttactaa ccgcacgcc                                                     19

<210> 91
<211> 21
<212> DNA
<213> Homo sapiens

<400> 91
aaactaccga ctacacctcc g                                                  21

<210> 92
<211> 24
<212> DNA
<213> Homo sapiens

<400> 92
ctctccgctc caaacgctaa cgcg                                              24

<210> 93
<211> 22
<212> DNA
<213> Homo sapiens

<400> 93
gccccaatac taaatcacga cg                                                22

<210> 94
<211> 21
<212> DNA
<213> Homo sapiens

<400> 94
gactcctcga aaaacaaacg a                                                 21

<210> 95
<211> 20
<212> DNA
<213> Homo sapiens

<400> 95
cgactcgctc tatctcgcac                                                   20

<210> 96
<211> 24
<212> DNA
<213> Homo sapiens

<400> 96
aaactaccta ctaaacgaaa cccg                                              24

<210> 97
<211> 21
<212> DNA
<213> Homo sapiens

<400> 97
ataaaaacac gaaaaccccg c                                                 21

<210> 98
<211> 20
<212> DNA
<213> Homo sapiens

<400> 98
aacgaatcaa actcccgaaa                                                   20

<210> 99
<211> 20
<212> DNA
<213> Homo sapiens

<400> 99
cgaacgcaaa accgaaatcg                                                   20

<210> 100
<211> 19
<212> DNA

<213> Homo sapiens

<400> 100
acgtcctcct cgaacgaaa                                                    19

<210> 101
<211> 22
<212> DNA
<213> Homo sapiens

<400> 101
tacacaaacc gttctaaccc ga                                                22

<210> 102
<211> 23
<212> DNA
<213> Homo sapiens

<400> 102
acaaaatacc gctaactaac gaa                                               23

<210> 103
<211> 27
<212> DNA
<213> Homo sapiens

<400> 103
caacgatacc caaaaaaat caacgcg                                            27

<210> 104
<211> 17
<212> DNA
<213> Homo sapiens

<400> 104
gcgcaacgaa caaaacg                                                      17

<210> 105
<211> 22
<212> DNA
<213> Homo sapiens

<400> 105
acgcaaaatt cttctcccaa aa                                                22

<210> 106
<211> 20
<212> DNA
<213> Homo sapiens

<400> 106
ccctacaaac gacgacgaac                                                   20

<210> 107
<211> 21
<212> DNA
<213> Homo sapiens

<400> 107
cttacgaacc atttaatccc g                                                 21

<210> 108
<211> 21

<212> DNA
<213> Homo sapiens

<400> 108
cgaataaccg aacgaccgat a                                                21

<210> 109
<211> 22
<212> DNA
<213> Homo sapiens

<400> 109
cactcttcgt actattcccg ct                                               22

<210> 110
<211> 23
<212> DNA
<213> Homo sapiens

<400> 110
gaatactcta attccacgcg act                                              23

<210> 111
<211> 20
<212> DNA
<213> Homo sapiens

<400> 111
gacttctcat accgcaatcg                                                  20

<210> 112
<211> 22
<212> DNA
<213> Homo sapiens

<400> 112
cgacacctac caaataaaat cg                                               22

<210> 113
<211> 20
<212> DNA
<213> Homo sapiens

<400> 113
aaaccctcac cgttatcgtc                                                  20

<210> 114
<211> 24
<212> DNA
<213> Homo sapiens

<400> 114
aaacgactac aaataaatac gcca                                             24

<210> 115
<211> 25
<212> DNA
<213> Homo sapiens

<400> 115
cctacgatcg aaaacgacgc gaacg                                            25

<210> 116

<211> 22
<212> DNA
<213> Homo sapiens

<400> 116
ataacgaccc tcgacctacg at                                                22

<210> 117
<211> 20
<212> DNA
<213> Homo sapiens

<400> 117
ctttcctacc accgaaacga                                                   20

<210> 118
<211> 24
<212> DNA
<213> Homo sapiens

<400> 118
caaaaacgaa tcccgtatcc gacg                                              24

<210> 119
<211> 24
<212> DNA
<213> Homo sapiens

<400> 119
caactccgaa cgaaacaata aacg                                              24

<210> 120
<211> 22
<212> DNA
<213> Homo sapiens

<400> 120
ctctccaaaa ttaccgtacg cg                                                22

<210> 121
<211> 19
<212> DNA
<213> Homo sapiens

<400> 121
gaacacaaat cccgcgtaa                                                    19

<210> 122
<211> 19
<212> DNA
<213> Homo sapiens

<400> 122
aaacaaatcc cgctccgaa                                                    19

<210> 123
<211> 21
<212> DNA
<213> Homo sapiens

<400> 123
tcctctacta tcaacgccga c                                                 21

<210> 124
<211> 22
<212> DNA
<213> Homo sapiens

<400> 124
ctcacaatac gtctaaccga cg                                                22

<210> 125
<211> 25
<212> DNA
<213> Homo sapiens

<400> 125
acacctcgta tcctcactaa aaacg                                             25

<210> 126
<211> 22
<212> DNA
<213> Homo sapiens

<400> 126
aatacgcaat acccgacgac cg                                                22

<210> 127
<211> 29
<212> DNA
<213> Homo sapiens

<400> 127
caattactac gcaaaaacga aacaaaacg                                         29

<210> 128
<211> 26
<212> DNA
<213> Homo sapiens

<400> 128
accgaaaaaa aaaacgaacc taaccg                                            26

<210> 129
<211> 106
<212> DNA
<213> Homo sapiens

<400> 129
tttaatgtta cgttttggcg ttcgtcgttc gtgttttttt ttttagtcgg ttttcgtaga 60
atgttaggta ttgacgttgg agagcggggt cggagagggt tggttc                     106

<210> 130
<211> 101
<212> DNA
<213> Homo sapiens

<400> 130
gcggttgtaa ggtttttggt cggtgagtga attagtaggt aaggatggta gttagggtat 60
ttatatttac gagggtggtg gtcgagaggt tgcggaaaaa t                          101

<210> 131
<211> 91
<212> DNA
<213> Homo sapiens

<400> 131
ggacgggtgt ttgcgtttta cgtttagttc gtttaggtgg gggttttcgt tttttcggtt 60
gttgcggttt tcgtttagtt ttttggtttc g 91

<210> 132
<211> 99
<212> DNA
<213> Homo sapiens

<400> 132
gtcgtttgtt taggttcgga tcgggttttg ttcgtttcgg agtttttgtt cgcgtcgcgg 60
agatttcgga gttcgcgcgt ttcgaggtta tttcgggtc 99

<210> 133
<211> 96
<212> DNA
<213> Homo sapiens

<400> 133
tcggggtttt tatttggttc gttttttttc gggtcggatg ttagttcgtc gagcgtaggg 60
tagcggggag ttggtagcga gatacgagtg acgatt 96

<210> 134
<211> 104
<212> DNA
<213> Homo sapiens

<400> 134
gtacgtgcgt ttattgcgag ttgcggcgtc gtatagtttc gtggcgtttt gggtattttt 60
gtttttgttg cgtttcgttt tggtcgattt tagtttggat aacg 104

<210> 135
<211> 111
<212> DNA
<213> Homo sapiens

<400> 135
ggtatcggtt tggttatcgc gcgcgaattg tgtcgatagt tttttgggga tgtggtgttt 60
atcgcgcggg acgtggcgcg gggttaggcg gtcgtgtagt agttgtaggc g 111

<210> 136
<211> 80
<212> DNA
<213> Homo sapiens

<400> 136
gttttcgatt gatttattaa ggttcgattt ggtttcggat atttcgtaga ttatttcgcg 60
gtttagagtt cggattttga 80

<210> 137
<211> 105
<212> DNA
<213> Homo sapiens

<400> 137
tcgtgggaag agagcgtagt agttgttggg gtcgtaggcg gtacggggtt tagtagttta 60
ggggttttgg tttagtgtgg gttttgcggt cgtttaggtt agtaa 105

<210> 138
<211> 100
<212> DNA
<213> Homo sapiens

<400> 138
ttttgttaag agttgtcgtt agttcggggt cggattagtt cgggggtatc gcgatgttgt 60
tgcgtttgtt gttggtttgg gcggtcgtag ggtttatatt 100

<210> 139
<211> 95
<212> DNA
<213> Homo sapiens

<400> 139
ggggtagtcg ttaattgcgt tttttttttt ttttcgtttt taattttaga gttttttatt 60
ttattgtttt ttgttttagc ggttttcggg gaaga 95

<210> 140
<211> 90
<212> DNA
<213> Homo sapiens

<400> 140
cgggagaaga aagttagtgc gtttttgggc gtaggggtta gtggggttcg gaggtatagg 60
tatttcgcga tattttaggt ttttcgattt 90

<210> 141
<211> 120
<212> DNA
<213> Homo sapiens

<400> 141
gagcgttcgg gttttgcggg gagtaggtta aggcggtcga gagaaagggg ggtcgagacg 60
ggggggtgga ggtttggggg ggtggggggg taggcggtcg ttattttttc gtcgaggtcg 120

<210> 142
<211> 100
<212> DNA
<213> Homo sapiens

<400> 142
ttgggaattt agttgtcgtc gtttcgtaga gttttttgtt ttcggagggc gtttattttc 60
gggtcgttta ttattcgcgt agtatcggta gtcgttgttt 100

<210> 143
<211> 85
<212> DNA
<213> Homo sapiens

<400> 143
gaggtttgcg gtttaggttc gatttttgcg atttgtttta ggtaggtttg tatgtgcgcg 60
gcggtcgcgt gttgtatagt gtgag 85

<210> 144
<211> 106
<212> DNA
<213> Homo sapiens

<400> 144
ggagttgggg tttacgagac ggggcgtgcg gggtatcggg cggtcggcgg ggagtcgtag 60
gtttttttag agggggcgcg agtcgggtcg tcggggaatt tattat 106

<210> 145
<211> 114
<212> DNA
<213> Homo sapiens

<400> 145
ggtgttttga tagaagtcgt tagtcggtgt tatgtttagg ataggtattt gtagttttgt 60
gtggacgtta acgttattag gaaggattat taggtcgtgg gcgttaatgg tttt 114

<210> 146
<211> 97
<212> DNA
<213> Homo sapiens

<400> 146
ggggttatag tttggcgttc ggattttggt tcgggttatt tgcgaaggag tcggttttgg 60
ttaaggtgtt tttttggacg ggtgtggttt ttagagc 97

<210> 147
<211> 89
<212> DNA
<213> Homo sapiens

<400> 147
ggtttcggtt tcgttttgtt cgttgttttc ggcgacggtc gtggtttttg ttttggggtt 60
aattatagag cgaggtttga gtcgtagag 89

<210> 148
<211> 113
<212> DNA
<213> Homo sapiens

<400> 148
gggatagtgg ggttgacgcg tggtttcggc gtcgcgcggt ttttcgaatt tcgagtttcg 60
cgttcggcgc ggtcggggtt tttaatcgtt ttttcgttcg tcgggatttt tat 113

<210> 149
<211> 83
<212> DNA
<213> Homo sapiens

<400> 149
gcgtgggttt tcgtcgtagt ttcgcggagt ttcggtgttt tttgtaatag ggggcggggg 60
gaatagcggc gagtagtttt ggg 83

<210> 150
<211> 120
<212> DNA
<213> Homo sapiens

<400> 150
gggtgttcga tttaagtcga gttcgagttc gagtttaggt aggagtttta tagatagttt 60
tttttttttt tattttttgt aggcgtttta cgcgtgcgat tttttcggtt aagattcgcg 120

<210> 151
<211> 118
<212> DNA
<213> Homo sapiens

<400> 151
gtttagttaa gttcggttcg ggggttttta ggttaggata tcgaggtaag agttatttga 60
atcgttggcg aattggtggt tgttgcggcg acggtagcgg aggttgtaaa ttgtaatc 118

<210> 152
<211> 113
<212> DNA

<213> Homo sapiens

<400> 152
agggagttta gttaagttcg gttcgggggt ttttaggtta ggatatcgag gtaagagtta 60
tttgaatcgt tggcgaattg gtggttgttg cggcgacggt agcggaggtt gta 113

<210> 153
<211> 90
<212> DNA
<213> Homo sapiens

<400> 153
tagagcggag gaagttgcgg atttggggtg ggggaattcg ttcgcggatt tttggttttt 60
atttcgcgtc ggtttttgtg ttcgtatttg 90

<210> 154
<211> 87
<212> DNA
<213> Homo sapiens

<400> 154
tcggagtttt atagggtaac gaagcgcggg tagcggttgc ggagtcgggc ggaggtgcgc 60
ggggtcgggg cgtgcggtta gtaagag 87

<210> 155
<211> 111
<212> DNA
<213> Homo sapiens

<400> 155
ttggagattt cgatagagcg tcggtttttt gattgttcgc gaagcgagac gcggggcgtc 60
gggtttagcg tagtgagcgg cgaggcgcgg cggaggtgta gtcggtagtt t 111

<210> 156
<211> 120
<212> DNA
<213> Homo sapiens

<400> 156
gcgataggtt tttagtaagt aagcgcgggc ggtattcgta gtttttagaa gtttgagatt 60
tggtcgtaag cggattcgtg cgttttaatt ttttgtcgcg ttagcgtttg gagcggagag 120


<210> 157
<211> 91
<212> DNA
<213> Homo sapiens

<400> 157
ttcggggtgt agcggtcgtc ggggttgggg tcggcgggag ttcgcgggat tttttagaag 60
agcggtcggc gtcgtgattt agtattgggg c 91

<210> 158
<211> 101
<212> DNA
<213> Homo sapiens

<400> 158
acgtaagagt ttgggagcgt tcgagtcgtt cggttgttcg gagttttatc gtttaggatc 60
gggagatgtt ggaaatgtaa tcgtttgttt ttcgaggagt c 101

<210> 159
<211> 96

<212> DNA
<213> Homo sapiens

<400> 159
gtttcggggt tcgtttttcg gtaggttcgg ggagaggtgg ggtgataatg ggttggggtg 60
cgcgcgtgtt ttataggtgc gagatagagc gagtcg 96

<210> 160
<211> 97
<212> DNA
<213> Homo sapiens

<400> 160
tttgtcggcg tcgttatttt cgtacggttc gttttcgtcg cgggcgtata tagggtagta 60
gtcgtacgcg tcgcgggttt cgtttagtag gtagttt 97

<210> 161
<211> 114
<212> DNA
<213> Homo sapiens

<400> 161
cgtttatggg tcggttacgt cgggtgttcg tttatttttc gacgttagta ggagcgcgcg 60
cgtaggtttc gcggggtcgg gagggcggta cgggcggggt tttcgtgttt ttat 114

<210> 162
<211> 83
<212> DNA
<213> Homo sapiens

<400> 162
   agttgagaat cggacggggt gggatcgagg agggtgcgaa gcgttattgt ttaggtttcg 60
   ttttttcggg agtttgattc gtt 83

<210> 163
<211> 91
<212> DNA
<213> Homo sapiens

<400> 163
gggattataa gtcgcgtcgc gttgtcgttg gttttttagt aattttcgat atggcgttga 60
ggcggttatc gcgatttcgg ttttgcgttc g 91

<210> 164
<211> 91
<212> DNA
<213> Homo sapiens

<400> 164
gcgcgtagag ttgtaaaggt tcgagtagga gtacggttta ggcgaagcgt attatttttt 60
ttgttagatt gatttcgttc gaggaggacg t 91

<210> 165
<211> 92
<212> DNA
<213> Homo sapiens

<400> 165
ggtagaggcg aggagttgtt cgttttgtac gtttttaatc gtattacgtg aataaatagt 60
tgaggggcgg tcgggttaga acggtttgtg ta 92

<210> 166
<211> 97

<212> DNA
<213> Homo sapiens

<400> 166
gatgtcgttt gggagtagtg cgggtttttg tattgttaag gttttatagg tacgggttgg 60
gcgggggtgg gtagttcgtt agttagcggt attttgt 97

<210> 167
<211> 109
<212> DNA
<213> Homo sapiens

<400> 167
gcggggtttt ttttatcggt tagattcggg gagaggcgcg cggaggttgc gaaggtttta 60
gaagggcggg gagggggcgt cgcgcgttga ttttttttgg gtatcgttg 109

<210> 168
<211> 97
<212> DNA
<213> Homo sapiens

<400> 168
gttttcgttg tcgttacggg ttcgtttttt ttttttttcg gtttttaggg taaggcgcgg 60
ggcgcggggt tggatgtagg cgttttgttc gttgcgc 97

<210> 169
<211> 113
<212> DNA
<213> Homo sapiens

<400> 169
agaatttagg tcggttttta tcgtttttta gaacgattgt attattgtcg ttgtcgtcgg 60
tttgatattg ttttagtttt agtgttggta gttttgggag aagaattttg cgt 113

<210> 170
<211> 82
<212> DNA
<213> Homo sapiens

<400> 170
gtcggatgaa gtattcgggc gtttttattg cggaagggcg gggatggttg tgacgtaggc 60
gtgttcgtcg tcgtttgtag gg 82

<210> 171
<211> 96
<212> DNA
<213> Homo sapiens

<400> 171
ttatttcgtt tttagggagt tttcgggtta tttttttatt cgggttgttt cgcggttttt 60
aaggagtttt attttcggga ttaaatggtt cgtaag 96

<210> 172
<211> 109
<212> DNA
<213> Homo sapiens

<400> 172
tttcgtgtgg gaagagttcg tttgggtgta gcgtcgcggt tcgtaatatt agtaacggta 60
gtagtagtag tattggtaac gacgatagta tcggtcgttc ggttattcg 109

<210> 173
<211> 97

<212> DNA
<213> Homo sapiens

<400> 173
ttttggttat taggtagttc ggttcggcgg ttcgttcggg gtattagttc ggtgtagggc 60
gcggagtcgt tttgtagcgg gaatagtacg aagagtg 97

<210> 174
<211> 114
<212> DNA
<213> Homo sapiens

<400> 174
ttattttgcg agcggtttcg cgatacgagg tagtcgtttt cgtttttcga cgcggttatg 60
ggttcggtcg gcgcgggggt aagttagagc gagtcgcgtg gaattagagt attc 114

<210> 175
<211> 75
<212> DNA
<213> Homo sapiens

<400> 175
gatttgggcg tttttggttt ttcgcggttt cgagttttcg ataaattttt tgcgtcgatt 60
gcggtatgag aagtc 75

<210> 176
<211> 113
<212> DNA
<213> Homo sapiens

<400> 176
gggcgttgag gtagaagaac gtgtacgagg tgaaggatta taaatttatc gcgcgttttt 60
ttaagtagtt tattttttgt agttattgta tcgattttat ttggtaggtg tcg 113

<210> 177
<211> 102
<212> DNA
<213> Homo sapiens

<400> 177
ttaggtcgga ggtttcgttt tttttttttt ggtttttttt ttttttcgtg ggtcggtcgt 60
taacgacgtt agagtcggaa atgacgataa cggtgagggt tt 102

<210> 178
<211> 103
<212> DNA
<213> Homo sapiens

<400> 178
gatgtttcgg tcgaggtttc gtcgtagttt ttttttagtt tttaggtcgc ggcgttttta 60
ttcgggattt tttcggattt ggcgtattta tttgtagtcg ttt 103

<210> 179
<211> 126
<212> DNA
<213> Homo sapiens

<400> 179
tgtagttttc ggagttagtg tcgcgcgttc gtcgtttcgc gtttttttgt tcgtcgtatt 60
ttcgggagtc ggggcgtatt tagttcgtag cgtcgttttt tcgttcgcgt cgttttcgat 120
cgtagg 126

<210> 180

<211> 96
<212> DNA
<213> Homo sapiens

<400> 180
ttttgttcgt cgtattttcg ggagtcgggg cgtatttagt tcgtagcgtc gttttttcgt 60
tcgcgtcgtt ttcgatcgta ggtcgagggt cgttat 96

<210> 181
<211> 80
<212> DNA
<213> Homo sapiens

<400> 181
agtatagagt ggggagcgta gcgacgaaga atgaataggg tttcgtgagg ttttaaatat 60
tcgtttcggt ggtaggaaag 80

<210> 182
<211> 79
<212> DNA
<213> Homo sapiens

<400> 182
ttgcgttagt cgtttgcgtt cgtttttagt ttatattatg aaagcgttta tcggtcgtcg 60
gatacgggat tcgtttttg 79

<210> 183
<211> 79
<212> DNA
<213> Homo sapiens

<400> 183
gttagtcgag ttcggaggta tcgggaggtc gagagtcgtc gggattttag ttttgcgttt 60
attgtttcgt tcggagttg 79

<210> 184
<211> 95
<212> DNA
<213> Homo sapiens

<400> 184
gcgtcggagg ttaaggttgt ttcgtacggt tcggcgggcg agcgagttcg ggttgtagta 60
gtttcgtcgg cggcgcgtac ggtaattttg gagag 95

<210> 185
<211> 99
<212> DNA
<213> Homo sapiens

<400> 185
cgggttagag tattgttcgg tggtgtttag gaggagtagg agtaggagta gaagtagaag 60
cggggttcgg agttgcgcgt ttacgcggga tttgtgttc 99

<210> 186
<211> 114
<212> DNA
<213> Homo sapiens

<400> 186
gattttatcg gggaaatatc gcggatagtc gggttagtag cgttcggagt ttattttagg 60
tttttaaatt tgtagtattt tttagagcgc gcgcgttcgg agcgggattt gttt 114

<210> 187

<211> 117
<212> DNA
<213> Homo sapiens

<400> 187
ttatttcgta ggttgaggtt agggcgtggc ggttgttggg atttcggagt tttttagtag 60
taggggttgc gggaggaagt gaagtcggga ggggttgtcg gcgttgatag tagagga 117

<210> 188
<211> 108
<212> DNA
<213> Homo sapiens

<400> 188
gttgtatttt cgcggagcgt tcggtagaaa tagtttaggg aagacgaaaa atagttagcg 60
gagtcgttta ggttgtagtt ataaagcgtc ggttagacgt attgtgag 108

<210> 189
<211> 80
<212> DNA
<213> Homo sapiens

<400> 189
gtttaggttg tggtttaggt cgtcggtttt cggttatgtt tagttttttt gaggtcgttt 60
ttagtgagga tacgaggtgt 80

<210> 190
<211> 97
<212> DNA
<213> Homo sapiens

<400> 190
ggggttttta ggtattcggt tcgtacgtaa atttttagtt cggggttttt tgattttcgc 60
gtttattttt tagttcggtc gtcgggtatt gcgtatt 97

<210> 191
<211> 95
<212> DNA
<213> Homo sapiens

<400> 191
tcggttttta gttttttcgg tcgcggggtg ggagttgggg gttgggtcgg tagtcgggat 60
ttcgggcgtt ttgtttcgtt tttgcgtagt aattg 95

<210> 192
<211> 116
<212> DNA
<213> Homo sapiens

<400> 192
ttatcgagaa gcgtcggttt cggggttgtt tatagcggtt cgggagaggt tgtggtggtt 60
tcgagcgcga gtgtgtaggt gataggatag cggttaggtt cgtttttttt ttcggt 116

<210> 193
<211> 106
<212> DNA
<213> Homo sapiens

<400> 193
ctcaatgtca cgctctggcg ctcgtcgccc gtgctccccc ttccagccgg tttccgcaga 60
atgccaggta ctgacgttgg agagcggggc cggagagggc tggttc 106

<210> 194

<211> 101
<212> DNA
<213> Homo sapiens

<400> 194
gcggctgcaa ggcctttggc cggtgagtga accagtaggc aaggatggca gccagggcac 60
ccatactcac gagggtggtg gccgagaggc tgcggaaaaa c 101

<210> 195
<211> 91
<212> DNA
<213> Homo sapiens

<400> 195
ggacgggtgt ctgcgctcca cgcttagctc gtccaggtgg gggctcccgc ctcctcggct 60
gctgcggtcc ccgcccagct ccttggtccc g 91

<210> 196
<211> 99
<212> DNA
<213> Homo sapiens

<400> 196
gccgcctgcc caggcccgga ccgggctttg tccgccccgg agcccctgcc cgcgccgcgg 60
agaccccgga gcccgcgcgc tccgaggcca ccccgggcc 99

<210> 197
<211> 96
<212> DNA
<213> Homo sapiens

<400> 197
ccggggccct tacctggtcc gctttccccc gggccggatg ccagcccgcc gagcgcaggg 60
cagcggggag ctggtagcga gacacgagtg acgact 96

<210> 198
<211> 104
<212> DNA
<213> Homo sapiens

<400> 198
gcacgtgcgc tcactgcgag ctgcggcgcc gcacagcttc gtggcgctct gggcacccct 60
gttcctgctg cgctccgccc tggccgactt cagcctggac aacg 104

<210> 199
<211> 111
<212> DNA
<213> Homo sapiens

<400> 199
ggcatcggct tggccatcgc gcgcgaactg tgccgacagt tctctgggga tgtggtgctc 60
accgcgcggg acgtggcgcg gggccaggcg gccgtgcagc agctgcaggc g 111

<210> 200
<211> 80
<212> DNA
<213> Homo sapiens

<400> 200
gcccccgact gacccatcaa ggtccgattt ggcttcggac acctcgcaga tcaccccgcg 60
gctcagagcc cggatcctga 80

<210> 201

<211> 105
<212> DNA
<213> Homo sapiens

<400> 201
ccgtgggaag agagcgtagc agctgctggg gccgcaggcg gcacggggct cagcagccca 60
ggggtcctgg cccagtgtgg gccctgcggc cgcccaggcc agcaa 105

<210> 202
<211> 100
<212> DNA
<213> Homo sapiens

<400> 202
ccctgtcaag agctgccgcc agcccggggc cggaccagtc cgggggcatc gcgatgctgc 60
tgcgcctgtt gctggcctgg gcggccgcag ggcccacact 100

<210> 203
<211> 95
<212> DNA
<213> Homo sapiens

<400> 203
ggggcagccg tcaactgcgc cttctcccct cctccgcccc caaccttaga gccccccacc 60
ccactgcttc ctgctctagc ggcccccggg gaaga 95

<210> 204
<211> 90
<212> DNA
<213> Homo sapiens

<400> 204
cgggagaaga aagccagtgc gtctctgggc gcaggggcca gtggggctcg gaggcacagg 60
caccccgcga cactccaggt tccccgaccc 90

<210> 205
<211> 120
<212> DNA
<213> Homo sapiens

<400> 205
gagcgcccgg gctttgcggg gagcaggcta aggcggccga gagaaagggg ggtcgagacg 60
ggggggtgga ggtttggggg ggtggggggg caggcggccg ccatcttctc gccgaggccg 120

<210> 206
<211> 100
<212> DNA
<213> Homo sapiens

<400> 206
ctgggaatcc agctgtcgcc gccccgcaga gccccctgtc cccggagggc gctcatttcc 60
gggccgccca ccacccgcgt agcaccggca gccgctgtcc 100

<210> 207
<211> 85
<212> DNA
<213> Homo sapiens

<400> 207
gaggtctgcg gcccaggttc gattcctgcg acttgtccta ggcaggcctg tatgtgcgcg 60
gcggccgcgt gctgtacagt gtgag 85

<210> 208
<211> 106
<212> DNA
<213> Homo sapiens

<400> 208
ggagttgggg ctcacgagac ggggcgtgcg gggcaccggg cggccggcgg ggagtcgcag 60
gcttccccag agggggcgcg agccgggccg ccggggaact caccat 106

<210> 209
<211> 114
<212> DNA
<213> Homo sapiens

<400> 209
ggtgccctga cagaagtcgt cagccggtgt catgcccagg acaggcatct gcagccttgt 60
gtggacgtca acgccaccag gaaggaccat caggccgtgg gcgtcaatgg tctt 114

<210> 210
<211> 97
<212> DNA
<213> Homo sapiens

<400> 210
ggggtcacag cctggcgctc ggaccctggc ccgggtcatc tgcgaaggag ccggctttgg 60
ccaaggtgcc ttcctggacg ggtgtggttc ccagagc 97

<210> 211
<211> 89
<212> DNA
<213> Homo sapiens

<400> 211
ggccccggct ccgccctgcc cgctgccctc ggcgacggcc gtggtccctg ccctggggtc 60
aattacagag cgaggtctga gccgcagag 89

<210> 212
<211> 113
<212> DNA
<213> Homo sapiens

<400> 212
gggacagtgg ggctgacgcg tggcttcggc gccgcgcggt ctcccgaatc ccgagccccg 60
cgcccggcgc ggccggggtc cccaaccgcc ctcccgctcg ccgggacccc cac 113

<210> 213
<211> 83
<212> DNA
<213> Homo sapiens

<400> 213
gcgtgggccc ccgccgcagc tccgcggagc ctcggtgtct cctgcaacag ggggcggggg 60
gaacagcggc gagcagccct ggg 83

<210> 214
<211> 120
<212> DNA
<213> Homo sapiens

<400> 214
gggtgtccga cccaagccga gcccgagccc gagcccaggc aggagcttta cagacagcct 60
cttcccttcc cacttcctgc aggcgcccca cgcgtgcgat cctcccggcc aagacccgcg 120

<210> 215
<211> 118
<212> DNA
<213> Homo sapiens

<400> 215
gcccagccaa gtccggcccg ggggccccta ggctaggaca tcgaggcaag agccacctga 60
accgctggcg aattggtggc tgctgcggcg acggcagcgg aggttgcaaa ttgcaatc 118

<210> 216
<211> 113
<212> DNA
<213> Homo sapiens

<400> 216
agggagccca gccaagtccg gcccgggggc ccctaggcta ggacatcgag gcaagagcca 60
cctgaaccgc tggcgaattg gtggctgctg cggcgacggc agcggaggtt gca 113

<210> 217
<211> 90
<212> DNA
<213> Homo sapiens

<400> 217
cagagcggag gaagctgcgg acctggggtg ggggaacccg cccgcggacc cctggccccc 60
accccgcgcc ggcctctgtg cccgcatctg 90

<210> 218
<211> 87
<212> DNA
<213> Homo sapiens

<400> 218
tcggagtccc acagggcaac gaagcgcggg tagcggctgc ggagccgggc ggaggtgcgc 60
ggggccgggg cgtgcggcca gcaagag 87

<210> 219
<211> 111
<212> DNA
<213> Homo sapiens

<400> 219
ctggagacct cgacagagcg ccggccccct gactgcccgc gaagcgagac gcggggcgcc 60
gggtctagcg cagtgagcgg cgaggcgcgg cggaggtgca gccggcagcc c 111

<210> 220
<211> 120
<212> DNA
<213> Homo sapiens

<400> 220
gcgacaggcc tccagcaagc aagcgcgggc ggcatccgca gtctccagaa gtttgagact 60
tggccgtaag cggactcgtg cgccccaact ctttgccgcg ccagcgcctg gagcggagag 120

<210> 221
<211> 91
<212> DNA
<213> Homo sapiens

<400> 221
cccggggtgc agcggccgcc ggggctgggg ccggcgggag tccgcgggac cctccagaag 60

agcggccggc gccgtgactc agcactgggg c                                    91

<210> 222
<211> 101
<212> DNA
<213> Homo sapiens

<400> 222
acgtaagagc ctgggagcgc ccgagccgcc cggctgcccg gagccccatc gcctaggacc 60
gggagatgct ggaaatgcaa ccgcctgttc cccgaggagc c                       101

<210> 223
<211> 96
<212> DNA
<213> Homo sapiens

<400> 223
gctccggggc tcgctctccg gcaggcccgg ggagaggtgg ggtgacaatg ggttggggtg 60
cgcgcgtgcc tcataggtgc gagacagagc gagccg                              96

<210> 224
<211> 97
<212> DNA
<213> Homo sapiens

<400> 224
cctgccggcg ccgccacccc cgcacggctc gccctcgccg cgggcgcaca tagggcagca 60
gccgcacgcg tcgcgggtct cgcccagcag gcagccc                             97

<210> 225
<211> 114
<212> DNA
<213> Homo sapiens

<400> 225
cgcccatggg ccggtcacgc cgggtgcccg ctcacccccc gacgccagca ggagcgcgcg 60
cgcaggcccc gcggggccgg gagggcggca cgggcggggc ccccgtgctc tcac          114

<210> 226
<211> 83
<212> DNA
<213> Homo sapiens

<400> 226
agctgagaac cggacggggt gggatcgagg agggtgcgaa gcgccactgt ttaggtttcg 60
ctttcccggg agcctgaccc gcc                                            83

<210> 227
<211> 91
<212> DNA
<213> Homo sapiens

<400> 227
gggactacaa gccgcgccgc gctgccgctg gcccctcagc aaccctcgac atggcgctga 60
ggcggccacc gcgactccgg ctctgcgctc g                                   91

<210> 228
<211> 91
<212> DNA
<213> Homo sapiens

<400> 228
gcgcgcagag ctgcaaaggc ccgagcagga gcacggtcca ggcgaagcgc atcactcctt 60

ttgccagatt gaccccgctc gaggaggacg t 91

<210> 229
<211> 92
<212> DNA
<213> Homo sapiens

<400> 229
ggcagaggcg aggagctgtc cgccttgcac gtttccaatc gcattacgtg aacaaatagc 60
tgaggggcgg ccgggccaga acggcttgtg ta 92

<210> 230
<211> 97
<212> DNA
<213> Homo sapiens

<400> 230
gatgtcgtct gggagcagtg cgggcccctg cattgccaag gccttatagg cacgggctgg 60
gcgggggtgg gcagtccgcc agccagcggc attctgc 97

<210> 231
<211> 109
<212> DNA
<213> Homo sapiens

<400> 231
gcggggctcc ccctaccggc cagacccggg gagaggcgcg cggaggctgc gaaggttcca 60
gaagggcggg gagggggcgc cgcgcgctga ccctccctgg gcaccgctg 109

<210> 232
<211> 97
<212> DNA
<213> Homo sapiens

<400> 232
gccttcgctg ccgccacggg cccgtcttct tcctccttcg gctcccaggg taaggcgcgg 60
ggcgcggggt tggatgcagg cgccctgccc gctgcgc 97

<210> 233
<211> 113
<212> DNA
<213> Homo sapiens

<400> 233
agaattcagg ccggcctcta tcgcttccca gaacgattgc accactgccg ctgccgccgg 60
cctgacactg cctcagcctc agtgctggca gctttgggag aagaaccctg cgc 113

<210> 234
<211> 82
<212> DNA
<213> Homo sapiens

<400> 234
gccggatgaa gcattcgggc gttcccactg cggaagggcg gggatggctg tgacgcaggc 60
gtgcccgccg tcgcctgcag gg 82

<210> 235
<211> 96
<212> DNA
<213> Homo sapiens

<400> 235
ccactccgcc cccagggagc tcccgggtca tcctctcatc cgggctgccc cgcggccccc 60

aaggagcccc acccccggga ccaaatggcc cgcaag 96

<210> 236
<211> 109
<212> DNA
<213> Homo sapiens

<400> 236
ccccgtgtgg gaagagctcg tctgggtgca gcgccgcggc ccgcaacatt agcaacggca 60
gcagcagtag cactggtaac gacgacagca ccggccgccc ggccacccg 109

<210> 237
<211> 97
<212> DNA
<213> Homo sapiens

<400> 237
ccttggtcac caggtagccc ggctcggcgg cccgcccggg gcatcagctc ggtgcagggc 60
gcggagccgt tctgcagcgg gaacagcacg aagagtg 97

<210> 238
<211> 114
<212> DNA
<213> Homo sapiens

<400> 238
tcactctgcg agcggccccg cgacacgagg cagccgctcc cgtcctccga cgcggccatg 60
ggcccggccg gcgcgggggc aagttagagc gagccgcgtg gaatcagagc atcc 114

<210> 239
<211> 75
<212> DNA
<213> Homo sapiens

<400> 239
gacctgggcg cctctggctc tccgcggtcc cgagttctcg acaaactttc tgcgccgact 60
gcggcatgag aagcc 75

<210> 240
<211> 113
<212> DNA
<213> Homo sapiens

<400> 240
gggcgctgag gcagaagaac gtgcacgagg tgaaggacca caaattcatc gcgcgcttct 60
tcaagcagcc caccttctgc agccactgca ccgacttcat ctggtaggtg ccg 113

<210> 241
<211> 102
<212> DNA
<213> Homo sapiens

<400> 241
ccaggccgga ggccccgccc ccttcctcct ggctcctccc ctcctccgtg ggccggccgc 60
caacgacgcc agagccggaa atgacgacaa cggtgagggt tc 102

<210> 242
<211> 103
<212> DNA
<213> Homo sapiens

<400> 242
gatgctccgg ccgaggtccc gccgcagccc tcccccagcc cccaggtcgc ggcgccctca 60

cccgggaccc ctccggacct ggcgcatcca tctgcagccg ccc 103

<210> 243
<211> 126
<212> DNA
<213> Homo sapiens

<400> 243
tgcagcctcc ggagtcagtg ccgcgcgccc gccgccccgc gccttcctgc tcgccgcacc 60
tccgggagcc ggggcgcacc cagcccgcag cgccgcctcc ccgcccgcgc cgcctccgac 120
cgcagg 126

<210> 244
<211> 96
<212> DNA
<213> Homo sapiens

<400> 244
tcctgctcgc cgcacctccg ggagccgggg cgcacccagc ccgcagcgcc gcctccccgc 60
ccgcgccgcc tccgaccgca ggccgagggc cgccac 96

<210> 245
<211> 80
<212> DNA
<213> Homo sapiens

<400> 245
agtacagagt ggggagcgca gcgacgaaga atgaacaggg cctcgtgagg tcccaaacac 60
ccgtttcggt ggcaggaaag 80

<210> 246
<211> 79
<212> DNA
<213> Homo sapiens

<400> 246
ctgcgccagc cgcttgcgct cgtccttagc ccacaccatg aaagcgttca tcggccgccg 60
gatacgggac tcgcccttg 79

<210> 247
<211> 79
<212> DNA
<213> Homo sapiens

<400> 247
gccagccgag tccggaggca tcgggaggtc gagagccgcc gggaccccag ctctgcgttc 60
actgccccgt ccggagctg 79

<210> 248
<211> 95
<212> DNA
<213> Homo sapiens

<400> 248
gcgccggagg ccaaggttgc cccgcacggc ccggcgggcg agcgagctcg ggctgcagca 60
gccccgccgg cggcgcgcac ggcaactttg gagag 95

<210> 249
<211> 99
<212> DNA
<213> Homo sapiens

<400> 249

cgggtcagag cactgtccgg tggtgcccag gaggagtagg agcaggagca gaagcagaag 60
cggggtccgg agctgcgcgc ctacgcggga cctgtgtcc 99

<210> 250
<211> 114
<212> DNA
<213> Homo sapiens

<400> 250
gacctcaccg gggaaacacc gcggacagtc gggccagcag cgcccggagc tcactccagg 60
tctccaaact tgcagcactt cccagagcgc gcgcgctcgg agcgggacct gctt 114

<210> 251
<211> 117
<212> DNA
<213> Homo sapiens

<400> 251
ttaccccgca ggctgaggcc agggcgtggc ggctgctggg atcccggagc ttctcagtag 60
caggggctgc gggaggaagt gaagccggga ggggctgccg gcgctgacag cagagga 117

<210> 252
<211> 108
<212> DNA
<213> Homo sapiens

<400> 252
gctgcatctt cgcggagcgc ccggcagaaa tagcctaggg aagacgaaaa acagctagcg 60
gagccgccca ggctgcagct ataaagcgcc ggccagacgc actgtgag 108

<210> 253
<211> 80
<212> DNA
<213> Homo sapiens

<400> 253
gcccaggctg tggcctaggc cgtcggttcc cggccatgcc tagctcctct gaggtcgccc 60
ttagtgagga cacgaggtgc 80

<210> 254
<211> 97
<212> DNA
<213> Homo sapiens

<400> 254
ggggccccca ggcacccggc ccgcacgcaa accctcagcc cggggccccc tgacccccgc 60
gttcacccct cagcccggcc gccgggcact gcgcatc 97

<210> 255
<211> 95
<212> DNA
<213> Homo sapiens

<400> 255
ccggccttca gtcccctcgg ccgcggggtg ggagctgggg gctgggccgg cagccgggac 60
cccgggcgtc ctgtcccgtt tctgcgcagc aactg 95

<210> 256
<211> 116
<212> DNA
<213> Homo sapiens

<400> 256

ccaccgagaa gcgccggcct cggggctgtc tacagcggcc cgggagaggc tgtggtggcc 60
ccgagcgcga gtgtgtaggt gacaggacag cggccaggcc cgcccctccc ctcggt 116

<210> 257
<211> 23
<212> DNA
<213> Homo sapiens

<400> 257
tcggagtttt atagggtaac gaa 23

<210> 258
<211> 21
<212> DNA
<213> Homo sapiens

<400> 258
ttggagattt cgatagagcg t 21

<210> 259
<211> 23
<212> DNA
<213> Homo sapiens

<400> 259
ttaaataaaa ggtttacgag cgg 23

<210> 260
<211> 23
<212> DNA
<213> Homo sapiens

<400> 260
cgtaagtaat gtttcgagcg agt 23

<210> 261
<211> 24
<212> DNA
<213> Homo sapiens

<400> 261
gttttcgtaa gtaatgtttc gagc 24

<210> 262
<211> 21
<212> DNA
<213> Homo sapiens

<400> 262
ttcggggtat tgtttacgaa g 21

<210> 263
<211> 22
<212> DNA
<213> Homo sapiens

<400> 263
gtcgtaatag gttcggttcg tt 22

<210> 264
<211> 19
<212> DNA
<213> Homo sapiens

<400> 264
ctcttactaa ccgcacgcc                                                    19

<210> 265
<211> 21
<212> DNA
<213> Homo sapiens

<400> 265
aaactaccga ctacacctcc g                                                 21

<210> 266
<211> 22
<212> DNA
<213> Homo sapiens

<400> 266
taaatatccg acgacaaacg aa                                                22

<210> 267
<211> 21
<212> DNA
<213> Homo sapiens

<400> 267
tacttccacg tctacctccc g                                                 21

<210> 268
<211> 21
<212> DNA
<213> Homo sapiens

<400> 268
tacttccacg tctacctccc g                                                 21

<210> 269
<211> 23
<212> DNA
<213> Homo sapiens

<400> 269
aataaaatct aaaaccgcac acg                                               23

<210> 270
<211> 21
<212> DNA
<213> Homo sapiens

<400> 270
aacacgtcct actatcctcg c                                                 21

<210> 271
<211> 87
<212> DNA
<213> Homo sapiens

<400> 271
tcggagtttt atagggtaac gaagcgcggg tagcggttgc ggagtcgggc ggaggtgcgc 60
ggggtcgggg cgtgcggtta gtaagag                                           87

<210> 272
<211> 111

<212> DNA
<213> Homo sapiens

<400> 272
ttggagattt cgatagagcg tcggtttttt gattgttcgc gaagcgagac gcggggcgtc 60
gggtttagcg tagtgagcgg cgaggcgcgg cggaggtgta gtcggtagtt t 111

<210> 273
<211> 103
<212> DNA
<213> Homo sapiens

<400> 273
   ttaaataaaa ggtttacgag cggcggtttc gacgtgggga gtaagtaggt ttttttggtt 60
   tttgtagtag gcggatttag gttcgtttgt cgtcggatat tta 103

<210> 274
<211> 95
<212> DNA
<213> Homo sapiens

<400> 274
cgtaagtaat gtttcgagcg agtagatttt aggataataa ataaattaaa attatatttt 60
aaattattgg agatcgggag gtagacgtgg aagta 95

<210> 275
<211> 100
<212> DNA
<213> Homo sapiens

<400> 275
gttttcgtaa gtaatgtttc gagcgagtag attttaggat aataaataaa ttaaaattat 60
attttaaatt attggagatc gggaggtaga cgtggaagta 100

<210> 276
<211> 113
<212> DNA
<213> Homo sapiens

<400> 276
ttcggggtat tgtttacgaa gatcgtttat atgttgttgt atttttttat ttatttgtta 60
cgtgatcgcg tttgtttatt ataggtttaa cgtgtgcggt tttagatttt att 113

<210> 277
<211> 120
<212> DNA
<213> Homo sapiens

<400> 277
gtcgtaatag gttcggttcg ttatagtagg ttttgaaggc gggtttttag cgttcgagta 60
tcgcgaggag ggtgtcgtag tggttagtcg cgttcgtcgg cgaggatagt aggacgtgtt 120

<210> 278
<211> 87
<212> DNA
<213> Homo sapiens

<400> 278
tcggagtccc acagggcaac gaagcgcggg tagcggctgc ggagccgggc ggaggtgcgc 60
ggggccgggg cgtgcggcca gcaagag 87

<210> 279

<211> 111
<212> DNA
<213> Homo sapiens

<400> 279
ctggagacct cgacagagcg ccggccccct gactgcccgc gaagcgagac gcggggcgcc 60
gggtctagcg cagtgagcgg cgaggcgcgg cggaggtgca gccggcagcc c 111

<210> 280
<211> 103
<212> DNA
<213> Homo sapiens

<400> 280
tcaaacaaaa ggctcacgag cggcggcccc gacgtgggga gcaagcaggc ttctctggcc 60
cctgcagcag gcggacccag gcccgcctgc cgccggacat tca 103

<210> 281
<211> 95
<212> DNA
<213> Homo sapiens

<400> 281
cgtaagcaat gccccgagcg agtagatttc aggacaacaa acaaatcaaa attacacttc 60
aaattattgg agatcgggag gcagacgtgg aagca 95

<210> 282
<211> 100
<212> DNA
<213> Homo sapiens

<400> 282
gtcttcgtaa gcaatgcccc gagcgagtag atttcaggac aacaaacaaa tcaaaattac 60
acttcaaatt attggagatc gggaggcaga cgtggaagca 100

<210> 283
<211> 113
<212> DNA
<213> Homo sapiens

<400> 283
ctcggggcat tgcttacgaa gaccgtttat atgttgctgc atccctctac ctatctgtta 60
cgtgaccgcg cttgtccatc acaggcccaa cgtgtgcggc tccagattcc act 113

<210> 284
<211> 120
<212> DNA
<213> Homo sapiens

<400> 284
gccgcaacag gttcggtccg ctacagcagg ctctgaaggc gggtttctag cgcccgagta 60
tcgcgaggag ggtgccgcag tggccagccg cgtccgccgg cgaggacagc aggacgtgct 120

<210> 285
<211> 19
<212> DNA
<213> Homo sapiens

<400> 285
gtagtggcga gatgacgga 19

<210> 286

<211> 23
<212> DNA
<213> Homo sapiens

<400> 286
agtgtttaga gagttcgtcg gtt                                               23

<210> 287
<211> 21
<212> DNA
<213> Homo sapiens

<400> 287
gtcgggtgtt atatggtgcg t                                                 21

<210> 288
<211> 22
<212> DNA
<213> Homo sapiens

<400> 288
ttttgagagg tcgttatcgt gt                                                22

<210> 289
<211> 19
<212> DNA
<213> Homo sapiens

<400> 289
cgttaggtag ggaggaggc                                                    19

<210> 290
<211> 22
<212> DNA
<213> Homo sapiens

<400> 290
atttgggtag aaaagttcgt tc                                                22

<210> 291
<211> 22
<212> DNA
<213> Homo sapiens

<400> 291
aacgatttgg ggttagagtt tc                                                22

<210> 292
<211> 19
<212> DNA
<213> Homo sapiens

<400> 292
ttttcgttat ggacgcgga                                                    19

<210> 293
<211> 25
<212> DNA
<213> Homo sapiens

<400> 293
cgttcgtata gagttcggta atgtc                                             25

<210> 294
<211> 21
<212> DNA
<213> Homo sapiens

<400> 294
cgtttttggt tttgttttcg t                                                21

<210> 295
<211> 21
<212> DNA
<213> Homo sapiens

<400> 295
tttagtaggt ttggtcgagg c                                                21

<210> 296
<211> 21
<212> DNA
<213> Homo sapiens

<400> 296
   tataggtaag gggcggtttt c                                                 21

<210> 297
<211> 18
<212> DNA
<213> Homo sapiens

<400> 297
gcgttgaagt cggggttc                                                    18

<210> 298
<211> 19
<212> DNA
<213> Homo sapiens

<400> 298
ttagaggggc gtagggtgc                                                   19

<210> 299
<211> 21
<212> DNA
<213> Homo sapiens

<400> 299
cgttgtagga ttttgggggt c                                                21

<210> 300
<211> 19
<212> DNA
<213> Homo sapiens

<400> 300
aaccgaaacc aaacaaacg                                                   19

<210> 301
<211> 22
<212> DNA
<213> Homo sapiens

<400> 301
cgtaacgaat aaactacgcg at                                               22

<210> 302
<211> 21
<212> DNA
<213> Homo sapiens

<400> 302
aaaaacaatc ctcgtccgaa a                                                21

<210> 303
<211> 22
<212> DNA
<213> Homo sapiens

<400> 303
ttactcgaac tattccccga tt                                               22

<210> 304
<211> 24
<212> DNA
<213> Homo sapiens

<400> 304
aacgataaaa taaaaacaac gacc                                             24

<210> 305
<211> 21
<212> DNA
<213> Homo sapiens

<400> 305
ctcaataatt ttcccgacga c                                                21

<210> 306
<211> 22
<212> DNA
<213> Homo sapiens

<400> 306
taaccaaact accgctttac ga                                               22

<210> 307
<211> 20
<212> DNA
<213> Homo sapiens

<400> 307
ataaccttac cgaccccgaa                                                  20

<210> 308
<211> 22
<212> DNA
<213> Homo sapiens

<400> 308
cctataacta aacgcgacgc ac                                               22

<210> 309
<211> 19
<212> DNA
<213> Homo sapiens

<400> 309

aacctaattc ccgcccgtt 19

<210> 310
<211> 19
<212> DNA
<213> Homo sapiens

<400> 310
acgctaaccc caaaatccg 19

<210> 311
<211> 20
<212> DNA
<213> Homo sapiens

<400> 311
gcgactctaa aaattccgct 20

<210> 312
<211> 24
<212> DNA
<213> Homo sapiens

<400> 312
cccgtacttc gctaacttta aacg 24

<210> 313
<211> 21
<212> DNA
<213> Homo sapiens

<400> 313
gccaaactaa aatcccaacg a 21

<210> 314
<211> 19
<212> DNA
<213> Homo sapiens

<400> 314
acccttctcc cgctcgata 19

<210> 315
<211> 92
<212> DNA
<213> Homo sapiens

<400> 315
gtagtggcga gatgacggat atttagcgag tttaatgggc gtcgaacgcg tttaggtttg 60
gtggatttgt tagcgtttgt ttggtttcgg tt 92

<210> 316
<211> 94
<212> DNA
<213> Homo sapiens

<400> 316
agtgtttaga gagttcgtcg gttttatcgt ttttttaaag gagaattcgg tttatcgttc 60
gtcgcggcgg cgatcgcgta gtttattcgt tacg 94

<210> 317
<211> 116
<212> DNA

<213> Homo sapiens

<400> 317
gtcgggtgtt atatggtgcg tgataatttg tttttgattt gggtttattt gaagagcgta      60
gaattttaat aaataaatag ttttttggga tttgttttcg gacgaggatt gttttt         116

<210> 318
<211> 107
<212> DNA
<213> Homo sapiens

<400> 318
ttttgagagg tcgttatcgt gttatgggcg tgcgtaattg tttttacggt aataatatgt      60
taggataacg cgatattttt tttgaaatcg gggaatagtt cgagtaa                   107

<210> 319
<211> 117
<212> DNA
<213> Homo sapiens

<400> 319
cgttaggtag ggaggaggcg gggaggggtt ggttttagaa gtgcgtgttt gaagcggtta      60
atgtgtgtaa attagtaagg aggaggggtg cggggtcgtt gtttttattt tatcgtt        117

<210> 320
<211> 87
<212> DNA
<213> Homo sapiens

<400> 320
atttgggtag aaaagttcgt tcgtgacgtt attaagtttc ggaagttttt tggcgtcggc      60
gtaagggtcg tcgggaaaat tattgag                                          87

<210> 321
<211> 109
<212> DNA
<213> Homo sapiens

<400> 321
aacgatttgg ggttagagtt tcgggagttg gcggagcgcg gagttcgtat cgtttttaga      60
ggtaggacgt agtttttttt tttgaattcg taaagcggta gtttggtta                 109

<210> 322
<211> 88
<212> DNA
<213> Homo sapiens

<400> 322
ttttcgttat ggacgcggac gattttcggg tttttaaggg ttttttgcgg aagtttttgg      60
agtatttttt cggggtcggt aaggttat                                         88

<210> 323
<211> 108
<212> DNA
<213> Homo sapiens

<400> 323
cgttcgtata gagttcggta atgtcgaggt ttttttaacg ggtcggtttg cgaggagtaa      60
aaaaggggtt tagaggaggg tagcgcgtgc gtcgcgttta gttatagg                  108

<210> 324
<211> 119
<212> DNA

<213> Homo sapiens

<400> 324
cgtttttggt tttgttttcg tcgcggagcg gaatttttta agtcgcggtt tgaggaggaa      60
ggaaaagggg gcggttcggg agagtcgttg cgaaattagt aacgggcggg aattaggtt      119

<210> 325
<211> 120
<212> DNA
<213> Homo sapiens

<400> 325
tttagtaggt ttggtcgagg cgggattttc gtcgttttag ttttcgtttt cgttttcgcg      60
ttttttcgtc gcgcggagtt ttggttggtt tgttttttaa tcggattttg gggttagcgt      120

<210> 326
<211> 109
<212> DNA
<213> Homo sapiens

<400> 326
tataggtaag ggggcggttt cgtttcgcgt tttggaacga ttttacggtt tcgtttatat      60
tttcgttttt ggttttattt tcgtcgtaga gcggaatttt tagagtcgc      109

<210> 327
<211> 75
<212> DNA
<213> Homo sapiens

<400> 327
gcgttgaagt cggggttcgt tttgtggttt cgttcggttc gcgtttgtta gcgtttaaag      60
ttagcgaagt acggg      75

<210> 328
<211> 113
<212> DNA
<213> Homo sapiens

<400> 328
ttagaggggc gtagggtgcg cgggggtcgt tggtttttcg ggtatttttt ttttgcggtt      60
ttttcgtttt ttttcggagt tggtgtttga ggtcgttggg attttagttt ggc      113

<210> 329
<211> 84
<212> DNA
<213> Homo sapiens

<400> 329
cgttgtagga ttttgggggt cggacggtgg gatacggtta attttcgggg agatgttgtg      60
gtttttatcg agcgggagaa gggt      84

<210> 330
<211> 92
<212> DNA
<213> Homo sapiens

<400> 330
gcagtggcga gatgacggac acccagcgag tccaatgggc gtcgaacgcg tctaggcttg      60
gtggacttgt cagcgcctgc ctggcttcgg tc      92

<210> 331
<211> 94
<212> DNA

<213> Homo sapiens

<400> 331
agtgcccaga gagtccgccg gtcccaccgc cccttcaaag gagaacccgg cccaccgccc 60
gccgcggcgg cgaccgcgca gcccactcgt cacg 94

<210> 332
<211> 116
<212> DNA
<213> Homo sapiens

<400> 332
gccgggtgtc acatggtgcg tgataacttg cccttgattt gggttcattt gaagagcgta 60
gaactctaac aaataaacag ccttttggga cctgtccccg gacgaggact gccccc 116

<210> 333
<211> 107
<212> DNA
<213> Homo sapiens

<400> 333
ttttgagagg ccgccaccgt gttatgggcg tgcgcaactg cctctacggc aataatatgt 60
caggacaacg cgatatcccc cctgaaatcg gggaacagcc cgagcaa 107

<210> 334
<211> 117
<212> DNA
<213> Homo sapiens

<400> 334
cgccaggcag ggaggaggcg gggaggggct ggccccagaa gtgcgtgtct gaagcggcca 60
atgtgtgcaa atcagcaagg aggaggggtg cggggccgct gcccccacct caccgcc 117

<210> 335
<211> 87
<212> DNA
<213> Homo sapiens

<400> 335
atttgggtag aaaagctcgc tcgtgacgtc accaagctcc ggaagtctcc tggcgtcggc 60
gcaagggccg ccgggaaaac cattgag 87

<210> 336
<211> 109
<212> DNA
<213> Homo sapiens

<400> 336
aacgacctgg ggctagagcc ccgggagctg gcggagcgcg gagtccgcat cgtctccaga 60
ggtaggacgc agcttttctc tctgaatccg caaagcggca gcttggtca 109

<210> 337
<211> 88
<212> DNA
<213> Homo sapiens

<400> 337
tcctcgccat ggacgcggac gactcccggg cccccaaggg ctccttgcgg aagttcctgg 60
agcacctctc cggggccggc aaggccat 88

<210> 338
<211> 108
<212> DNA

<213> Homo sapiens

<400> 338
cgctcgcaca gagcccggca atgccgaggc cctcccaacg ggtcggtctg cgaggagcaa 60
aaaaggggtt cagaggaggg cagcgcgtgc gtcgcgctca gctatagg 108

<210> 339
<211> 120
<212> DNA
<213> Homo sapiens

<400> 339
cccagcaggc ctggccgagg cgggaccttc gtcgctccag cccccgtccc cgcccccgcg 60
cctccccgcc gcgcggagct ctggttggct tgctttccaa ccggactttg gggctagcgt 120

<210> 340
<211> 120
<212> DNA
<213> Homo sapiens

<400> 340
cccagcaggc ctggccgagg cgggaccttc gtcgctccag cccccgtccc cgcccccgcg 60
cctccccgcc gcgcggagct ctggttggct tgctttccaa ccggactttg gggctagcgt 120

<210> 341
<211> 109
<212> DNA
<213> Homo sapiens

<400> 341
cacaggtaag gggcggcccc cgccccgcgc cctggaacga cctcacggcc ccgcccacat 60
ccccgcccct ggccccacct ccgccgcaga gcggaaccct cagagtcgc 109

<210> 342
<211> 75
<212> DNA
<213> Homo sapiens

<400> 342
gcgctgaagt cggggcccgc cctgtggccc cgcccggccc gcgcttgcta gcgcccaaag 60
ccagcgaagc acggg 75

<210> 343
<211> 113
<212> DNA
<213> Homo sapiens

<400> 343
tcagaggggc gcagggtgcg cgggggccgt tggccctccg ggcacttccc ctttgcggtc 60
tccccgccct ccttcggagc tggtgcctga ggtcgctggg acctcagcct ggc 113

<210> 344
<211> 84
<212> DNA
<213> Homo sapiens

<400> 344
cgctgcagga ccctgggggc cggacggtgg gatacggcca atctccgggg agatgctgtg 60
gctcttaccg agcgggagaa gggt 84

<210> 345
<211> 27
<212> DNA

<213> Homo sapiens

<400> 345
ttatatgtcg gttacgtgcg tttatat                                          27

<210> 346
<211> 23
<212> DNA
<213> Homo sapiens

<400> 346
agcgtagaga taggttggta acg                                              23

<210> 347
<211> 21
<212> DNA
<213> Homo sapiens

<400> 347
tacgcgtagg ttttaagtcg c                                                21

<210> 348
<211> 25
<212> DNA
<213> Homo sapiens

<400> 348
aaagttagta ggagtaagag gacgc                                            25

<210> 349
<211> 20
<212> DNA
<213> Homo sapiens

<400> 349
ggggttatag tttggcgttc                                                  20

<210> 350
<211> 20
<212> DNA
<213> Homo sapiens

<400> 350
ttcggggtgt agcggtcgtc                                                  20

<210> 351
<211> 19
<212> DNA
<213> Homo sapiens

<400> 351
gtttcggggt tcgtttttc                                                   19

<210> 352
<211> 19
<212> DNA
<213> Homo sapiens

<400> 352
agttttcgga gaagacggc                                                   19

<210> 353
<211> 32

<212> DNA
<213> Homo sapiens

<400> 353
cgttaggtta ttttttaaat agagtcggta gc                                    32

<210> 354
<211> 20
<212> DNA
<213> Homo sapiens

<400> 354
gggattataa gtcgcgtcgc                                                  20

<210> 355
<211> 22
<212> DNA
<213> Homo sapiens

<400> 355
cgtgaataaa tagttgaggg gc                                               22

<210> 356
<211> 27
<212> DNA
<213> Homo sapiens

<400> 356
gcggggtttt ttttatcggt tagattc                                          27

<210> 357
<211> 25
<212> DNA
<213> Homo sapiens

<400> 357
gcggttttta aggagtttta ttttc                                            25

<210> 358
<211> 22
<212> DNA
<213> Homo sapiens

<400> 358
aacgatttgg ggttagagtt tc                                               22

<210> 359
<211> 25
<212> DNA
<213> Homo sapiens

<400> 359
cgttcgtata gagttcggta atgtc                                            25

<210> 360
<211> 23
<212> DNA
<213> Homo sapiens

<400> 360
tcgtcgtttt agttttcgtt ttc                                              23

<210> 361

<211> 18
<212> DNA
<213> Homo sapiens

<400> 361
gcgttgaagt cggggttc                                                    18

<210> 362
<211> 21
<212> DNA
<213> Homo sapiens

<400> 362
aggtaggttt tagttttcgg c                                                21

<210> 363
<211> 26
<212> DNA
<213> Homo sapiens

<400> 363
tgtagttttc ggagttagtg tcgcgc                                           26

<210> 364
<211> 19
<212> DNA
<213> Homo sapiens

<400> 364
gagatgtttc gagggttgc                                                   19

<210> 365
<211> 22
<212> DNA
<213> Homo sapiens

<400> 365
gttagtcgag ttcggaggta tc                                               22

<210> 366
<211> 19
<212> DNA
<213> Homo sapiens

<400> 366
gaaccaaaac gctccccat                                                   19

<210> 367
<211> 22
<212> DNA
<213> Homo sapiens

<400> 367
aaaacgataa cccttaaacc ga                                               22

<210> 368
<211> 22
<212> DNA
<213> Homo sapiens

<400> 368
tcccgaacta aacgaaaccc cg                                               22

<210> 369
<211> 19
<212> DNA
<213> Homo sapiens

<400> 369
aatacgacgc caaaaaccg                                                    19

<210> 370
<211> 21
<212> DNA
<213> Homo sapiens

<400> 370
gctctaaaaa ccacacccgt c                                                 21

<210> 371
<211> 22
<212> DNA
<213> Homo sapiens

<400> 371
gccccaatac taaatcacga cg                                                22

<210> 372
<211> 20
<212> DNA
<213> Homo sapiens

<400> 372
cgactcgctc tatctcgcac                                                   20

<210> 373
<211> 26
<212> DNA
<213> Homo sapiens

<400> 373
acgactaaac tactataacc ctacga                                            26

<210> 374
<211> 27
<212> DNA
<213> Homo sapiens

<400> 374
cgactatacg aactaacgaa taaaccg                                           27

<210> 375
<211> 20
<212> DNA
<213> Homo sapiens

<400> 375
cgaacgcaaa accgaaatcg                                                   20

<210> 376
<211> 20
<212> DNA
<213> Homo sapiens

<400> 376
gacaatcccg aaaaacgaac                                                   20

<210> 377
<211> 27
<212> DNA
<213> Homo sapiens

<400> 377
caacgatacc caaaaaaaat caacgcg                                          27

<210> 378
<211> 21
<212> DNA
<213> Homo sapiens

<400> 378
ctacgaaatt ccctttacgc t                                                21

<210> 379
<211> 22
<212> DNA
<213> Homo sapiens

<400> 379
taaccaaact accgctttac ga                                               22

<210> 380
<211> 22
<212> DNA
<213> Homo sapiens

<400> 380
cctataacta aacgcgacgc ac                                               22

<210> 381
<211> 19
<212> DNA
<213> Homo sapiens

<400> 381
acgctaaccc caaaatccg                                                   19

<210> 382
<211> 24
<212> DNA
<213> Homo sapiens

<400> 382
cccgtacttc gctaacttta aacg                                             24

<210> 383
<211> 20
<212> DNA
<213> Homo sapiens

<400> 383
gacctcaaac accaactccg                                                  20

<210> 384
<211> 25
<212> DNA
<213> Homo sapiens

<400> 384

cctacgatcg aaaacgacgc gaacg 25

<210> 385
<211> 20
<212> DNA
<213> Homo sapiens

<400> 385
ccgcaatatc actaaaccga 20

<210> 386
<211> 24
<212> DNA
<213> Homo sapiens

<400> 386
caactccgaa cgaaacaata aacg 24

<210> 387
<211> 38
<212> DNA
<213> Homo sapiens

<400> 387
cgtctgcccc gtcgaaaacc cgccgattaa cgcagacg 38

<210> 388
<211> 36
<212> DNA
<213> Homo sapiens

<400> 388
cgacatgcgc ggaggggtta gcgtggttgc atgtcg 36

<210> 389
<211> 37
<212> DNA
<213> Homo sapiens

<400> 389
cgacatgcct acgaccgcgt cgcccattag catgtcg 37

<210> 390
<211> 32
<212> DNA
<213> Homo sapiens

<400> 390
cgacatgcgg gcggtcgggg tcgcgcatgt cg 32

<210> 391
<211> 42
<212> DNA
<213> Homo sapiens

<400> 391
cgacatgcgg ttcgggttat ttgcgaagga gtcggcatgt cg 42

<210> 392
<211> 41
<212> DNA
<213> Homo sapiens

<400> 392
cgtctgcttg gggtcggcgg gagttcgcgg gattgcagac g                41

<210> 393
<211> 35
<212> DNA
<213> Homo sapiens

<400> 393
cgacatgcac gcgcgcaccc caacccagca tgtcg                       35

<210> 394
<211> 38
<212> DNA
<213> Homo sapiens

<400> 394
cgacatgcac cacgaccacg cgaatcgaac gcatgtcg                    38

<210> 395
<211> 37
<212> DNA
<213> Homo sapiens

<400> 395
cgacatgcac gccgaccgcg atctaactcg catgtcg                     37

<210> 396
<211> 35
<212> DNA
<213> Homo sapiens

<400> 396
cgacacgata tggcgttgag gcggttatcg tgtcg                       35

<210> 397
<211> 38
<212> DNA
<213> Homo sapiens

<400> 397
cgtctgccac aaaccgttct aacccgaccg cgcagacg                    38

<210> 398
<211> 32
<212> DNA
<213> Homo sapiens

<400> 398
cgacatgccg acgccccctc cccggcatgt cg                           32

<210> 399
<211> 39
<212> DNA
<213> Homo sapiens

<400> 399
cgacatgggt tcgtaaggtt tggggtagcg gccatgtcg                   39

<210> 400
<211> 32
<212> DNA
<213> Homo sapiens

<400> 400
cgacatgcgc gctccgccaa ctccgcatgt cg                                    32

<210> 401
<211> 38
<212> DNA
<213> Homo sapiens

<400> 401
cgacatgcgg ggtttagagg agggtagcgc gcatgtcg                              38

<210> 402
<211> 27
<212> DNA
<213> Homo sapiens

<400> 402
actccgcgtt ttttcgtcgc gcggagt                                          27

<210> 403
<211> 42
<212> DNA
<213> Homo sapiens

<400> 403
cgtctgcgtg gtttcgttcg gttcgcgttt gttaggcaga cg                         42

<210> 404
<211> 36
<212> DNA
<213> Homo sapiens

<400> 404
cgacatgcgg gtgcgcgggg gtcgttgggc atgtcg                                36

<210> 405
<211> 47
<212> DNA
<213> Homo sapiens

<400> 405
cgacatgctc gggagtcggg gcgtatttag ttcgtagcgg catgtcg                    47

<210> 406
<211> 43
<212> DNA
<213> Homo sapiens

<400> 406
cgacatgcgt tcgtgttttg gtttgtcgcg gtttggcatg tcg                        43

<210> 407
<211> 36
<212> DNA
<213> Homo sapiens

<400> 407
cgacatgccc gacgactctc gacctcccgc atgtcg                                36

<210> 408
<211> 74
<212> DNA

<213> Homo sapiens

<400> 408
ttatatgtcg gttacgtgcg tttatattta gttaatcggc gggttttcga cgggaatggg 60
gagcgttttg gttc 74

<210> 409
<211> 111
<212> DNA
<213> Homo sapiens

<400> 409
agcgtagaga taggttggta acggttttag ggaggcgcgg aggggttagc gtggttggtt 60
taaaaggata tagggattga ggggtaagat cggtttaagg gttatcgttt t 111

<210> 410
<211> 113
<212> DNA
<213> Homo sapiens

<400> 410
tacgcgtagg ttttaagtcg cggttaatgg gcgacgcggt cgtagattcg ttcggtttcg 60
ttttgttttg tgagtttttt cggtcgggtt gcggggtttc gtttagttcg gga 113

<210> 411
<211> 117
<212> DNA
<213> Homo sapiens

<400> 411
aaagttagta ggagtaagag gacgcgtagg agggtttcgg tcgcggttat ttttgggcgg 60
tcggggtcgc ggtttcggga gcggtgcggg cgcgggttcg gtttttggcg tcgtatt 117

<210> 412
<211> 97
<212> DNA
<213> Homo sapiens

<400> 412
ggggttatag tttggcgttc ggattttggt tcgggttatt tgcgaaggag tcggttttgg 60
ttaaggtgtt tttttggacg ggtgtggttt ttagagc 97

<210> 413
<211> 91
<212> DNA
<213> Homo sapiens

<400> 413
ttcggggtgt agcggtcgtc ggggttgggg tcggcgggag ttcgcgggat tttttagaag 60
agcggtcggc gtcgtgattt agtattgggg c 91

<210> 414
<211> 96
<212> DNA
<213> Homo sapiens

<400> 414
gtttcggggt tcgtttttcg gtaggttcgg ggagaggtgg ggtgataatg ggttggggtg 60
cgcgcgtgtt ttataggtgc gagatagagc gagtcg 96

<210> 415
<211> 101
<212> DNA

<213> Homo sapiens

<400> 415
agttttcgga gaagacggcg tttttaacgt tcgattcgcg tggtcgtggt agcgttacgc 60
gagtttttta ggcgatcgta gggttatagt agtttagtcg t 101

<210> 416
<211> 117
<212> DNA
<213> Homo sapiens

<400> 416
cgttaggtta ttttttaaat agagtcggta gcgcgtttcg ttcggtattt ttcgaagagt 60
tagatcgcgg tcggcgttag cgttatcgtt cggtttattc gttagttcgt atagtcg 117

<210> 417
<211> 91
<212> DNA
<213> Homo sapiens

<400> 417
gggattataa gtcgcgtcgc gttgtcgttg gttttttagt aattttcgat atggcgttga 60
ggcggttatc gcgatttcgg ttttgcgttc g 91

<210> 418
<211> 124
<212> DNA
<213> Homo sapiens

<400> 418
cgtgaataaa tagttgaggg gcggtcgggt tagaacggtt tgtgtaattt tgtaaacgtg 60
ttagaaagtt taaaattttt tttttttttt ttattttaga tattgttcgt ttttcgggat 120
tgtc 124

<210> 419
<211> 109
<212> DNA
<213> Homo sapiens

<400> 419
gcggggtttt ttttatcggt tagattcggg gagaggcgcg cggaggttgc gaaggtttta 60
gaagggcggg gagggggcgt cgcgcgttga ttttttttgg gtatcgttg 109

<210> 420
<211> 99
<212> DNA
<213> Homo sapiens

<400> 420
gcggttttta aggagtttta ttttcgggat taaatggttc gtaaggtttg gggtagcggc 60
gttgtaggag atgagtttag cgtaaaggga atttcgtag 99

<210> 421
<211> 109
<212> DNA
<213> Homo sapiens

<400> 421
aacgatttgg ggttagagtt tcgggagttg gcggagcgcg gagttcgtat cgtttttaga 60
ggtaggacgt agtttttttt tttgaattcg taaagcggta gtttggtta 109

<210> 422
<211> 108

<212> DNA
<213> Homo sapiens

<400> 422
cgttcgtata gagttcggta atgtcgaggt ttttttaacg ggtcggtttg cgaggagtaa      60
aaaaggggtt tagaggaggg tagcgcgtgc gtcgcgttta gttatagg                  108

<210> 423
<211> 92
<212> DNA
<213> Homo sapiens

<400> 423
tcgtcgtttt agttttcgtt ttcgttttcg cgttttttcg tcgcgcggag ttttggttgg      60
tttgtttttt aatcggattt tggggttagc gt                                    92

<210> 424
<211> 75
<212> DNA
<213> Homo sapiens

<400> 424
gcgttgaagt cggggttcgt tttgtggttt cgttcggttc gcgtttgtta gcgtttaaag      60
ttagcgaagt acggg                                                       75

<210> 425
<211> 133
<212> DNA
<213> Homo sapiens

<400> 425
aggtaggttt tagttttcgg cgcggggagg cggcgcgttt tagaggggcg tagggtgcgc      60
gggggtcgtt ggtttttcgg gtattttttt tttgcggttt tttcgttttt tttcggagtt     120
ggtgtttgag gtc                                                        133

<210> 426
<211> 126
<212> DNA
<213> Homo sapiens

<400> 426
tgtagttttc ggagttagtg tcgcgcgttc gtcgtttcgc gtttttttgt tcgtcgtatt      60
ttcgggagtc ggggcgtatt tagttcgtag cgtcgttttt tcgttcgcgt cgttttcgat     120
cgtagg                                                                126

<210> 427
<211> 117
<212> DNA
<213> Homo sapiens

<400> 427
gagatgtttc gagggttgcg cgggtttttc ggttcgaagt cgtcgttcgt gttttggttt      60
gtcgcggttt ggtttatagc gtatttaggg tttttagtcg gtttagtgat attgcgg        117

<210> 428
<211> 79
<212> DNA
<213> Homo sapiens

<400> 428
gttagtcgag ttcggaggta tcgggaggtc gagagtcgtc gggattttag ttttgcgttt      60
attgtttcgt tcggagttg                                                   79

<210> 429
<211> 74
<212> DNA
<213> Homo sapiens

<400> 429
ccacatgtcg gtcacgtgcg cccacaccca gccaatcggc gggctcccga cgggaatggg 60
gagcgccctg gtcc 74

<210> 430
<211> 111
<212> DNA
<213> Homo sapiens

<400> 430
agcgcagaga caggctggca acggcttcag ggaggcgcgg aggggtcagc gtggctggct 60
taaaaggata cagggactga ggggcaagac cggctcaagg gtcaccgctt c 111

<210> 431
<211> 113
<212> DNA
<213> Homo sapiens

<400> 431
cacgcgcagg ccccaagtcg cggccaatgg gcgacgcggc cgcagatccg cccggccccg 60
ccctgccctg tgagttcctc cggccgggct gcggggctcc gctcagtccg gga 113

<210> 432
<211> 117
<212> DNA
<213> Homo sapiens

<400> 432
aaagccagca ggagcaagag gacgcgcagg agggcttcgg tcgcggtcat ctctgggcgg 60
ccggggtcgc ggtcccggga gcggtgcggg cgcgggtccg gctcctggcg ccgcact 117

<210> 433
<211> 97
<212> DNA
<213> Homo sapiens

<400> 433
ggggtcacag cctggcgctc ggaccctggc ccgggtcatc tgcgaaggag ccggctttgg 60
ccaaggtgcc ttcctggacg ggtgtggttc ccagagc 97

<210> 434
<211> 91
<212> DNA
<213> Homo sapiens

<400> 434
cccggggtgc agcggccgcc ggggctgggg ccggcgggag tccgcgggac cctccagaag 60
agcggccggc gccgtgactc agcactgggg c 91

<210> 435
<211> 96
<212> DNA
<213> Homo sapiens

<400> 435
gctccggggc tcgctctccg gcaggcccgg ggagaggtgg ggtgacaatg ggttggggtg 60
cgcgcgtgcc tcataggtgc gagacagagc gagccg 96

<210> 436
<211> 101
<212> DNA
<213> Homo sapiens

<400> 436
agcccccgga gaagacggcg cccccaacgc ccgacccgcg tggccgtggc agcgccacgc 60
gagccctcta ggcgaccgca gggccacagc agctcagccg c 101

<210> 437
<211> 117
<212> DNA
<213> Homo sapiens

<400> 437
cgtcaggcca ctccttaaat agagccggca gcgcgctccg ctcggcattt cccgaagagc 60
cagatcgcgg ccggcgccag cgccaccgtc cggtccaccc gccagcccgc acagccg 117

<210> 438
<211> 91
<212> DNA
<213> Homo sapiens

<400> 438
gggactacaa gccgcgccgc gctgccgctg gcccctcagc aaccctcgac atggcgctga 60
ggcggccacc gcgactccgg ctctgcgctc g 91

<210> 439
<211> 124
<212> DNA
<213> Homo sapiens

<400> 439
cgtgaacaaa tagctgaggg gcggccgggc cagaacggct tgtgtaactt tgcaaacgtg 60
ccagaaagtt taaaatctct cctccttcct tcactccaga cactgcccgc tctccgggac 120
tgcc 124

<210> 440
<211> 109
<212> DNA
<213> Homo sapiens

<400> 440
gcggggctcc ccctaccggc cagacccggg gagaggcgcg cggaggctgc gaaggttcca 60
gaagggcggg gagggggcgc cgcgcgctga ccctccctgg gcaccgctg 109

<210> 441
<211> 99
<212> DNA
<213> Homo sapiens

<400> 441
gcggccccca aggagcccca cccccgggac caaatggccc gcaaggtttg gggcagcggc 60
gttgcaggag atgagctcag cgcaaaggga accccgcag 99

<210> 442
<211> 109
<212> DNA
<213> Homo sapiens

<400> 442
aacgacctgg ggctagagcc ccgggagctg gcggagcgcg gagtccgcat cgtctccaga 60
ggtaggacgc agcttttctc tctgaatccg caaagcggca gcttggtca 109

<210> 443
<211> 108
<212> DNA
<213> Homo sapiens

<400> 443
cgctcgcaca gagcccggca atgccgaggc cctcccaacg ggtcggtctg cgaggagcaa 60
aaaaggggtt cagaggaggg cagcgcgtgc gtcgcgctca gctatagg 108

<210> 444
<211> 92
<212> DNA
<213> Homo sapiens

<400> 444
tcgtcgctcc agcccccgtc cccgcccccg cgcctccccg ccgcgcggag ctctggttgg 60
cttgctttcc aaccggactt tggggctagc gt 92

<210> 445
<211> 75
<212> DNA
<213> Homo sapiens

<400> 445
gcgctgaagt cggggcccgc cctgtggccc cgcccggccc gcgcttgcta gcgcccaaag 60
ccagcgaagc acggg 75

<210> 446
<211> 119
<212> DNA
<213> Homo sapiens

<400> 446
aggcaggtcc cagtccccgg cgcggggagg cggcgcgctt cagaggggcg cagggtgcgc 60
gggggccgtt ggccctccgg gcacttcccc tttgcggtct ccccgccctc cttcggagc 119

<210> 447
<211> 126
<212> DNA
<213> Homo sapiens

<400> 447
tgcagcctcc ggagtcagtg ccgcgcgccc gccgccccgc gccttcctgc tcgccgcacc 60
tccgggagcc ggggcgcacc cagcccgcag cgccgcctcc ccgcccgcgc cgcctccgac 120
cgcagg 126

<210> 448
<211> 117
<212> DNA
<213> Homo sapiens

<400> 448
gagatgcccc gagggctgcg cgggtctccc ggcccgaagc cgccgcccgt gttctggcct 60
gtcgcggtct ggtctacagc gtacccaggg cccccagccg gcctagtgac actgcgg 117

<210> 449
<211> 79
<212> DNA
<213> Homo sapiens

<400> 449
gccagccgag tccggaggca tcgggaggtc gagagccgcc gggaccccag ctctgcgttc 60

actgccccgt ccggagctg 79